(19) Europäisches Patentamt / European Patent Office / Office européen des brevets

(11) **EP 2 713 877 B1**

(12) **EUROPÄISCHE PATENTSCHRIFT**

(45) Veröffentlichungstag und Bekanntmachung des Hinweises auf die Patenterteilung:
**31.07.2019 Patentblatt 2019/31**

(21) Anmeldenummer: **12725343.3**

(22) Anmeldetag: **23.05.2012**

(51) Int Cl.:
*A61B 5/145* (2006.01)  *C12Q 1/00* (2006.01)
*G01N 33/487* (2006.01)  *G01N 33/543* (2006.01)

(86) Internationale Anmeldenummer:
**PCT/EP2012/059593**

(87) Internationale Veröffentlichungsnummer:
**WO 2012/160091 (29.11.2012 Gazette 2012/48)**

(54) **SENSORVORRICHTUNG ZUM NACHWEIS EINES ANALYTEN**

SENSOR DEVICE FOR DETECTING AN ANALYTE

DISPOSITIF DE DÉTECTION POUR DÉTECTER UN ANALYTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.05.2011 EP 11167141**

(43) Veröffentlichungstag der Anmeldung:
**09.04.2014 Patentblatt 2014/15**

(73) Patentinhaber:
• **Roche Diabetes Care GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(72) Erfinder:
• **FÜRST, Otto**
**68519 Viernheim (DE)**
• **PILL, Johannes**
**69181 Leimen (DE)**
• **RÖSICKE, Bernd**
**68305 Mannheim (DE)**

(74) Vertreter: **Herzog, Fiesser & Partner**
**Patentanwälte PartG mbB**
**Dudenstrasse 46**
**68167 Mannheim (DE)**

(56) Entgegenhaltungen:
EP-A1- 2 228 004  WO-A1-02/37107
WO-A1-2006/008505  WO-A2-00/04386
DE-A1- 19 501 159  US-A- 4 324 256
US-A- 4 846 937  US-A1- 2003 087 296
US-A1- 2005 123 442  US-A1- 2007 007 148
US-A1- 2010 112 546  US-A1- 2010 155 239

**Beschreibung**

Gebiet der Erfindung

Die Erfindung betrifft eine Sensorvorrichtung zum Nachweis mindestens eines Analyten in einer Körperflüssigkeit gemäß Anspruch 1. Weiterhin betrifft die Erfindung ein Sensorsystem, welches neben mindestens einer erfindungsgemäßen Sensorvorrichtung mindestens ein Lesegerät umfasst. Weiterhin betrifft die Erfindung ein Verfahren zum Nachweis mindestens eines Analyten in einem Fluid gemäß Anspruch 14. Derartige Sensorvorrichtungen, Sensorsysteme und Verfahren werden beispielsweise allgemein in der medizinischen Diagnostik eingesetzt, um in vitro oder in vivo Körperfunktionen zu überwachen. Beispiele sind die kontinuierliche oder diskontinuierliche Überwachung von Analytkonzentrationen in mindestens einer Körperflüssigkeit, wie beispielsweise interstitieller Flüssigkeit, Urin, Speichel oder Blut. Als zu überwachende Analyte kommen beispielsweise einzeln oder in beliebiger Kombination Glucose, Cholesterin, Lactat, mindestens ein Metabolit allgemein oder andere Arten von Analyten und/oder Analytkombinationen in Betracht. Grundsätzlich ist der Begriff des Analyten jedoch weit zu fassen und kann eine oder mehrere chemische Substanzen umfassen. Prinzipiell ist die vorliegende Erfindung jedoch auch auf andere Arten von Analyten und/oder andere Arten von fluiden Medien, beispielsweise andere Arten von Gasen und/oder Flüssigkeiten, und/oder auf andere Einsatzgebiete übertragbar, beispielsweise neben der medizinischen Analytik eine Umweltanalytik, eine Gefahrstoffdetektion, eine Arbeitsplatzüberwachung oder andere Arten von Einsatzgebieten in der Chemie, der Verfahrenstechnik, der Verbrennungstechnik, der Automobiltechnik oder anderen technischen Gebieten.

Stand der Technik

Die Überwachung bestimmter Körperfunktionen, insbesondere die Überwachung einer oder mehrerer Konzentrationen eines oder mehrerer Analyte, spielt bei der Vorbeugung und Behandlung verschiedener Krankheiten eine wesentliche Rolle. Ohne Beschränkung weiterer möglicher Anwendungen wird die Erfindung im Folgenden im Wesentlichen unter Bezugnahme auf eine Blutglucoseüberwachung beschrieben. Grundsätzlich ist die Erfindung jedoch auch auf andere Arten von Analyten und/oder die Überwachung anderer Arten von fluiden Medien übertragbar, sodass beispielsweise, alternativ oder zusätzlich zu einem qualitativen und/oder quantitativen Nachweis eines Analyten in einer Körperflüssigkeit, an ein oder mehrere Analyte in anderen Arten von fluiden Medien überwacht werden können. Neben sogenannten Punktmessungen, bei welchen einem Benutzer gezielt eine Probe einer Körperflüssigkeit wie beispielsweise Urin, Speichel, Blut, interstitielle Flüssigkeit oder andere Arten von Körperflüssigkeit, entnommen und auf die Analytkonzentration untersucht wird, etablieren sich zunehmend auch kontinuierliche in vivo- und/oder in vitro-Messungen. So etabliert sich beispielsweise eine kontinuierliche Glucosemessung im Interstitium (auch als Continuous Monitoring, CM, bezeichnet) in jüngerer Vergangenheit als wichtige Methode zum Management, zur Überwachung und zur Steuerung beispielsweise eines Diabetes-Status.

Mittlerweile kommen dabei in der Regel direkt implantierte elektrochemische Sensorelemente zum Einsatz, welche häufig auch als nadelartige Sensoren (needle-type sensors, NTS) bezeichnet werden. Dabei wird der aktive Sensorbereich direkt an den Messort gebracht, welcher in der Regel im interstitiellen Gewebe angeordnet ist und beispielsweise unter Verwendung eines Enzyms (beispielsweise Glucoseoxidase, GOD, und/oder Glucosedehydrogenase) Glucose in elektrische Ladung umsetzt, die im Verhältnis zur Glucose-Konzentration steht und als Messgröße verwendet werden kann. Beispiele derartiger transkutaner Messsysteme sind in US 6,360,888 B1 oder in US 2008/0242962 A1 beschrieben.

Auch WO2007/130694 beschreibt ein implantierbares Messsystem für einen Analyten wie beispielsweise Glukose. Dieses misst mit Hilfe von Rezeptoren, an die reversibel ein Protein gebunden wird. Mittels einer Galvanischen Zelle wird dann die Konzentration des Analyten ermittelt.

Auf ähnliche Weise wird Glukose auch in US2005/0123442 gemessen.

Aktuelle Continuous Monitoring-Systeme sind somit in der Regel transkutane Systeme. Dies bedeutet, dass der eigentliche Sensor unterhalb der Haut des Benutzers angeordnet ist. Ein Auswerte- und Steuerteil des Systems (im Folgenden auch als Lesegerät bezeichnet) befindet sich jedoch in der Regel außerhalb des Körpers des Benutzers, also außerhalb des menschlichen oder tierischen Körpers. Der Sensor wird dabei in der Regel mittels mindestens eines Insertionsbestecks appliziert, welches exemplarisch ebenfalls in US 6,360,888 B1 beschrieben wird. Auch andere Arten von Insertionsbestecken sind bekannt. Die Tragedauer eines Sensors beträgt in der Regel circa eine Woche. Danach lassen in der Regel Einflüsse, wie beispielsweise ein Enzymverbrauch und/oder eine Abkapselung im Körper, die Sensitivität des Sensors abfallen, sodass ein Ausfall des Sensors zu erwarten ist. Die Verlängerung der Tragedauer stellt somit ein aktuelles Entwicklungsgebiet dar.

Aktuelle Sensoren in der Human- oder Veterinär-Diagnostik basieren somit in der Regel auf dem Prinzip elektrochemischer und/oder auch fotooptischer Prozesse. Dabei wird in der Regel der nachzuweisende Analyt über ein oder mehrere Zwischenschritte mittels spezifischer Reagenzien chemisch umgesetzt. Ein Primärsignal kann dann beispielsweise ein Farbumschlag und/oder ein Ladungsäquivalent sein. Beispielsweise wird, wie oben beschrieben, aus Blut- und/oder interstitieller Flüssigkeit Glucose und Sauerstoff in elektrochemischen Sensoren durch das Enzym GOD zu Gluconolacton und $H_2O_2$ umgesetzt. Die weitere Umsetzung des $H_2O_2$ liefert dann Ladungsäquivalente, beispielsweise zwei Elektronen. Ein Messsignal ist bei einem Überschuss des Enzyms in der Regel nur abhängig von der Glucosekonzentration. Allerdings muss in der Regel genügend Sauerstoff vorhanden sein, und auch die Stabilität der jeweiligen bei dem elektrochemischen Nachweis verwendeten Anode bezüglich einer Redoxreaktion und eines pH-Werts ist erforderlich. Insgesamt führt dies in der Regel bei herkömmlichen Sensorelementen zu einer Alterung, sodass ein kontinuierlicher Betrieb derartiger Sensoren zeitlich begrenzt ist.

Neben derartigen bekannten Sensorelementen zum Analytnachweis sind aus dem Stand der Technik zahlreiche andere Sensorsysteme bekannt, mittels derer eine oder mehrere Analyte nachgewiesen werden können. Beispielsweise sind Halbleitersensoren, beispielsweise sogenannte ISFETs, bekannt, die in der chemischen oder medizinischen Diagnostik eingesetzt werden können.

So ist beispielsweise aus DE 35 13 168 A1 ein Biosensor bekannt, bei welchem in eine einem Messmedium zugewandte oberflächennahe Schicht beispielsweise über einen Gate-Bereich eines Feldeffekttransistors kleine Grundbausteine von Makromolekülen eingefügt werden. Auf diese Weise lassen sich beispielsweise sogenannte Biochips erzielen. Aus DE 38 54 886 T2 ist ein Sensor zur Durchführung von Immunoassays bekannt, der eine Folie aus halbleitenden Polymeren mit einer vorderen und einer rückseitigen Oberfläche umfasst, sowie mehrere elektroleitfähige Flächen. Weiterhin wird eine Immunoassay-Sensorzelle mit einem offenen oberen und unteren Ende mit einem Doppelkammereinsatz beschrieben.

Derartige bekannte Halbleiterstrukturen oder organische Halbleiterstrukturen basieren in der Regel darauf, dass auf die halbleitenden Eigenschaften eines Substratmaterials durch chemische Prozesse, beispielsweise durch Bindung bzw. Besetzung von Bindungsstellen, Einfluss genommen wird. Dabei erfolgt eine möglichst innige Verbindung eines biologischen Teils, beispielsweise Analyt-affiner Strukturen wie Proteinen oder Nukleinsäuren, mit dem Halbleiter. In DE 35 13 168 A1 wird beispielsweise einem Problem einer unspezifischen Bindung mittels eines Differenzverfahrens auf Basis halbleitender Polymere im differentiellen Modus begegnet.

In M. A. Rahman et al.: Electrochemical Sensors Based on Organic Conjugated Polymers, Sensors 2008, 8, 118-141 werden Biosensoren auf Basis halbleitender Polymere beschrieben, bei denen beispielsweise eine kompetitive immunologische Reaktion oder auch eine direkte Hybridisierung erfolgt. Dabei werden verschiedene Konzepte beschrieben und miteinander verglichen.

Aus B. Em: ISFET, Theory and Practice, IEEE Sensor Conference Toronto, October 2003 sind verschiedene Ausführungsformen sogenannter Ionen-sensiver Feldeffekttransistoren bekannt. Unter anderem wird dabei ein auf einem ISFET basierender Operationsverstärker beschrieben.

Aus dem Stand der Technik ist weiterhin eine Mehrzahl von Konzepten zur Bestimmung von Kohlendioxid bekannt. So wird beispielsweise in US 4,324,256 eine Vorrichtung zur transkutanen Messung eines Kohlendioxid-Partialdrucks mittels einer pH-sensitiven Messelektrode hinter einer $CO_2$-permeablen Membran beschrieben. Da der pH-Wert eine Funktion des $CO_2$-Partialdrucks ist, lässt sich mittels der pH-Elektrode die Kohlendioxid-Konzentration bestimmen. Analog wird in WO 2006/008505 A1 eine physiologische Sensorvorrichtung zur Messung eines $CO_2$-Partialdrucks beschrieben. Wiederum ist eine für Kohlendioxid permeable Membran vorgesehen, welche eine Kammer mit zwei Elektroden abschließt. Auch WO 00/04386 A2 beschreibt einen Kohlendioxid-Sensor mit einer geschlossenen Kammer, der eine im Wesentlichen wasserdichte Wand und eine für Kohlendioxid permeable Membran aufweist. In der Kammer sind zwei Elektroden vorgesehen. In US 4,846,937 wird ein Verfahren zur Bestimmung eines Kohlendioxid-Gehalts in gasförmigen oder flüssigen Proben offenbart. Wiederum ist eine semipermeable Sensormembran vorgesehen, um einen Messraum mit einer Sensorelektrode und einem Elektrolyten abzuschließen. US 2010/0155239 A1 beschreibt eine planare $CO_2$-Sensorvorrichtung, die eine Elektrode mit einer ionenselektiven Schicht, eine Elektrolytschicht und eine äußere Schicht aufweist.

In EP 2 228 004 A1 wird ein implantierbarer, selbst-kalibrierender Biosensor mit einem mikrofluidischen Schaltkreis beschrieben. Dabei sind zwei oder mehr Elektroden auf einer Außenseite des mikrofluidischen Schaltkreises angeordnet. Weiterhin sind poröse biochemische Sensormaterialien vorgesehen, die nach einer Implantation mit einem Körpergewebe in Kontakt stehen.

DE 19 591 159 A1 beschreibt einen Mikrosensor zur Bestimmung der Konzentration von Glucose und anderen Analyten in Flüssigkeit auf Basis einer Affinitäts-Viskosimetrie. Dabei wird eine Dialysekammer verwendet, die ein in Wasser dispergiertes Polymersystem enthält. Weiterhin ist ein druckempfindlicher, volumenempfindlicher oder strömungsempfindlicher Signalgeber vorgesehen, welcher elektrische Signale entsprechend der Viskosität liefert.

Die bekannten Sensorkonzepte für diagnostische und nicht-diagnostische Anwendungen sind mit einer Vielzahl von technischen und medizinischen Herausforderungen konfrontiert. Kontinuierlich messende, diagnostische Sensoren, wie beispielsweise die oben beschriebenen CM-Sensoren, kommen, im Gegensatz zu den oben genannten Halbleiterstrukturen, hingegen meist im subkutanen Umfeld, also in einem feuchten Milieu, zum Einsatz. Eine technische Herausforderung besteht dabei insbesondere darin, dass der Analyt, beispielsweise Glucose, aus dem Interstitium oder der Körperflüssigkeit, in den Sensor gelangen muss. Bei üblichen Sensorchemien, welche in der Regel als Testchemie aufgebaut sind, muss beispielsweise der Analyt aus dem Interstitium durch eine spezifische sensorseitige Membran und einem geeigneten Konzentrationsgradienten zwecks Erzielung einer diffusionskontrollierten Reaktion in ein Testchemiefeld eindiffundieren. Dabei wird die Umsetzung abhängig von einer Enzym-Menge bzw. Enzym-Aktivität. Durch die Membran wird weiterhin vermieden, dass parasitäre Effekte auftreten, beispielsweise durch Fremdmoleküle, insbesondere durch andere redoxaktive Substanzen, und das Messsignal überlagern. Ferner soll die Membran umgekehrt auch verhindern, dass Substanzen aus dem Testchemiefeld und dem Sensormaterial heraus in den Körper gelangen.

Die Aufbereitung der Messsignale erfolgt bei üblichen elektrochemischen Sensoren in der Regel durch nachfolgende Signalverstärkung, beispielsweise einen Ladungsstrom und/oder ein optisches Signal, eine Analog-/Digital-Wandlung und eine Verarbeitung mittels eines geeigneten Mikrocontrollers. Die Verarbeitungselektronik besteht in der Regel aus klassischen Silicium-Komponenten. Die dem Sensor nachfolgende Signal-Verarbeitungseinheit ist, beispielsweise aufgrund herstellrelevanter Gründe, meist über Stecksysteme vom eigentlichen Sensor getrennt.

In den meisten vorgenannten Sensorsystemen limitiert in der Regel eine Alterung des Sensors dessen Einsatzdauer. Auch eine mechanische Beanspruchung des relativ komplizierten Sensoraufbaus, sei es bei herkömmlichen elektrochemischen Analytsensoren für den Einsatz in der Medizintechnik oder bei den beschriebenen Halbleitersensoren, kann limitierend sein. Im Fall subkutaner Applikationen ist daher ein häufigeres Wechseln, also ein häufigerer invasiver Eingriff, nötig, beispielsweise ein Wechsel im Wochenrhythmus.

Im Falle von Halbleitersensoren und einer Einflussnahme auf Halbleitereigenschaften, beispielsweise von Silicium, sind zum Andocken des nachzuweisenden Analyten in der Regel spezifische Bindungsstellen mit hohen Aktivierungsenergien erforderlich, was im direkten Kontakt zum feuchten Milieu in der Regel schwer möglich ist.

Weiterhin erfolgen eine Sensorherstellung und eine Herstellung einer Vorrichtung zur elektronischen Nachverarbeitung in der Regel in unterschiedlichen Herstellprozessen. So ist in der Regel zu beachten, dass, beispielsweise bei den oben beschriebenen Halbleitersensoren, unterschiedliche Herstellprozesse kombiniert werden müssen, beispielsweise chemische Herstellprozesse und klassische Halbleiterprozesse. Eine Integration und eine Baugrößen- und Herstellkostenreduktion ist daher in der Regel nur begrenzt möglich.

Eine weitere technische Herausforderung bei bekannten Sensorelementen besteht in der Regel darin, dass eine klassische Messwerterfassung zumeist durch eine serielle Umsetzung von einem Detektionsschritt über eine mehrstufige chemische Umwandlung und dann über eine Signalverstärkung in mehreren Stufen erfolgt, gefolgt oder begleitet in der Regel von mindestens einer Analog-Digital-Wandlung und/oder einer Anzeige der Messwerte. Dabei wird in der Regel, bedingt durch das mehrfache serielle Umsetzen der Messgröße, ein Rauschen der jeweiligen Vorgängerstufe in einer nachfolgenden Stufe verstärkt. Einem Grundprinzip der Signalverarbeitung, nämlich das primäre Signal in der ersten Stufe möglichst hoch zu verstärken, kann daher in vielen bekannten Sensoren nicht adäquat entsprochen werden.

Eine weitere technische Herausforderung besteht darin, dass elektrochemische Sensoren für eine Humandiagnostik, insbesondere für einen subkutanen Einsatz, in der Regel steril ausgestaltet werden müssen. Eine derartige Sterilisation erfolgt in der Regel mittels eines Strahlensterilisationsverfahrens, da andere Methoden, wie beispielsweise thermische und/oder chemische Sterilisationsmethoden, in der Regel die messwertrelevante Reaktionschemie zerstören würden. Die gegebenenfalls im Sensor integrierten Elektronikkomponenten, beispielsweise auf Siliciumbasis, werden jedoch in vielen Fällen durch die benötigten hohen Strahlendosen, beispielsweise Strahlendosen um 25 kGray oder über 25 kGray, geschädigt und müssten somit während des Sterilisationsprozesses vom Sensor getrennt werden, was letztendlich eine Integration erschwert und/oder unmöglich macht.

Aufgabe der Erfindung

Es ist daher eine Aufgabe der vorliegenden Erfindung, eine Sensorvorrichtung zum Nachweis mindestens eines Analyten bereitzustellen, welche die oben beschriebenen Nachteile zumindest weitgehend vermeidet. Insbesondere soll die Sensorvorrichtung eine hochintegrierte Bauweise ermöglichen, optional mit bereits teilweise integrierter Elektronik. Weiterhin soll die erfindungsgemäße Lösung insbesondere einen Einsatz im Rahmen subkutaner Analytsensoren ermöglichen. Dazu sollte der Prozess zum Nachweis der Konzentration des Analyten möglichst lange ablaufen können.

Offenbarung der Erfindung

Diese Aufgabe wird durch eine Sensorvorrichtung und ein Verfahren zum Nachweis mindestens eines Analyten in einem Fluid mit den Merkmalen der unabhängigen Ansprüche gelöst. Vorteilhafte Weiterbildungen der Erfindung, welche einzeln oder in Kombination realisierbar sind, sind in den abhängigen Ansprüchen dargestellt.

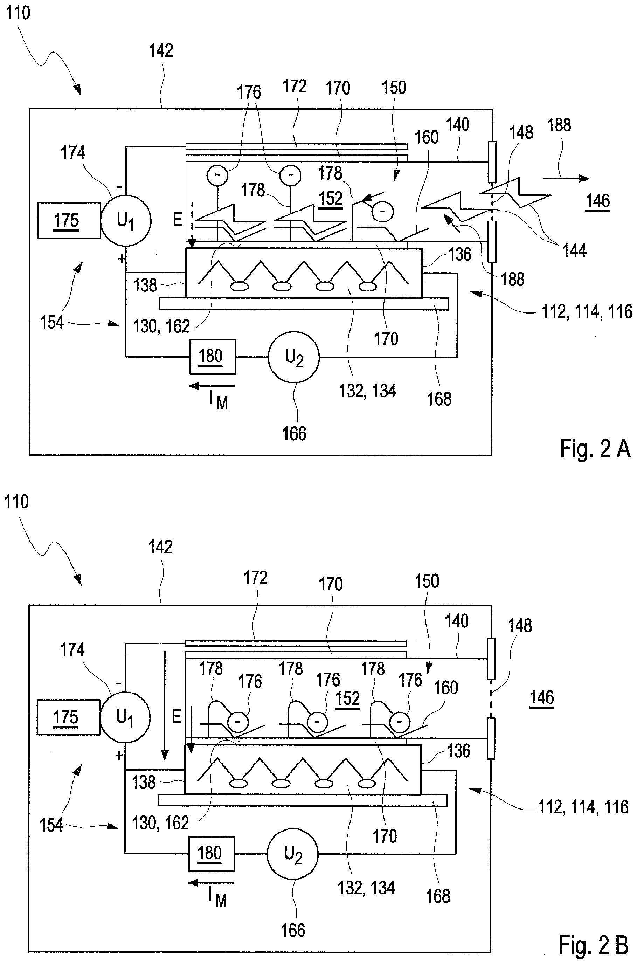

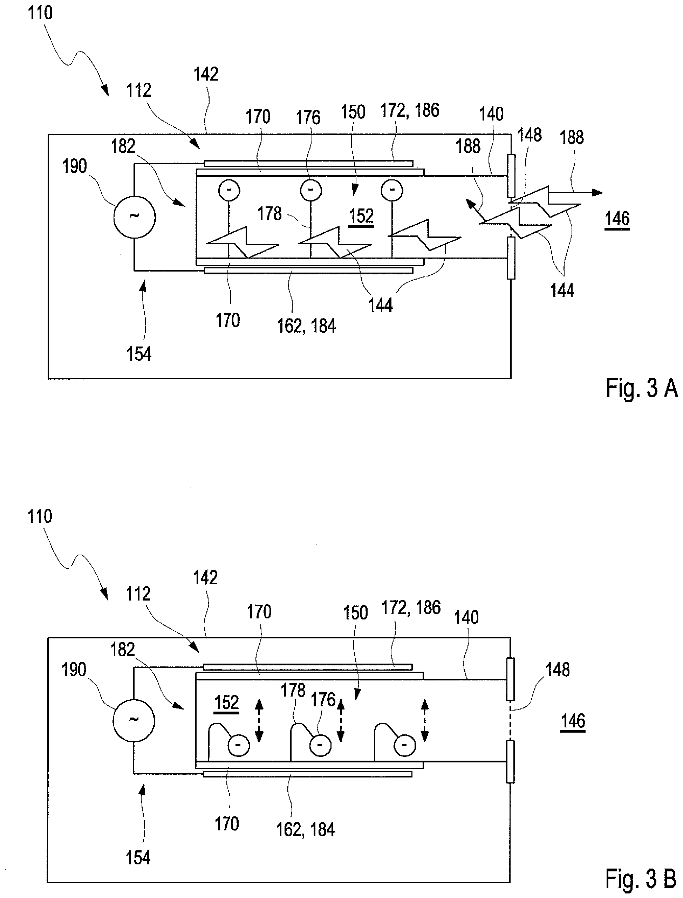

Insbesondere die Figuren 2A, 2B, 3A und 3B und deren begleitender Text in der Beschreibung zeigen die Erfindung.

In einem ersten Aspekt der vorliegenden Erfindung wird eine Sensorvorrichtung zum Nachweis mindestens eines Analyten in einem Fluid vorgeschlagen. Wie unten noch näher ausgeführt wird, kann die Sensorvorrichtung insbesondere zur vollständigen oder teilweisen Insertion in ein Körpergewebe eines Benutzers eingerichtet sein, beispielsweise in ein subkutanes interstitielles Gewebe.

Der Analytnachweis erfolgt quantitativ, ist also ein Nachweis mindestens einer Analytkonzentration. Bei dem mindestens einen Analyten kann es sich grundsätzlich um einen oder mehrere beliebige Analyten handeln. Besonders bevorzugt ist es, wenn der mindestens eine nachzuweisende Analyt ausgewählt ist aus der Gruppe bestehend aus: Glucose; Cholesterin; Lactat oder Kombinationen der genannten Substanzen und/oder anderer Primärsubstanzen und/oder Metaboliten. Der Analytnachweis kann insbesondere spezifisch erfolgen. Bei dem mindestens einen Fluid kann es sich grundsätzlich um ein beliebiges Gas und/oder eine beliebige Flüssigkeit handeln. Besonders bevorzugt ist es, wenn das Fluid eine Flüssigkeit ist oder umfasst, insbesondere eine Körperflüssigkeit, beispielsweise Speichel, Urin, Blut, interstitielle Flüssigkeit oder eine andere Körperflüssigkeit. Der Nachweis kann insbesondere in einem Körpergewebe erfolgen.

Die Sensorvorrichtung umfasst mindestens eine geschlossene Detektorkammer und mindestens einen elektrischen Sensor mit mindestens einer Sensorelektrode. Der elektrische Sensor ist vorzugsweise elektrisch durch die Detektorkammer, also insbesondere durch den Inhalt der Detektorkammer, beeinflussbar. Die Detektorkammer ist derart mit dem Fluid in Verbindung bringbar, beispielsweise vollständig oder teilweise in ein Körpergewebe mit dem Fluid insertierbar, dass der Analyt in die Detektorkammer eindringen kann, vorzugsweise reversibel. Unter einem reversiblen Eindringen ist dabei ein Vorgang zu verstehen, bei welchem der Analyt in die Detektorkammer eindringen und die Detektorkammer auch wieder verlassen kann, beispielsweise aufgrund von Diffusionsprozessen. Die Detektorkammer umfasst mindestens eine Detektorsubstanz, wobei die Detektorsubstanz eingerichtet ist, um mindestens eine elektrische Eigenschaft des elektrischen Sensors, insbesondere mindestens eine elektrische Eigenschaft der Sensorelektrode und/oder der Detektorkammer, in Abhängigkeit von einer Konzentration des Analyten in der Detektorkammer zu beeinflussen. In der Detektorkammer ist mindestens ein Rezeptor aufgenommen, der eingerichtet ist, um den Analyten reversibel zu binden. Die Detektorsubstanz ist derart ausgestaltet, dass die dielektrischen Eigenschaften der Detektorsubstanz abhängig sind von der Konzentration des Analyten in der Detektorkammer.

Unter einer Detektorkammer ist dabei grundsätzlich ein beliebiger Raum zu verstehen, welcher vollständig oder teilweise mit der Detektorsubstanz und/oder anderen Substanzen gefüllt werden kann, sodass diese an einem Verlassen der Detektorkammer gehindert werden können. Insbesondere kann die Detektorkammer, wie unten noch näher beschrieben wird, ein Kompartiment umfassen, in welchem ein fluides, insbesondere flüssiges, Medium vorliegt. Dieses fluide Medium kann verschieden von dem Fluid sein, in welchem der Analyt nachgewiesen werden soll, kann jedoch auch vollständig oder teilweise gleich zu diesem Fluid ausgestaltet sein oder eine oder mehrere gleiche Bestandteile enthalten.

Die Detektorkammer kann eine oder mehrere Kammerwände aufweisen, insbesondere Kammerwände, welche vollständig oder teilweise aus Kunststoff hergestellt sind. Unter einer geschlossenen Kammer ist dabei, im Gegensatz zu üblichen Reaktionsgefäßen, eine Kammer zu verstehen, welche verhindert, dass die Detektorsubstanz die Kammer verlässt. Beispielsweise kann die Detektorkammer derart ausgestaltet sein, dass die Detektorsubstanz nicht in ein umgebendes Körpergewebe gelangen kann. Insbesondere kann die Detektorkammer allseitig umschlossen sein, beispielsweise von mindestens einem Gehäuse, insbesondere einem Gehäuse mit mindestens einer Membran. Auch andere Arten der Ausgestaltung der Detektorkammer sind jedoch alternativ oder zusätzlich möglich, welche bewirken, dass die Detektorsubstanz die Detektorkammer nicht oder nur in einem unwesentlichen Maßstab verlassen kann, beispielsweise indem in einem implantierten oder insertierten (beide Begriffe werden im wesentlichen synonym verwendet) Zustand über einen Zeitraum von mindestens einer Woche nicht mehr als 1%, vorzugsweise nicht mehr als 0,1 % und besonders bevorzugt nicht mehr als 0,01 % oder sogar nicht mehr als 0,001 % der Gesamtmenge der Detektorsubstanz die Detektorkammer verlässt. Beispielsweise kann die Geschlossenheit der Kammer derart realisiert werden, dass diese ein Medium umfasst, in welches der Analyt eindringen kann, in welchem jedoch die Detektorsubstanz immobilisiert ist. Beispielsweise kann es sich hierbei um ein poröses Medium handeln, welches von dem Fluid und/oder dem fluiden Medium und/oder dem Analyten durchdringbar ist, in welchem jedoch die Detektorsubstanz eingeschlossen und/oder immobilisiert ist, so dass diese das poröse Medium nicht verlassen kann. Beispielsweise kann es sich um einen porösen Kunststoff handeln. Weiterhin sollte die Geschlossenheit der Kammer, insbesondere der Detektorkammer, insbesondere derart realisiert werden, dass elektrische Komponenten, beispielsweise Elektroden, insbesondere gegeneinander elektrisch isoliert sind, insbesondere derart, dass das Fluid und/oder das fluide Medium keine elektrischen Kurzschlüsse verursachen kann.

Die Detektorkammer kann insbesondere langgestreckt ausgestaltet sein, beispielsweise indem die Kammer eine Längserstreckung aufweist, mit einer Dimension, welche eine Erstreckung der Kammer quer zu der Längserstreckung um mindestens einen Faktor 2, vorzugsweise um mindestens einen Faktor 5 und besonders bevorzugt um mindestens einen Faktor 10 übersteigt. Die Detektorkammer kann insbesondere einen Innenraum mit einem Volumen aufweisen, welches zwischen $0,1 \text{ mm}^3$ und $10 \text{ mm}^3$ liegt, vorzugsweise zwischen $0,5 \text{ mm}^3$ und $5 \text{ mm}^3$ liegt und besonders bevorzugt zwischen $0,8 \text{ mm}^3$ und $2,2 \text{ mm}^3$ liegt. Insbesondere kann eine Kammer mit einem Volumen von $10 \text{ mm} \cdot 10$

mm · 0,01 mm = 1 mm$^3$ verwendet werden.

Ein optionales Gehäuse, welches die Detektorkammer allseitig oder zumindest abschnittsweise gegenüber einem umgebenden Medium, beispielsweise einem Körpergewebe, abschließen kann, kann beispielsweise vollständig oder teilweise aus einem Kunststoffmaterial gefertigt sein. Wie unten noch näher beschrieben wird, kann auch die mindestens eine Sensorelektrode zumindest teilweise Bestandteil des Gehäuses der Detektorkammer sein.

Weiterhin kann die Detektorkammer, alternativ oder zusätzlich, wie unten ebenfalls noch näher beschrieben wird, mindestens eine Membran umfassen, insbesondere mindestens eine semipermeable Membran, wobei die Membran ein reversibles Eindringen des Analyten ins Innere der Detektorkammer, umfassend insbesondere auch ein Austreten aus der Detektorkammer, ermöglicht, wobei die Membran jedoch verhindert, dass die mindestens eine Detektorsubstanz aus der Detektorkammer entweicht.

Die optionale Membran kann insbesondere als Durchlass spezifischer Teilchen in die Detektorkammer hinein und/oder aus der Detektorkammer heraus fungieren. Alternativ oder zusätzlich kann diese als Barriere zum Zweck einer zeitlichen Steuerung des Nachweisprozesses fungieren. Insbesondere kann die Membran gasdicht ausgestaltet sein. Die Membran kann insbesondere derart ausgestaltet sein, dass diese lediglich in einer Flüssigkeit, beispielsweise in wässriger Lösung, ihre permeablen oder semipermeablen Eigenschaften entfaltet.

Unter einer Elektrode, insbesondere der Sensorelektrode, ist im Rahmen der vorliegenden Erfindung eine Oberfläche zu verstehen, welche elektrische Ladungen aufnehmen und/oder transportieren kann. Beispielsweise kann diese Elektrode eine Oberfläche eines zur Aufnahme von elektrischen Ladungen eingerichteten Dielektrikums und/oder eine Oberfläche eines elektrisch leitfähigen oder elektrisch halbleitenden Elements sein, welches anorganischer und/oder organischer Natur sein kann. Alternativ oder zusätzlich kann die Elektrode auch mindestens eine leitfähige Schicht und/oder deren Oberfläche aufweisen, beispielsweise eine metallische Schicht und/oder eine organische leitfähige Schicht. Diese organische leitfähige Schicht kann insbesondere auf einem Substrat aufgebracht sein, beispielsweise einem Isolatorsubstrat, wobei jedoch vorzugsweise die leitfähige Schicht auf einem elektrisch leitfähigen Element und/oder elektrisch halbleitenden Element aufgebracht ist, beispielsweise einem organischen oder anorganischen Halbleiter oder Leiter. Die Elektrode muss somit nicht notwendigerweise eine leitfähige Schicht oder ein elektrisch leitfähiges Element selbst sein, sondern kann grundsätzlich auch eine Oberfläche eines elektrisch isolierenden oder elektrisch leitfähigen oder halbleitenden Elements sein. Die Sensorelektrode kann bevorzugt beispielsweise eine Gate-Elektrode eines Feldeffekttransistors sein und/oder kann eine Elektrode und/oder eine Platte eines Kondensators sein.

Der elektrische Sensor soll vorzugsweise, wie oben ausgeführt, elektrisch durch die Detektorkammer beeinflussbar sein. Dies schließt jegliche elektrische Beeinflussung ein, einschließlich einer Polarisierung, einem Aufbringen von Ladungen, einer Veränderung eines Potenzials oder eine Veränderung anderer elektrischer und/oder dielektrischer Eigenschaften. Beispielsweise kann die mindestens eine Sensorelektrode des elektrischen Sensors durch die Detektorkammer beeinflussbar sein, beispielsweise indem ein Potenzial der Sensorelektrode durch die Detektorkammer beeinflusst wird. Alternativ oder zusätzlich können auch direkt oder indirekt Ladungen auf die mindestens eine Sensorelektrode aufgebracht werden. Verschiedene Arten der Beeinflussung werden unten noch näher beschrieben. Eine Beeinflussung durch die Detektorkammer schließt eine Beeinflussung durch den Inhalt der Detektorkammer, insbesondere eine Beeinflussung durch die mindestens eine Detektorsubstanz und/oder Teile derselben und/oder durch den Analyten oder Kombinationen der genannten Möglichkeiten ein. Die Sensorelektrode kann Bestandteil der Detektorkammer sein oder kann auf andere Weise mit der Detektorkammer in Verbindung stehen, sodass diese vorzugsweise durch die Detektorkammer elektrisch beeinflussbar ist. Beispielsweise kann die Sensorelektrode Bestandteil eines Gehäuses der Detektorkammer sein, beispielsweise einer Kammerwand der Detektorkammer. Die Sensorelektrode kann beispielsweise direkt dem in der Detektorkammer aufgenommenen Medium, insbesondere der Detektorsubstanz, ausgesetzt sein. Alternativ oder zusätzlich kann zwischen der Sensorelektrode und dem Inneren der Detektorkammer, wie unten noch näher beschrieben wird, mindestens ein Zwischenelement eingebracht sein, insbesondere mindestens eine Isolatorschicht und/oder mindestens eine Schutzschicht. Besonders bevorzugt ist es jedoch, wenn die Elektrode unmittelbar einem Innenraum der Detektorkammer ausgesetzt ist. Physikalisch betrachtet kann somit das Innere der Detektorkammer, beispielsweise ein in der Detektorkammer aufgenommenes Medium, selbst Bestandteil der Sensorelektrode sein.

Unter einer Detektorsubstanz ist dementsprechend eine grundsätzlich beliebige Substanz zu verstehen, welche eingerichtet ist, um mindestens eine elektrische Eigenschaft des elektrischen Sensors, insbesondere mindestens eine elektrische Eigenschaft der Sensorelektrode, in Abhängigkeit von einer Konzentration des Analyten in der Detektorkammer zu beeinflussen, vorzugsweise Analyt-spezifisch. Beispielsweise kann die Detektorsubstanz derart eingerichtet und/oder derart in der Detektorkammer aufgenommen sein, dass der Analyt eine Ladungsverteilung in der Detektorsubstanz in der Detektorkammer und/oder dielektrische Eigenschaften der Detektorsubstanz in der Detektorkammer ändert. Beispielsweise können sich durch den Analyten, vorzugsweise reversibel, eine Permittivität und/oder eine Permittivitätszahl, auch Dielektrizitätszahl genannt, jeweils beispielsweise als reale oder auch als komplexe Größen erfassbar, der Detektorsubstanz ändern, lokal oder auch gemittelt über die Detektorkammer oder einen Bereich derselben. Die Sensorvorrichtung mit der Sensorelektrode kann eingerichtet sein, um in Alleinstellung der Sensorelektrode und/oder in Zusammenwirken mit einem oder mehreren weiteren Elementen, wie beispielsweise mindestens einer Gegenelektrode, diese Analyt-bedingten Änderungen der Ladungsverteilung und/oder diese analyt-bedingten Änderungen der dielektrischen Eigenschaften der Detektorsubstanz zu erfassen, beispielsweise durch Erzeugung mindestens eines Signals und/oder durch mindestens eine Signaländerung. Die Erfassung kann statisch und/oder dynamisch erfolgen. Beispielsweise kann die Detektorsubstanz derart eingerichtet sein, dass dielektrische Eigenschaften der Detektorsubstanz durch Anwesenheit des Analyten beeinflusst werden, insbesondere verändert werden, wobei die Sensorvorrichtung beispielsweise eingerichtet sein kann, um durch diese Beeinflussung der dielektrischen Eigenschaften hervorgerufene Beeinflussungen eines elektrischen Wechselfeldes zu erfassen. Auch andere Messtechniken sind jedoch alternativ oder zusätzlich einsetzbar.

Die Detektorkammer ist, wie oben beschrieben, derart mit dem Fluid in Verbindung bringbar, dass der Analyt in die Detektorkammer eindringen kann. Dieses Eindringen des Analyten kann unidirektional oder, vorzugsweise, bidirektional und/oder reversibel erfolgen. Besonders bevorzugt ist es, wenn die Detektorkammer eingerichtet ist, um ein Konzentrationsgleichgewicht, insbesondere ein diffusionsgetriebenes Konzentrationsgleichgewicht, einer Analytkonzentration innerhalb der Detektorkammer und außerhalb der Detektorkammer zu ermöglichen. Die Diffusion des Analyten kann insbesondere selektiv erfolgen. Beispielsweise kann die Detektorkammer derart eingerichtet sein, dass der Analyt in die Detektorkammer eindringen kann, wohingegen ein Austreten anderer Substanzen aus der Detektorkammer in die Umgebung, beispielsweise in das Fluid und/oder das Körpergewebe, vorzugsweise zumindest weitgehend verhindert wird. Zu diesem Zweck kann die Detektorkammer, wie unten noch näher beschrieben wird, beispielsweise mindestens eine Membran umfassen, welche einen Durchtritt des Analyten ermöglicht, welche jedoch vorzugsweise undurchdringlich ist für andere Bestandteile des Fluids und/oder die Detektorsubstanz. Unter einer Membran ist dabei ein einschichtiges oder auch mehrschichtiges Element zu verstehen, welches einen Durchtritt des mindestens einen Analyten ermöglicht, vorzugsweise einen spezifischen Durchtritt und/oder selektiven Durchtritt, welcher lediglich für den mindestens einen nachzuweisenden Analyten ermöglicht ist, nicht jedoch für andere Stoffe, beispielsweise andere Bestandteile des Fluids oder lediglich für eine sehr begrenzte Anzahl weiterer Stoffe. Die Membran kann vorzugsweise als zumindest teilweise poröses, für den Analyten durchlässiges Material ausgestaltet sein, beispielsweise ein poröses Kunststoffmaterial. Das poröse Material kann beispielsweise Poren umfassen, welche von ihrer Größe her an einen Durchtritt des Analyten angepasst sind, jedoch Moleküle, welche größer sind als der Analyt, an einem Durchtritt hindern.

Beispielsweise kann die Membran mindestens eine Membranfolie aufweisen. Die Membran kann beispielsweise eine semipermeable Membran sein, welche undurchlässig ist für die mindestens eine Detektorsubstanz und/oder Bestandteile derselben. Insbesondere kann die Membran folienartig aufgebaut sein, beispielsweise mit einer Dicke, welche eine laterale Ausdehnung der Membran um mindestens einen Faktor 10, vorzugsweise um mindestens einen Faktor 100 oder mehr unterschreitet, insbesondere wenn keine GlucoseSättigung vorliegt, beispielsweise wenn ein retardierter Transport gewünscht ist. Beispielsweise kann die Membran eine Dicke von weniger als 500 $\mu$m aufweisen, insbesondere eine Dicke von weniger als 200 $\mu$m, besonders bevorzugt eine Dicke von weniger als 100 $\mu$m und insbesondere eine Dicke von maximal 50 $\mu$m. Membranen, beispielsweise auf einen Träger aufgebrachte Membranschichten, können typischerweise flächenbezogene Massen von beispielsweise 5 bis 20 mg/cm$^2$ aufweisen. Bei einer Dichte von ungefähr 1 g/cm$^3$ impliziert dies Membrandicken von beispielsweise 50 $\mu$m bis 100 $\mu$m. Alternativ kann die Membran auch eine Dicke von 1 bis 15 $\mu$m aufweisen. Die Sensorvorrichtung gemäß der vorliegenden Erfindung verbraucht in der Regel vorzugsweise keine Glucose, weshalb im Wesentlichen eine schnelle Einstellung des Gleichgewichts wünschenswert ist. Daher ist die Membran bevorzugt möglichst dünn, beispielsweise wenige Mikrometer dick, bevorzugt 1 bis 5 $\mu$m dick, wobei die Membran bevorzugt noch stabil sein sollte. Als Membranmaterialien kommen insbesondere Kunststoffmaterialien und/oder Polymere und/oder Meterialien auf organischer Basis, in Betracht, wobei jedoch beliebige anorganische und/oder organische Materialien eingesetzt werden können. Insbesondere können ein oder mehrere hydrophile Materialien als Membranmaterial eingesetzt werden. Die Materialien können als fertige Membran oder in situ, insbesondere als Vorläufermaterial, beispielsweise gelöst in mindestens einem Lösemittel, appliziert und sodann zu einer Membran geformt werden. Besonders bevorzugt ist die Verwendung eines oder mehrerer der folgenden Materialien als Membranmaterialien: eine Cellulose und/oder ein Cellulosederivat, insbesondere eine Nitrocellulose und/oder beispielsweise Celluloseacetat und/oder beispielsweise Ethylcellulose; ein Polysulfon, beispielsweise Polyethylensulfon; ein fluoriertes Polymer, beispielsweise Polyvinylidenfluorid oder FEP (fluorinated ethylene propylene); ein Polyetheretherketon (PEEK); ein Polyacrylonitril (PAN); ein Polycarbonat (PC); ein, beispielsweise hydrophiles, Polyurethan (PU); ein Nylon; eine regenerierte Cellulose. Alternativ oder zusätzlich zu den genannten Materialien und/oder Kombinationen derselben lassen sich für die mindestens eine Membran auch andere Materialien einsetzen. Umfasst die Membran ein poröses Material, so weist die Membran vorzugsweise eine mittlere Porengröße $d_{50}$ zwischen 1 $\mu$m und 10 $\mu$m, vorzugsweise zwischen 2 $\mu$m und 5 $\mu$m, auf. Prinzipiell kann die Membran auch ohne Poren sein, insbesondere ohne Poren, welche von dem Membranmaterial als solche differenziert betrachtet werden könnten. Beispielsweise kann der Analyt, insbesondere Glucose, diffusionskontrolliert durch die dünnen Membrane, welche bevorzugt nur wenige $\mu$m dick sind und/oder im Wesentlichen porenfrei sind, dringen, beispielsweise derart, dass bevorzugt keine Glucosesättigung auftreten kann. Bei der erfindungsgemäßen Sensorvorrichtung, welche bevorzugt keinen Glucoseverbrauch aufweist, ist beispielsweise eine rasche Gleichgewichtseinstellung, beispielsweise durch Poren und/oder über eine rasche Diffusion, beispielsweise durch eine porenfreie Membran, bevorzugt. Insbesondere sollte die Membran einen hohen "effektiven DiffusionsKoeffizienten" für den Analyten, insbesondere Glucose, aufweisen und vorzugsweise unerwünschte Diffusion anderer Substanzen verhindern und vorzugsweise mechanische Stabilität aufweisen. Daher sollte die Membran vorzugsweise keine Poren aufweisen oder selektive Poren und/oder sollte einen hohen Diffusionskoeffizienten für den Analyten, insbesondere für Glucose, aufweisen.

Alternativ oder zusätzlich zu der Verwendung mindestens einer Membran, welche zwischen einem Inneren der Detektorkammer und einem Außenraum, beispielsweise dem äußeren Fluid und/oder dem Körpergewebe, angeordnet ist, kann die Detektorkammer auch eine oder mehrere weitere Elemente umfassen, welche ermöglichen, dass der Analyt in die Detektorkammer eindringt, wohingegen vorzugsweise ein Austritt von Detektorsubstanz aus der Detektorkammer in das umgebende Fluid verhindert wird.

Wie oben beschrieben, ist die Detektorsubstanz eingerichtet, um mindestens eine elektrische Eigenschaft des elektrischen Sensors, insbesondere mindestens eine elektrische Eigenschaft der Sensorelektrode, zu beeinflussen. Dies kann beispielsweise eine direkte Beeinflussung der Sensorelektrode sein, beispielsweise indem eine Ladungsträgerdichte und/oder eine Ladung und/oder eine Leitfähigkeit der Sensorelektrode und/oder ein Strom durch die Sensorelektrode direkt oder indirekt beeinflusst werden. Alternativ oder zusätzlich kann diese Beeinflussung jedoch auch eine Beeinflussung einer Anordnung sein, welche die Sensorelektrode und/oder einen anderen Bestandteil des elektrischen Sensors umfasst.

Unter einer Beeinflussung der mindestens einen elektrischen Eigenschaft des elektrischen Sensors in Abhängigkeit von einer Konzentration des mindestens einen Analyten in der Detektorkammer, beispielsweise in einem in der Detektorkammer aufgenommenen Fluid, insbesondere einer Flüssigkeit, ist eine Eigenschaft zu verstehen, bei welcher sich die mindestens eine Eigenschaft stetig oder unstetig zumindest innerhalb eines gewissen Konzentrationsbereichs mit der Konzentration des Analyten ändert. Besonders bevorzugt ist es, wenn diese Beeinflussung derart erfolgt, dass ein eindeutiger, vorzugsweise linearer, Zusammenhang, insbesondere eine Korrelation, beispielsweise in Form einer Funktion oder Abbildung zwischen der mindestens einen elektrischen Eigenschaft und der Analytkonzentration besteht. Beispielsweise kann ein Zusammenhang bestehen, durch welchen jeweils einem Wert der elektrischen Eigenschaft ein Wert der Analytkonzentration zugeordnet wird, insbesondere genau ein diskreter Wert. Die Beeinflussung kann durch den Analyten selbst erfolgen und/oder durch die Detektorsubstanz und/oder durch eine Wechselwirkung zwischen dem Analyten und der Detektorsubstanz, welche ohne oder auch mit vollständiger oder teilweiser Umsetzung des Analyten erfolgen kann, wie unten noch näher ausgeführt wird.

Die Sensorvorrichtung gemäß der obigen Beschreibung kann auf verschiedene Weisen, welche beliebig miteinander kombinierbar sind, vorteilhaft weitergebildet werden. Wie oben ausgeführt, kann die Detektorkammer insbesondere eingerichtet sein, um einen reversiblen Austausch des Analyten zwischen einem Innenraum der Detektorkammer und einer Umgebung der Detektorkammer, insbesondere einem umgebenden Körpergewebe, zu ermöglichen. Wie oben ausgeführt, kann dies auf verschiedene Weisen erfolgen.

Die Detektorkammer, insbesondere mindestens ein Gehäuse der Detektorkammer, welches einen Innenraum der Detektorkammer umschließt, vorzugsweise vollständig, kann insbesondere mindestens eine Membran aufweisen, wie oben ausgeführt, insbesondere mindestens eine Analyt-spezifische Membran. Diese Membran kann insbesondere eingerichtet sein, um das Eindringen des Analyten in die Detektorkammer reversibel zu ermöglichen und um vorzugsweise die mindestens eine Detektorsubstanz in der Detektorkammer zurückzuhalten. Der nachgewiesene Analyt kann bei einem reversiblen Eindringen beispielsweise selbständig, vorzugsweise durch Diffusion, die Detektorkammer wieder verlassen. Das reversible Eindringen kann insbesondere einen Austausch des Analyten in der Detektorkammer umfassen. Wünschenswert ist ein Austausch, insbesondere ein vollständiger Austausch, des Analyten in der Detektorkammer innerhalb einer bestimmten Zeitspanne, wobei die Zeitspanne, beispielsweise eine Messzeitkonstante, vorzugsweise geringfügig kürzer sein sollte als eine Zeitspanne, beispielsweise eine Systemzeitkonstante, in der sich beispielsweise die Analytkonzentration in dem Fluid ändert. Die Systemzeitkonstante kann insbesondere durch einen Organismus, insbesondere durch den Körper des Benutzers, bestimmt sein und beispielsweise nicht durch die Sensorvorrichtung und/oder eine Messanordnung. Ist beispielsweise die Messzeitkonstante länger als die Systemzeitkonstante, insbesondere die des Organismus, können beispielsweise Messfehler auftreten. Vorzugsweise sollte das reversible Eindringen des Analyten in die Detektorkammer ein insbesondere zeitnahes Folgen der Konzentration des Analyten in der Detektorkammer auf eine Änderung der Analytkonzentration in dem Fluid ermöglichen, insbesondere mit einer möglichst kurzen zeitlichen Verzögerung. Insbesondere sollte die Membran derart ausgestaltet sein, dass die Analytkonzentration in der Detektorkammer korreliert mit der Konzentration des Analyten außerhalb der Detektorkammer, bevorzugt sollten diese beiden Konzentrationen im Gleichgewicht sein oder im Falle eines Ungleichgewichts sollte sich in möglichst kurzer Zeit, insbesondere innerhalb einer Zeitspanne in welcher sich beispielsweise die Analytkonzentration in dem Fluid ändert, beispielsweise innerhalb einer Zeitspanne in welcher die Konzentration von Glucose in einem menschlichen Körper variiert, das Gleichgewicht einstellen.

Die Detektorkammer kann insbesondere zumindest teilweise mit einem flüssigen Medium, insbesondere einem wässrigen Medium, angefüllt sein. Die mindestens eine Detektorsubstanz kann ganz oder teilweise in dem mindestens einen flüssigen Medium enthalten sein, beispielsweise dispergiert und/oder gelöst und/oder emulgiert sein oder kann auch selbst Bestandteil des flüssigen Mediums sein, beispielsweise indem diese Detektorsubstanz vollständig oder teilweise selbst ein flüssiges Medium darstellt. Alternativ oder zusätzlich kann diese mindestens eine Detektorsubstanz jedoch auch, wie unten noch näher ausgeführt wird, in der Detektorkammer gebunden vorliegen, so dass eine Bewegungsfreiheit der Detektorsubstanz zumindest innerhalb gewisser Grenzen eingeschränkt ist. Beispielsweise kann die Detektorsubstanz vollständig oder teilweise und direkt oder indirekt an mindestens eine Wand der Detektorkammer und/oder an ein anderes Medium, beispielsweise ein poröses Medium, im Inneren der Detektorkammer gebunden sein. Beispielsweise kann die Detektorsubstanz direkt oder indirekt an eine Kammerwand selbst oder auch eine Elektrode des elektrischen Sensors, beispielsweise die mindestens eine Sensorelektrode gebunden sein. Diese Bindung kann direkt oder indirekt erfolgen, beispielsweise direkt oder unter Zwischenschaltung mindestens einer Zwischenschicht und/oder mindestens eines Spacers.

Der elektrische Sensor kann zusätzlich zu der Sensorelektrode weiterhin mindestens eine Gegenelektrode, welche beispielsweise als Hilfselektrode ausgestaltet sein kann, umfassen. Die Gegenelektrode kann bevorzugt als Elektrode, insbesondere als eine leitfähige Elektrode, oder als Elektrode oder Platte eines Kondensators ausgestaltet sein. Die mindestens eine Gegenelektrode kann ganz oder teilweise Bestandteil des Gehäuses der Detektorkammer sein. Die Sensorvorrichtung kann insbesondere eingerichtet sein, um die Gegenelektrode mit mindestens einem elektrischen Potenzial zu beaufschlagen. Die Beaufschlagung mit dem mindestens einen elektrischen Potenzial kann bevorzugt über Positionierung elektrischer Ladung an und/oder in der Gegenelektrode erzielt werden. Die Gegenelektrode kann beispielsweise über eine elektrische Leitung mit dem mindestens einen elektrischen Potenzial beaufschlagt werden. Beispielsweise kann die Gegenelektrode einen elektrisch leitenden oder halbleitenden Bereich aufweisen, welcher durch Anlegen einer elektrischen Spannung und/oder eines elektrischen Stroms mit dem mindestens einen elektrischen Potenzial beaufschlagt werden kann. Bei der elektrischen Spannung und/oder dem elektrischen Strom kann es sich prinzipiell um eine Gleichspannung bzw. einen Gleichstrom handeln. Bevorzugt kann es sich bei der angelegten elektrischen Spannung und/oder dem elektrischen Strom um ein zeitlich veränderbares, insbesondere regelbares, Signal handeln, beispielsweise um eine Spannungssequenz und/oder eine Stromsequenz. Die Spannungssequenz und/oder die Stromsequenz können beispielsweise mindestens ein zeitlich periodisches Signal umfassen und/oder eine Abfolge von Signalpulsen und/oder einen stufenförmigen Signalverlauf. Bevorzugt kann es sich bei der Spannungssequenz und/oder der Stromsequenz an der Gegenelektrode um ein alternierendes, insbesondere ein alternierendes stufenförmiges, Signal handeln.

Die Detektorkammer kann zumindest teilweise zwischen der Sensorelektrode und der Gegenelektrode angeordnet sein. Prinzipiell kann die Detektorkammer auch vollständig zwischen der Sensorelektrode und der Gegenelektrode angeordnet sein. Bevorzugt kann eine Anordnung derart gewählt werden, dass die Gegenelektrode und die Sensorelektrode nicht direkt elektrisch leitfähig miteinander verbunden sind, was beispielsweise durch eine geeignete elektrische Isolierung zwischen der Gegenelektrode und der Sensorelektrode realisiert werden kann.

Die Sensorvorrichtung kann weiterhin mindestens einen Kompensationssensor umfassen, welcher beispielsweise als Teil einer Kompensationsschaltung betrieben werden kann. Insbesondere kann der Kompensationssensor mindestens einen Referenzsensor umfassen und/oder ganz oder teilweise als Referenzsensor ausgestaltet sein. Der Kompensationssensor kann bevorzugt mindestens einen Kondensator und/oder mindestens einen Feldeffekttransistor umfassen. Unter einem Kompensationssensor kann ein Bestandteil der Sensorvorrichtung verstanden werden, welcher exemplarisch eine Referenz, beispielsweise einen Referenzwert, liefern kann. Die Sensorvorrichtung kann insbesondere ausgestaltet sein, um die Referenz, beispielsweise den Referenzwert, mit mindestens einem Signal des elektrischen Sensors zu kombinieren und ein gemeinsames, kompensiertes Signal zu erzeugen. So kann beispielsweise mittels der Referenz ein Offset erzeugt werden, welcher von dem Signal des elektrischen Sensors subtrahiert wird. Auch andere Kombinationen der Referenz mit dem Signal sind alternativ oder zusätzlich denkbar. Der Kompensationssensor kann beispielsweise ganz oder teilweise umfasst sein von der Kompensationsschaltung. Bei der Referenz kann es sich bevorzugt um eine elektrische Eigenschaft des Kompensationssensors handeln. Bei dem Referenzwert kann es sich bevorzugt um eine elektrische Spannung und/oder einen elektrischen Strom handeln. Die elektrische Eigenschaft kann beispielsweise ausgewählt sein aus der Gruppe bestehend aus: einer Ladungsträgerdichte; einer Ladung; einer Leitfähigkeit; einem Strom; einer Kapazität des Kondensators; einer Dielektrizitätskonstanten eines Mediums; einem Elektrodenabstand. Auch Kombinationen der genannten und/oder anderer Eigenschaften können die elektrischen Eigenschaften umfassen. Unter einem Kompensationssensor kann bevorzugt ein Bestandteil der Sensorvorrichtung verstanden werden, welcher beispielsweise dazu dient mindestens eine Fehlergröße zu kompensieren. Die Kompensationsschaltung und/oder der Kompensationssensor können bevorzugt dazu dienen, mindestens eine Fehlergröße, beispielsweise mindestens einen Offset und/oder einen Gleichlauf, zu kompensieren. Der Kompensationssensor kann bevorzugt elektrisch durch die Detektorkammer, also insbesondere durch den Inhalt der Detektorkammer, beeinflussbar sein. Der Kompensationssensor, insbesondere die Referenz, kann insbesondere dazu verwendet werden, den Nachweis des mindestens einen Analyten unabhängig von störenden Einflüssen durchzuführen. Der Kompensationssensor und/oder die Referenz kann weiterhin beispielsweise in einem Verfahren zur Kalibrierung und/oder zur Eichung und/oder zur Diagnose von mindestens einer Fehlfunktion, insbesondere im Rahmen des Nachweises des Analyten, verwendet werden.

Der Kompensationssensor kann vorzugsweise unbeeinflusst von der Konzentration des Analyten in der Detektorkammer sein. Diese Unbeeinflussung von der Konzentration des Analyten in der Detektorkammer kann absolut sein, kann jedoch auch eine Unterdrückung eines Einflusses der Konzentration des Analyten in der Detektorkammer umfassen. Diese Unbeeinflussung kann beispielsweise dadurch realisiert werden, dass der Kompensationssensor keinen Rezeptor und/oder keine Detektorsubstanz umfasst und/oder dass der Kompensationssensor mindestens eine Detektorsubstanz und/oder mindestens einen Rezeptor umfasst, welcher der elektrische Sensor nicht umfasst.

Der Kompensationssensor kann vorzugsweise baugleich zu dem elektrischen Sensor ausgebildet sein, insbesondere mit der Maßgabe, dass die mindestens eine elektrische Eigenschaft des Kompensationssensors vorzugsweise möglichst unbeeinflusst durch die Konzentration des Analyten in der Detektorkammer ist. Unter "baugleich" kann insbesondere verstanden werden, dass der Kompensationssensor identisch zu dem elektrischen Sensor aufgebaut ist bis auf Komponenten, welche die elektrische Eigenschaft des Kompensationssensors abhängig von dem Analyten, insbesondere von der Konzentration des Analyten, in der Detektorkammer machen, beispielsweise die Detektorsubstanz und/oder der Rezeptor, welche von dem elektrischen Sensor umfasst werden können oder mindestens eine Komponente der Detektorsubstanz welche von dem elektrischen Sensor umfasst sein kann.

Der Kompensationssensor kann beispielsweise mindestens eine Kompensations-Sensorelektrode umfassen. Die Kompensations-Sensorelektrode kann insbesondere zumindest im Wesentlichen baugleich zu der Sensorelektrode des elektrischen Sensors ausgestaltet sein, mit der Maßgabe, dass die mindestens eine elektrische Eigenschaft der Kompensations-Sensorelektrode durch die Konzentration des Analyten vorzugsweise unbeeinflusst ist, zumindest bis auf einen Faktor 1/10 im Vergleich zu der Beeinflussung der Sensorelektrode bei derselben Analytkonzentration. Die Detektorsubstanz kann mit der Sensorelektrode, insbesondere mit der Sensorelektrode des elektrischen Sensors, verbunden sein. Die Kompensations-Sensorelektrode kann vorzugsweise, insbesondere teilweise, frei von der Detektorsubstanz und/oder dem Rezeptor sein, insbesondere der Detektorsubstanz und/oder dem Rezeptor, welcher insbesondere von dem elektrischen Sensor umfasst werden kann.

Der elektrische Sensor und/oder der Kompensationssensor können insbesondere in eine elektrische Brückenschaltung und/oder die Kompensationsschaltung eingebunden sein. Die Brückenschaltung kann beispielsweise mindestens einen elektrischen Widerstand, insbesondere eine elektrische Komponente, welche einen elektrischen Wiederstand aufweist, umfassen. Bei dem elektrischen Widerstand kann es sich um einen reellen elektrischen Widerstand und/oder einen kapazitiven elektrischen Widerstand und/oder einen induktiven elektrischen Widerstand handeln. Bei der elektrischen Komponente kann es sich insbesondere um mindestens einen Kondensator und/oder mindestens einen ohmschen Widerstand handeln.

Die Detektorsubstanz kann insbesondere elektrisch geladene Teilchen mindestens einer Sorte umfassen. Diese Teilchen können vorzugsweise eine Teilchengröße $d_{50}$ aufweisen, welche kleiner ist als 0,5 mm, besonders bevorzugt kleiner ist als 0,1 mm und besonders bevorzugt kleiner als 1 $\mu$m oder sogar kleiner als 100 nm und/oder, dass die Teilchengröße $d_{50}$ bevorzugt kleiner als ein Zehntel eines Kammermaßes, insbesondere einer Abmessung der Detektorkammer und/oder des Volumens der Nachweißkammer, ist. Insbesondere kann es sich hierbei also um mikroskopische und/oder nanoskopische Teilchen handeln. Weiterhin können, alternativ oder zusätzlich, die elektrisch geladenen Teilchen auch elektrisch geladene Partikel und/oder elektrisch geladene Moleküle umfassen. Unter elektrisch geladenen Teilchen sind allgemein im Rahmen der vorliegenden Erfindung Teilchen zu verstehen, welche eine Nettoladung aufweisen und/oder Teilchen, welche auf ein und demselben Teilchen an unterschiedlichen Orten entgegengesetzte elektrische Ladungen aufweisen, also beispielsweise Dipole und/oder Multipole. Besonders bevorzugt ist es jedoch, wenn die elektrisch geladenen Teilchen eine Nettoladung von mindestens einer Elementarladung aufweisen, vorzugsweise von mindestens zwei Elementarladungen oder mehr.

Die elektrisch geladenen Teilchen können als frei bewegliche Teilchen in der Detektorkammer aufgenommen sein, beispielsweise in einem flüssigen Medium innerhalb der Detektorkammer, oder können auch, wie unten noch näher ausgeführt wird, gebunden vorliegen, beispielsweise über einen oder mehrere Spacer. In jedem Fall sollten jedoch die elektrisch geladenen Teilchen eine Bewegung innerhalb der Detektorkammer durchführen können, insbesondere eine Bewegung senkrecht zu der mindestens einen Sensorelektrode, beispielsweise eine Relativbewegung relativ zu dem mindestens einen elektrischen Sensor. Vorzugsweise ist eine Ladungsumverteilung der elektrisch geladenen Teilchen innerhalb der Detektorkammer möglich, beispielsweise indem diese elektrisch geladenen Teilchen frei beweglich sind oder über eine bewegliche Anbindung mit anderen Elementen, beispielsweise einer Innenwand der Detektorkammer, verbunden sind.

Vorzugsweise sind die Detektorkammer und die Detektorsubstanz derart ausgestaltet, dass ein mittlerer Abstand der elektrisch geladenen Teilchen von der Sensorelektrode und/oder eine Ladungsverteilung innerhalb der Detektorkammer abhängig sind von der Konzentration des Analyten in der Detektorkammer. Dies kann auf verschiedene Weisen erfolgen, welche unten exemplarisch noch näher ausgeführt werden. Beispielsweise kann dies dadurch erfolgen, dass der mindestens eine Analyt die elektrisch geladenen Teilchen näher an die mindestens eine Sensorelektrode und/oder näher an eine Kammerwand der Detektorkammer drängt. Alternativ kann der mindestens eine Analyt auch die elektrisch geladenen Teilchen von der mindestens einen Sensorelektrode verdrängen, sodass der mittlere Abstand zwischen den elektrisch geladenen Teilchen und der mindestens einen Sensorelektrode ansteigt. Wiederum alternativ oder zusätzlich kann auch eine andere Art der Beeinflussung der Ladungsverteilung, insbesondere der Verteilung der elektrisch geladenen Teilchen, innerhalb der Detektorkammer erfolgen, beispielsweise indem ein mittlerer Abstand zwischen den elektrisch geladenen Teilchen durch den Analyten oder durch ein Zusammenwirken des Analyten mit dem mindestens einen Detektorsubstanz verändert wird, also vergrößert oder verkleinert wird. Wiederum alternativ oder zusätzlich können auch bestimmte Inhomogenitäten der Verteilung der elektrisch geladenen Teilchen innerhalb der Detektorkammer durch den Analyten oder durch ein Zusammenwirken des Analyten mit der Detektorsubstanz hervorgerufen werden, welche, wie auch die anderen Effekte, die mindestens eine elektrische Eigenschaft des elektrischen Sensors beeinflussen kann.

Die Konzentration des Analyten und/oder eine Änderung derselben in der Detektorkammer können also vorzugsweise eine Ladungsverteilung und/oder einen mittleren Abstand der elektrisch geladenen Teilchen in der Detektorkammer verändern oder beeinflussen. Wie oben ausgeführt und wie nachfolgend noch näher ausgeführt wird, können die elektrisch geladenen Teilchen frei bewegliche Teilchen und/oder auch gebundene Teilchen sein. Die Detektorkammer und die Detektorsubstanz können insbesondere derart ausgestaltet sein, dass der mittlere Abstand der elektrisch geladenen Teilchen von der Sensorelektrode und/oder die Ladungsverteilung innerhalb der Detektorkammer sich bei einer Änderung der Konzentration des Analyten in der Detektorkammer reversibel ändern.

Die Ladungsverteilung innerhalb der Detektorkammer kann eine kontinuierliche Ladungsverteilung sein und/oder kann auch eine Ladungsverteilung in Form unterschiedlich geladener Schichten innerhalb der Detektorkammer umfassen. Dementsprechend können beispielsweise mindestens zwei unterschiedlich geladene Schichten innerhalb der Detektorkammer vorgesehen sein, wobei die Lage und/oder Anordnung dieser Schichten und/oder ein Abstand der Schichten von der Sensorelektrode durch die Konzentration des Analyten in der Detektorkammer beeinflussbar sein kann. Auf diese Weise können beispielsweise die Lage und/oder die Anordnung dieser Schichten und/oder der Abstand der Schichten von der Sensorelektrode durch eine Änderung der Konzentration des Analyten in der Detektorkammer beeinflussbar sein, vorzugsweise reversibel. Die Veränderung oder Beeinflussung des mittleren Abstands der elektrisch geladenen Teilchen in der Detektorkammer und/oder die Veränderung der Ladungsverteilung innerhalb der Detektorkammer durch die Konzentration des Analyten oder eine Änderung derselben, kann also beispielsweise derart erfolgen, dass die Detektorkammer und die Detektorsubstanz derart ausgestaltet sind, dass innerhalb der Detektorkammer eine Schichtbildung mindestens zweier Schichten mit unterschiedlicher elektrischer Ladung erfolgt, wobei die Verteilung der Schichten und/oder deren Abstand von der Sensorelektrode abhängig sind von der Konzentration des Analyten in der Detektorkammer, also beispielsweise durch die Konzentration des Analyten beeinflussbar und/oder durch eine Änderung der Konzentration des Analyten änderbar sind, insbesondere reversibel.

Insgesamt können somit die Detektorkammer und/oder die Detektorsubstanz derart ausgestaltet sein, dass eine Schichtbildung innerhalb der Detektorkammer und die Geometrie und/oder die Abstände dieser Schichtbildung abhängig sind von der Konzentration des Analyten in der Detektorkammer und durch diese Konzentration beeinflussbar und/oder durch eine Änderung dieser Konzentration veränderbar sind, vorzugsweise reversibel. Auf diese Weise kann beispielsweise die Analytkonzentration innerhalb der Detektorkammer einen Einfluss auf ein elektrisches Signal der Sensorvorrichtung haben. Beispielsweise können, wie unten noch näher ausgeführt wird, elektrische Feldstärken innerhalb eines Feldeffekttransistors und/oder eine Kapazität eines Kondensators und/oder Dielektrizitätseigenschaften der Detektorkammer oder eines Teils derselben durch die Analytkonzentration in der Detektorkammer beeinflussbar sein. Diese Beeinflussung kann insbesondere reversibel ausgestaltet sein, beispielsweise in Form eines Ausgleichsprozesses.

Die elektrisch geladenen Teilchen können insbesondere Puffereigenschaften aufweisen. Unter Puffereigenschaften sind Eigenschaften einer Substanz zu verstehen, bei welchen die Substanz ihre Umgebung dahingehend beeinflusst, dass sich der pH-Wert der Umgebung bei Zugabe einer Säure oder einer Base wesentlich weniger stark ändert als dies bei einem ungepufferten System, also bei Abwesenheit der Substanz, der Fall wäre. Vorzugsweise beinhalten die Puffereigenschaften, dass die Substanz sowohl Säureeigenschaften als auch Baseeigenschaften aufweist.

Alternativ oder zusätzlich können die geladenen Teilchen beispielsweise spezielle erweiterte und/oder geordnete Strukturen bilden, beispielsweise indem sie, sofern sie negativ geladen sind, Komplexbildung mit entgegengesetzt geladenen Teilchen, beispielsweise mit anorganischen oder organischen Kationen, eingehen. Da die unten benannten negativ geladenen Teilchen die korrespondierenden Basen der genannten Säuren, insbesondere von Polycarbonsäuren, sind, kann insbesondere eine wechselseitige Komplexbildung von Säure-Anionen mit Metallkationen und/oder mit protonierten, insbesondere organischen, Basen, beispielsweise mit Aminen wie terminalen Alkyl-Diaminen oder Heterocyclen mit, insbesondere mehreren, basischen Stickstoffatomen, beispielsweise Imidazol oder Bipyridine, auftreten. Erweiterte, aber insbesondere gut definierte, Aggregate mit verbleibender Nettoladung, welche hierbei beispielsweise entstehen können, könnten beispielsweise geeignetere Nachweissubstanzen als die ursprünglichen geladenen Teilchen darstellen, beispielsweise da, insbesondere falls vorliegend, eine Analyt- und Ladungs-abhängige Ausrichtung im gewünschten Sinne begünstigt sein könnte. Beispielsweise könnten komplexere Aggregate mit einer verbleibenden Nettoladung gebildet werden, wobei eine übergeordnete Struktur, beispielsweise lineare Strukturen, ggf. auch Gerüst-Strukturen, durch eine Zusammensetzung der einzelnen Komponenten vorgegeben wäre.

Die elektrisch geladenen Teilchen können insbesondere polyionische Moleküle umfassen, also Moleküle, welche mindestens eine ionische Gruppe umfassen, insbesondere polyanionische Moleküle.

Die elektrisch geladenen Teilchen können insbesondere mindestens eine Polycarbonsäure umfassen. Insbesondere kann diese mindestens eine Polycarbonsäure eine Polycarbonsäure des Benzols sein, vorzugsweise eine Polycarbonsäure ausgewählt aus der Gruppe bestehend aus Benzoldicarbonsäure (H2), beispielsweise meta- oder para-Phthalsäure, bevorzugt, da hier die beiden negativ geladenen Carboxyl-Gruppen benachbart sind, ortho-Phthalsäure, Benzoltricarbonsäure (H3), beispielsweise Trimesinsäure oder Trimellitsäure, Benzoltetracarbonsäure (H4), beispielsweise Pyromellitsäure, Benzolpentacarbonsäure (H5) und Benzolhexacarbonsäure (H6) (Mellitsäure); wobei in der Mellitsäure eine Anbindung an den Spacer im Allgemeinen nur über eine der sechs Carboxylgruppen möglich wäre, oder Dimeren, Trimeren oder Oligomeren oder Derivaten daraus.

Wie oben ausgeführt, kann die Detektorsubstanz insbesondere mindestens einen Spacer umfassen. Dieser Spacer kann insbesondere eingerichtet sein, um die elektrisch geladenen Teilchen an die Sensorelektrode und/oder eine Oberfläche der Detektorkammer an der Sensorelektrode zu binden. Beispielsweise kann diese Bindung direkt an die Sensorelektrode erfolgen. Alternativ oder zusätzlich kann auch eine Bindung an eine Oberfläche einer Gehäuseinnenwand der Detektorkammer oberhalb der Sensorelektrode im Inneren der Detektorkammer erfolgen. Beispielsweise kann sich auf der Sensorelektrode mindestens eine Zwischenschicht befinden, über welche der Innenraum der Detektorkammer von der Sensorelektrode getrennt ist, wobei die Anbindung über den Spacer an die Oberfläche dieser mindestens einen Zwischenschicht erfolgen kann.

Der Spacer soll insbesondere eingerichtet sein, um mindestens zwei Stellungen einzunehmen, in welchen die elektrisch geladenen Teilchen einen unterschiedlichen Abstand zu der Sensorelektrode aufweisen. Dies bedeutet, dass der mindestens eine Spacer eine Relativbewegung der geladenen Teilchen zu der Sensorelektrode ermöglichen soll, trotz Anbindung. Dies kann eine freie Beweglichkeit beinhalten, was beispielsweise bei langkettigen Spacermolekülen der Fall ist, oder auch lediglich eine Beweglichkeit zwischen zwei Stellungen oder einer anderen begrenzten Anzahl von Stellungen, in welchen die geladenen Teilchen einen unterschiedlichen Abstand zu der Sensorelektrode aufweisen. Beispielsweise kann der Spacer eingerichtet sein, um unterschiedliche Konformationen anzunehmen, also mindestens zwei unterschiedliche Konformationen, wobei in den unterschiedlichen Konformationen die über den Spacer angebundenen elektrisch geladenen Teilchen einen unterschiedlichen Abstand zu der Sensorelektrode aufweisen.

Der mindestens eine Spacer kann insbesondere mindestens ein kettenförmiges Molekül umfassen. Der mindestens eine Spacer kann insbesondere ausgewählt sein aus der Gruppe bestehend aus: verzweigten oder unverzweigten aliphatischen Ketten, insbesondere Alkylketten; verzweigten oder unverzweigten Polyetherketten; verzweigten oder unverzweigten Polyiminoketten und/oder Derivaten aus den genannten Materialien. Der Spacer kann insbesondere ladungsneutral sein, insbesondere unpolar. Auch polare Ausgestaltungen sind jedoch grundsätzlich möglich.

In der Detektorkammer kann insbesondere mindestens ein Rezeptor aufgenommen sein, insbesondere ein immobilisierter Rezeptor. Beispielsweise kann es sich hierbei um einen an der Sensorelektrode direkt und/oder an einer Oberfläche der Detektorkammer an der Sensorelektrode immobilisierten Rezeptor handeln. Dieser Rezeptor kann beispielsweise chemisch an der Sensorelektrode und/oder der Oberfläche der Detektorkammer an der Sensorelektrode angebunden sein, wobei, wie auch bei der Anbindung des Spacers, grundsätzlich verschiedene Bindungsarten in Betracht kommen, also beispielsweise kovalente Bindungen und/oder ionische Bindungen und/oder Komplexbindungen. Analoges gilt auch für die Anbindung des optionalen mindestens einen geladenen Teilchens an den Spacer. Der Rezeptor soll eingerichtet sein, um den Analyten zu binden, insbesondere reversibel. Diese Bindung kann beispielsweise mindestens eine Komplexbindung und/oder mindestens eine ionische Bindung und/oder mindestens eine elektrostatische Bindung und/oder mindestens eine Wasserstoffbrückenbindung und/oder mindestens eine kovalente Bindung und/oder mindestens eine Van-der-Waals-Bindung umfassen. Van-der-Waals-Kräfte können ebenfalls auftreten, sind aber in der Regel insbesondere infolge ihrer geringen Stärke und ihrer hohen Abhängigkeit vom Abstand im Allgemeinen nur von untergeordneter Bedeutung.

Der Rezeptor kann weiterhin auch eingerichtet sein, um die Detektorsubstanz zumindest teilweise zu binden, also die Detektorsubstanz oder einen Teil derselben zu binden. Insbesondere kann der Rezeptor eingerichtet sein, um die optionalen elektrisch geladenen Teilchen und/oder andere Bestandteile der Detektorsubstanz zu binden, beispielsweise eine oder mehrere Konkurrenzmoleküle, die nicht notwendigerweise eine Nettoladung aufweisen müssen. Diese Bindung der Detektorsubstanz und/oder eines Teils derselben kann insbesondere mit der Bindung des Analyten konkurrieren. Die Bindung kann vorzugsweise wiederum reversibel erfolgen.

Insbesondere kann der mindestens eine Rezeptor mindestens ein Lectin umfassen. Unter Lectinen sind dabei, wie allgemein üblich, chemische Substanzen, insbesondere komplexe Proteine oder Glycoproteine zu verstehen, welche Kohlenhydratstrukturen binden können, insbesondere spezifisch, insbesondere Glucose, was beispielsweise für medizinisch analytische Verfahren von besonderem Interesse sein kann. Derartige Lectine sind beispielsweise aus der Biochemie bekannt, wo diese sich, beispielsweise spezifisch, an Zellen bzw. Zellmembranen binden und von dort aus biochemische Reaktionen auslösen können. Vorzugsweise weisen Lectine keine enzymatische Aktivität auf. Insbesondere kann das mindestens eine Lectin ausgewählt sein aus der Gruppe Glucose-bindender Lectine, insbesondere solcher Lectine, die für einen Glucose-Nachweis eingesetzt werden können. Die Gruppe kann beispielsweise bestehen aus: Concanavalin A (Con A), insbesondere aus der Jackbohne und/oder der Schwertbohne, Lens culinaris-Agglutinin, insbesondere aus der Linse, Pealectin-I (PSA), insbesondere aus der Erbse.

Die Detektorsubstanz kann weiterhin mindestens einen Konkurrenten umfassen. Beispielsweise kann dieser mindestens eine Konkurrent mindestens eine Sorte von Konkurrenzteilchen und/oder Konkurrenzmolekülen umfassen. Dieser mindestens eine Konkurrent kann auch ganz oder teilweise identisch mit dem elektrisch geladenen Teilchen sein oder kann mindestens eine andere Sorte von Teilchen umfassen. Der Konkurrent soll eingerichtet sein, um an den Rezeptor zu binden, insbesondere reversibel. Der Analyt kann insbesondere mindestens einen Polyalkohol und/oder mindestens einen Zucker umfassen, insbesondere Glucose, wobei der Konkurrent ebenfalls mindestens einen Zucker umfasst, insbesondere ein mit einem Zucker markiertes Molekül, vorzugsweise Glucose-markiertes Dextran. Beispielsweise kann der nachzuweisende Analyt Glucose sein, der mindestens eine Rezeptor Concanavalin-A, und der mindestens eine Konkurrent Glucose-markiertes Dextran.

Die Detektorsubstanz kann vorzugsweise eingerichtet sein, um den Analyten nicht umzusetzen, wobei unter einer Umsetzung eine chemische Reaktion verstanden wird, bei welcher die Detektorsubstanz vollständig oder teilweise verändert und/oder verbraucht wird. Diese Ausgestaltung bietet den Vorteil, dass Alterungseffekte aufgrund eines Verbrauchs der Detektorsubstanz vermindert werden. Alternativ kann die Detektorsubstanz jedoch auch mindestens einen Nachweisreagens umfassen, welches eingerichtet ist, um mit dem Analyten zu reagieren, also den Analyten umzusetzen. Beispielsweise kann das Nachweisreagens mindestens ein Enzym umfassen, insbesondere ein Enzym, welches eingerichtet ist, um den Analyten zu oxidieren und/oder zu reduzieren. Auch andere Arten von Enzymen sind jedoch grundsätzlich einsetzbar. Insbesondere kann das mindestens eine Enzym ausgewählt sein aus der Gruppe von Enzymen, welche Glucose umsetzen, wie beispielsweise Glucoseoxidase (GOD), Glucosedehydrogenasen, insbesondere NAD+ abhängige und/oder PQQ-abhängige (GlucDOR) und/oder FAD-abhängige GlucDH.

Das Nachweisreagens kann insbesondere eingerichtet sein, um einen pH-Wert innerhalb der Detektorkammer zu beeinflussen, wobei die Detektorsubstanz mindestens einen pH-sensitiven Indikator umfasst, wobei der pH-sensitive Indikator eingerichtet ist, um mindestens eine Eigenschaft in Abhängigkeit von dem pH-Wert zu ändern, wobei die mindestens eine Eigenschaft eingerichtet ist, um die mindestens eine elektrische Eigenschaft des elektrischen Sensors zu beeinflussen. Beispielsweise kann die mindestens eine Eigenschaft des pH-sensitiven Indikators eine pH-abhängige Strukturänderung und/oder ein pHabhängiger Quellungszustand des mindestens einen Indikators sein und/oder eine mittlere Ladungsträgerdichte des pH-sensitiven Indikators und/oder eine Dielektrizitätskonstante des pH-sensitiven Indikators und/oder ein Dipolmoment des pH-sensitiven Indikators. Auch andere Eigenschaften können jedoch in Abhängigkeit von dem pH-Wert geändert werden und dadurch wiederum die mindestens eine elektrische Eigenschaft des elektrischen Sensors beeinflussen. Der pH-sensitive Indikator kann ein Polyanion umfassen, vorzugsweise eine Polyacrylsäure und/oder ein Derivat der Polyacrylsäure, z.B. Polymethacrylsäure. Deren anionische Gruppen werden bei steigendem pH-Wert protoniert und damit, zumindest teilweise, neutralisiert, womit beispielsweise ein ursprünglich ladungsbedingt gestrecktes geladenes Polymer sich verknäuelt. Der pH-sensitive Indikator kann insbesondere alternativ oder zusätzlich mindestens ein quellfähiges Material umfassen, insbesondere mindestens einen vernetzten Polyelektrolyten. Dies kann ein polyionisches Molekül sein, vorzugsweise mindestens ein polyanionisches Polymer, vorzugsweise ein Hydrogel auf Basis der Polyacrylsäure oder einem Derivat der Polyacrylsäure, z.B. Polymethacrylsäure. Hier nimmt in der Regel die Quellung eines Hydrogels mit steigendem pH, also bei dessen Deprotonierung zu und mit zunehmender Protonierung ab. Bei dem pH-sensitiven Indikator, beispielsweise einem pH-sensitives Reagenz, können beispielsweise mindestens zwei Möglichkeiten auftreten. Bei einer ersten Möglichkeit kann beispielsweise eine Längsstreckung eines Polyanions, insbesondere auf Grund einer Abstoßung von negativen Ladungen im Polyanion, beispielsweise eine Strukturänderung in Form einer Verknäuelung erfahren, insbesondere, wenn beispielsweise der pH-Wert abnimmt und/oder das gestreckte Polyanion durch Protonierung entladen wird. Bei einer zweiten Möglichkeit kann beispielsweise eine Quellung eines 3-dimensionalen, beispielsweise wasseraufnehmenden, Polymers, beispielsweise eines Hydrogels, infolge einer Änderung des pH-Wertes auftreten. Hierbei führt die Protonierung zu H-Brücken zwischen anionischen Gruppen, wodurch diese insbesondere zusammenrücken, d.h. die Quellung erfolgt vorzugsweise bei Deprotonierung. Wenn beispielsweise bei der ersten Möglichkeit und/oder der zweiten Möglichkeit an das Polymer eine Glucoseoxidase (GOD) angedockt ist oder zumindest allgemein dort lokalisiert ist, kann diese GOD den Analyten, insbesondere die Glucose, umsetzen. Der pH-Wert wird dabei in der Regel gesenkt, wobei in der Regel Protonen als Reaktionsprodukt entstehen. Das Polymer wird in der Regel protoniert, wobei als Folgen insbesondere die oben beschriebene erste Möglichkeit oder die zweite Möglichkeit auftreten kann.

Weitere mögliche Ausgestaltungen der Erfindung betreffen mögliche Ausgestaltungen des elektrischen Sensors. So kann der mindestens eine elektrische Sensor, wobei auch mehrere elektrische Sensoren gleicher oder unterschiedlicher Art kombiniert werden können, insbesondere mindestens einen Feldeffekttransistor umfassen. Unter einem

EP 2 713 877 B1

Feldeffekttransistor ist dabei ein Bauelement zu verstehen, welches mindestens eine erste Elektrode (Source-Elektrode), mindestens eine zweite Elektrode (Drain-Elektrode) und mindestens einen diese Elektroden verbindenden anorganischen und/oder organischen Halbleiter, beispielsweise als Kanal, umfasst und welches weiterhin mindestens eine Gate-Elektrode, welche vorzugsweise von der Drain-Elektrode und/oder der Source-Elektrode und/oder dem Kanal isoliert, insbesondere elektrisch isoliert, ist, umfasst, welche durch einen Feldeffekt einen Ladungstransport zwischen der Source-Elektrode und der Drain-Elektrode beeinflusst.

Umfasst der elektrische Sensor mindestens einen Feldeffekttransistor, so kann die Sensorelektrode insbesondere mindestens eine Gate-Elektrode des Feldeffekttransistors umfassen. Unter einer Gate-Elektrode wird dabei, wie oben ausgeführt, eine Elektrode im Sinne der vorliegenden Erfindung verstanden, welche eingerichtet ist, um über Feldeffekte einen Stromfluss zwischen zwei Elektroden (Source-Elektrode und Drain-Elektrode) durch einen Halbleiter organischer und/oder anorganischer Natur zu steuern. Der Begriff der Gate-Elektrode ist dabei aber weit zu fassen und kann grundsätzlich ein beliebiges einteiliges oder mehrteiliges Element umfassen, welches über einen Feldeffekt einen Ladungstransport in dem Halbleiter beeinflussen kann. In diesem Fall kann die mindestens eine elektrische Eigenschaft des elektrischen Sensors, welche durch die Detektorsubstanz und/oder deren Zusammenwirken mit dem Analyten beeinflusst wird, insbesondere mindestens ein Elektrodenpotenzial der Gate-Elektrode umfassen. In anderen Worten kann in dieser Ausgestaltung die Sensorvorrichtung derart eingerichtet sein, dass über die Detektorsubstanz und deren Wechselwirkung mit dem Analyten in der Detektorkammer der Feldeffekt des Feldeffekttransistors beeinflusst wird.

Der Feldeffekttransistor kann mindestens ein elektrisch leitfähiges oder halbleitendes organisches Material umfassen. Unter einem elektrisch leitfähigen oder halbleitenden organischen Material ist dabei ein Material zu verstehen, welches eingerichtet ist, um einen Ladungsträgertransport durch dieses Material zu gewährleisten. Derartige organische Materialien sind insbesondere organische Materialien mit einem ausgedehnten $\pi$-Elektronensystem, insbesondere organische Materialien mit einer Vielzahl von konjugierten Doppelbindungen. Die Ladungsträger können dabei intrinsisch in dem organischen Material enthalten sein oder können von außen in das organische Material injiziert werden. Daher ist im Allgemeinen eine Unterscheidung zwischen Isolatoren, Halbleitern und Leitern bei organischen Materialien in der Regel nach klassischen, anorganischen Maßstäben schwierig, sodass allgemein in Bezug auf organische Materialien von Leitern oder Halbleitern gesprochen wird, sofern diese einen Stromtransport ermöglichen. Der Feldeffekttransistor kann somit ein organischer Feldeffekttransistor sein. Dabei kann es sich um ein vollständig aus organischen Materialien aufgebautes Bauelement handeln, welches neben organischen Materialien keine anorganischen Materialien, zumindest als eigenständige Bauteile oder Schichten, enthält. Alternativ kann der organische Feldeffekttransistor jedoch auch einzelne oder mehrere anorganische Bauteile oder Schichten umfassen, sofern zumindest eine organische Halbleiter- oder Leiterschicht vorgesehen ist. Zumindest zwischen der Source- und der Drain-Elektrode sollte jedoch eine organische Halbleiter- oder Leiterschicht vorgesehen sein, deren Stromfluss durch Feldeffekte der Gate-Elektrode beeinflussbar ist. Die Source- und/oder Drain-Elektrode können jedoch einzeln oder beide aus organischen oder anorganischen Leitermaterialien bestehen. Auch die Gate-Elektrode kann, sofern diese (siehe die obige Definition) als eigenständiges Bauteil ausgestaltet ist, organische oder auch anorganische Materialien umfassen. Alternativ zu der vorgeschlagenen Verwendung eines organischen Feldeffekttransistors, welcher vorzugsweise zumindest einen organischen Leiter oder Halbleiter umfasst, sind jedoch grundsätzlich auch klassische, anorganische Halbleiter im Rahmen der vorliegenden Erfindung einsetzbar.

Der Feldeffekttransistor kann insbesondere Teil eines Operationsverstärkers sein, also eines elektronischen verstärkenden Bauelements, welches eingerichtet ist, um elektrische Signale, beispielsweise elektronische Signale, insbesondere Spannungs- und/oder Stromsignale, zu verstärken. Der Operationsverstärker kann insbesondere einen nicht invertierenden und einen invertierenden Eingang aufweisen und/oder einen Differenzverstärker, insbesondere als Eingangsschaltung. Ausführungsbeispiele eines Operationsverstärkers werden unten noch näher beschrieben. Weiterhin kann die Sensorvorrichtung auch Schaltungen umfassen, welche Differenzsignale aus Wechselströmen generieren können und/oder auswerten können.

Der elektrische Sensor kann zusätzlich zu der Gate-Elektrode weiterhin mindestens eine Gegenelektrode, die wie oben beschrieben beschaffen sein kann, umfassen. Die Sensorvorrichtung kann insbesondere eingerichtet sein, um die Gegenelektrode beispielsweise mit mindestens einem elektrischen Potenzial zu beaufschlagen. Die Detektorkammer kann zumindest teilweise zwischen der Gate-Elektrode und der Gegenelektrode angeordnet sein.

Alternativ oder zusätzlich zu einem Feldeffekttransistor kann der elektrische Sensor auch mindestens einen Kondensator, beispielsweise mit einer Kapazität von maximal 100 pF, insbesondere maximal 10 pF, beispielsweise einer Kapazität von 2 pF, insbesondere von maximal 10 pF, beispielsweise mit einer Kapazität von 0,1 pF bis 10 pF, umfassen, welcher mindestens zwei Elektroden umfasst, wobei mindestens eine dieser Elektroden oder ein Teil derselben von der mindestens einen Sensorelektrode gebildet wird. Der Kondensator kann zusätzlich zu der Sensorelektrode weiterhin mindestens eine Gegenelektrode umfassen. Die Detektorkammer kann zumindest teilweise zwischen der Sensorelektrode und der Gegenelektrode angeordnet sein. Vorzugsweise füllt die Detektorkammer den Zwischenraum zwischen der Sensorelektrode und der Gegenelektrode vollständig aus, wobei die Detektorkammer, insbesondere die Kammer, vorzugsweise so zu gestalten ist, dass insbesondere die Sensorelektrode und/oder die Gegenelektrode nicht durch das umgebende Medium, beispielsweise das fluide Medium und/oder das Fluid, leitend kurzgeschlossen sind. Beispielsweise können die Sensorelektrode und die Gegenelektrode als parallele Elektroden ausgestaltet sein, also als Elektroden, deren Elektrodenoberflächen im Wesentlichen parallel zueinander ausgerichtet sind, beispielsweise mit einer Abweichung von einer Parallelität von nicht mehr als 20°, insbesondere nicht mehr als 10° und besonders bevorzugt nicht mehr als 5°. Auch andere Kondensatoraufbauten sind jedoch grundsätzlich möglich, beispielsweise konzentrische Aufbauten, bei welchen beispielsweise die Sensorelektrode und/oder die Gegenelektrode als ringförmige Elektroden und/oder als stabförmige oder drahtförmige Elektroden ausgestaltet sind. Wiederum sind auch, wie unten exemplarisch näher beschrieben wird, zylindrische Aufbauten möglich, bei welchen die Sensorelektrode und/oder die Gegenelektrode auf einem Zylindermantel aufgebracht sind, beispielsweise indem die Detektorkammer selbst zylindrisch ausgebildet wird, wobei beispielsweise die Sensorelektrode und die Gegenelektrode auf einander gegenüberliegenden Segmenten des Zylindermantels angeordnet sind. Verschiedene Ausgestaltungen sind möglich.

Ist der elektrische Sensor als Kondensator ausgestaltet oder umfasst dieser elektrische Sensor mindestens einen Kondensator, mit der Sensorelektrode als Kondensatorelektrode, so kann die mindestens eine elektrische Eigenschaft grundsätzlich eine beliebige Eigenschaft des Kondensators umfassen. Insbesondere kann die mindestens eine Eigenschaft ausgewählt sein aus der Gruppe bestehend aus: einer Kapazität des Kondensators; einer Dielektrizitätskonstanten eines Mediums in der Detektorkammer; ein Elektrodenabstand der Elektroden des Kondensators. Auch Kombinationen der genannten und/oder anderer Eigenschaften können eingesetzt werden oder auch andere Eigenschaften des Kondensators.

Die Sensorvorrichtung kann weiterhin mindestens eine Ansteuerung und/oder einen Teil einer Ansteuerung umfassen. Die Ansteuerung kann eingerichtet sein, um die mindestens eine elektrische Eigenschaft des elektrischen Sensors zu erfassen. Beispielsweise kann diese mindestens eine Ansteuerung vollständig oder teilweise in den elektrischen Sensor integriert sein. Alternativ oder zusätzlich kann diese Ansteuerung jedoch auch unabhängig von dem mindestens einen elektrischen Sensor angeordnet sein, beispielsweise außerhalb des elektrischen Sensors. Während der elektrische Sensor vorzugsweise vollständig oder zumindest teilweise implantierbar ausgestaltet ist, kann die Ansteuerung beispielsweise vollständig oder teilweise außerhalb des Körpergewebes verbleiben. Vorzugsweise ist die Ansteuerung jedoch mit dem elektrischen Sensor verbunden, beispielsweise drahtgebunden. Diese Verbindung kann permanent oder auch lösbar ausgestaltet sein. Die mindestens eine Ansteuerung kann beispielsweise mindestens eine elektrische Schaltung umfassen, um die mindestens eine elektrische Eigenschaft des elektrischen Sensors zu erfassen. Beispielsweise kann die Ansteuerung zu diesem Zweck mindestens eine Beaufschlagungsvorrichtung umfassen, welche eingerichtet ist, um den elektrischen Sensor mit einem Strom und/oder einer Spannung zu beaufschlagen. Dabei können Gleichspannungen, Gleichströme oder auch Wechselspannungen oder Wechselströme eingesetzt werden. Die Ansteuerung kann an die Art des elektrischen Sensors, wobei auch mehrere elektrische Sensoren vorgesehen sein können, angepasst werden. So kann die Beaufschlagungsvorrichtung beispielsweise bei einem Feldeffekttransistor eingerichtet sein, um die Source- und/oder Drain-Elektrode mit der mindestens einen Spannung und/oder dem mindestens einen Strom zu beaufschlagen. Ist der elektrische Sensor vollständig oder teilweise als Kondensator ausgestaltet, so kann ebenfalls eine Beaufschlagung der Kondensatorelektroden mit einem Strom und/oder einer Spannung erfolgen. Weiterhin kann, alternativ oder zusätzlich, die mindestens eine Ansteuerung mindestens eine Messvorrichtung umfassen, beispielsweise mindestens eine Spannungsmessvorrichtung, beispielsweise mindestens eine Wechselspannungsmessvorrichtung und/oder mindestens eine Gleichspannungsmessvorrichtung, und/oder mindestens eine Strommessvorrichtung, beispielsweise mindestens eine Wechselstrommessvorrichtung und/oder mindestens eine Gleichstrommessvorrichtung, beispielsweise um einen Strom durch eine oder mehrere Zuleitungen zu dem elektrischen Sensor zu erfassen. Alternativ oder zusätzlich können eine oder mehrere Spannungsmessvorrichtungen vorgesehen sein, beispielsweise um eine Spannung zwischen mindestens zwei Messpunkten des elektrischen Sensors zu erfassen, beispielsweise eine Spannung zwischen zwei Elektroden des elektrischen Sensors, beispielsweise eine Spannung zwischen zwei Kondensatorelektroden eines Kondensators und/oder eine Spannung zwischen einer Source- und einer Drain-Elektrode und/oder anderen Elektroden eines Feldeffekttransistors. Auch andere Ausgestaltungen der Ansteuerung sind möglich. So kann die Ansteuerung beispielsweise, wie unten noch näher ausgeführt wird, mindestens einen Oszillator und/oder beispielsweise mit mindestens einer kapazitiven Brückenschaltung erfolgen.

Die Ansteuerung kann beispielsweise mit einem oder mehreren diskreten elektrischen Bauelementen ausgestaltet sein. Alternativ oder zusätzlich kann die Ansteuerung jedoch auch ganz oder teilweise als integrierter Schaltkreis ausgestaltet sein, beispielsweise als anwendungsspezifischer integrierter Schaltkreis (application-specific integrated circuit, ASIC). Die Ansteuerung kann insbesondere mindestens eine drahtgebundene oder drahtlose Schnittstelle umfassen, um mindestens ein Signal an eine weitere Vorrichtung abzugeben, beispielsweise an mindestens ein Lesegerät, was unten noch näher beschrieben wird.

Die Ansteuerung kann insbesondere eingerichtet sein, um mindestens ein Signal zu erzeugen, und optional dieses Signal beispielsweise über mindestens eine Schnittstelle weiterzugeben, insbesondere mindestens ein periodisches Signal, mit einer oder mehreren Frequenzen oder einem Frequenzband. Zu diesem Zweck kann die Ansteuerung beispielsweise mindestens einen Oszillator umfassen. Die Ansteuerung und der elektrische Sensor können insbesondere derart eingerichtet sein, dass das Signal durch die Analytkonzentration veränderbar ist. Beispielsweise kann dies in Form einer Beeinflussung einer Frequenz des Signals erfolgen. Bei der Frequenz kann es sich beispielsweise um eine Trägerfrequenz, beispielsweise eine Trägerfrequenz einer Radiofrequenz direkt, und/oder um eine Amplitude und/oder um eine Phase handeln. Bei der Beeinflussung der Frequenz kann es sich beispielsweise um eine Frequenzmodulation, beispielsweise falls die Frequenz eine Funktion einer Glukosekonzentration ist, und/oder eine Amplitudenmodulation und/oder eine Phasenmodulation handeln. Auf diese Weise kann, beispielsweise durch ein Lesegerät, aus der Beeinflussung des Signals direkt oder indirekt auf die Analytkonzentration in dem Fluid und/oder der Detektorkammer geschlossen werden, beispielsweise aus einer Frequenzverschiebung und/oder anderen Art der Frequenzänderung und/oder aus einer Phasenänderung, beispielsweise als Information in einer Phasenlage. Derartige Beeinflussungen eines Signals sind beispielsweise dadurch möglich, wie unten noch näher ausgeführt wird, dass der elektrische Sensor und/oder ein Teil desselben, beispielsweise ein Kondensator, in einen Schwingkreis und/oder in eine Oszillatorschaltung eingebunden werden, sodass die mindestens eine elektrische Eigenschaft des elektrischen Sensors sich unmittelbar auf die Schwingungseigenschaften des Oszillators und/oder des Schwingkreises und/oder sich unmittelbar auf einen Phase-locked loop (PLL) auswirkt.

Die Ansteuerung kann insbesondere eingerichtet sein, um den elektrischen Sensor mit mindestens einer Spannungssequenz und/oder mindestens einer Stromsequenz zu beaufschlagen und vorzugsweise mindestens eine Frequenzantwort des elektrischen Sensors zu erfassen. Die Spannungssequenz und/oder die Stromsequenz kann beispielsweise ein zeitlich periodisches Signal umfassen und/oder eine Abfolge von Signalpulsen und/oder einen stufenförmigen Signalverlauf. Beispielsweise kann der gesamte elektrische Sensor mit der gleichen Spannungssequenz und/oder Stromsequenz beaufschlagt werden, prinzipiell können einzelne Komponenten des elektrischen Sensors auch mit unterschiedlichen Spannungssequenzen und/oder Stromsequenzen beaufschlagt werden. Bei der Frequenzantwort kann es sich beispielsweise um einen elektrischen Strom und/oder eine elektrische Spannung und/oder eine Frequenz handeln. Die Frequenzantwort kann auch mehrere Frequenzen umfassen, insbesondere ein Frequenzspektrum.

Die Ansteuerung und/oder elektronische Komponenten können insbesondere gegenüber dem Fluid gekapselt sein. Weiterhin können die Ansteuerung und/oder elektronische Komponenten auch gegenüber dem fluiden Medium gekapselt sein. Unter einer Kapselung kann insbesondere eine Vermeidung oder Verhinderung eines Kontaktes, insbesondere eines elektrischen Kontaktes, verstanden werden, insbesondere kann unter einer Kapselung ein Bestandteil der Sensorvorrichtung verstanden werden, welcher dem Zwecke der Kapselung dienen kann. Eine Kapselung der Ansteuerung kann mindestens einen Teil des Gehäuses der Detektorkammer bilden. Die Kapselung kann vorzugsweise derart ausgestaltet sein, dass das Fluid und/oder das fluide Medium und/oder die Detektorsubstanz keinen elektrischen Kurzschluss verursachen können. Unter einem Kurzschluss kann hierbei insbesondere ein elektrischer Widerstand von kleiner als $10^9$ Ohm verstanden werden. Insbesondere kann die Kapselung derart ausgestaltet sein, dass zwischen den Elektroden des elektrischen Sensors und/oder zwischen den Elektroden des Kompensationssensors und/oder zwischen Komponenten der Ansteuerung und/oder Komponenten der Brückenschaltung kein Kurzschluss aufgrund des Fluids und/oder des fluiden Mediums und/oder der Detektorsubstanz entstehen kann.

Wie oben ausgeführt, ist es im Rahmen der vorliegenden Erfindung besonders bevorzugt, wenn der elektrische Sensor, insbesondere die Sensorelektrode, mindestens ein organisches leitfähiges oder halbleitendes Material aufweist. Wie oben ausgeführt, soll es sich dabei um ein organisches Material handeln, welches eingerichtet ist, um einen Stromtransport zu unterstützen. Derartige organische leitende oder halbleitende Materialien sind dem Fachmann grundsätzlich bekannt. Vorteilhaft an der Verwendung organischer leitfähiger und/oder halbleitender Materialien ist insbesondere, dass diese keine aufwändigen anorganischen Halbleiterprozesse erfordern, welche möglicherweise mit nasschemischen oder anderen chemischen Prozessen zur Herstellung der Sensorvorrichtung, insbesondere mit der Detektorkammer und der Detektorsubstanz, technisch nicht kompatibel sein könnten.

Die Integration mindestens eines organischen leitfähigen oder halbleitenden Materials, insbesondere eines organischen leitfähigen oder halbleitenden Polymers, kann auf verschiedene Weisen erfolgen. Beispielsweise kann der elektrische Sensor einen Schichtaufbau umfassen. Wie oben beschrieben, kann die Sensorelektrode beispielsweise lediglich eine Oberfläche des elektrischen Sensors umfassen oder kann auch eine oder mehrere Schichten umfassen. Umfasst die mindestens eine Sensorelektrode eine oder mehrere Elektrodenschichten, so können diese beispielsweise organischer und/oder anorganischer Natur sein, wobei auch Kombinationen, also Hybride, möglich sind. Beispielsweise ist bei einem organischen Feldeffekttransistor ein Schichtaufbau möglich, bei welchem mindestens ein organischer Leiter und/oder Halbleiter vorgesehen ist, beispielsweise mindestens ein leitendes oder halbleitendes Polymer. Auf dieses kann mindestens eine Elektrodenschicht organischer und/oder anorganischer Natur aufgebracht sein, oder die Oberfläche des organischen Leiters oder Halbleiters kann selbst als Sensorelektrode fungieren. Als organische Elektrodenschicht kann beispielsweise mindestens ein leitfähiges Polymer dienen, beispielsweise auf Basis von Polyanilin oder auf Basis von Polythiophenen, beispielsweise PEDOT/PSS. Optional kann mindestens eine anorganische Elektrodenschicht vorgesehen sein, beispielsweise eine metallische Elektrodenschicht. Analoge Aufbauten sind grundsätzlich auch möglich, wenn der elektrische Sensor mindestens einen Kondensator umfasst. Auch in diesem Fall kann die mindestens eine Sensorelektrode als Kondensatorelektrode organischer und/oder anorganischer Natur ausgestaltet sein, wobei besonders bevorzugt ist, wenn diese mindestens eine Sensorelektrode mindestens ein leitfähiges oder halbleitendes organisches Material aufweist, beispielsweise mindestens ein organisches leitfähiges oder halbleitendes Polymer.

Wird im Rahmen des mindestens einen elektrischen Sensors, insbesondere für die mindestens eine Sensorelektrode, mindestens ein organisches leitfähiges oder halbleitendes Material verwendet, so kann dieses grundsätzlich auf verschiedene Weisen ausgestaltet werden, die dem Fachmann grundsätzlich bekannt sind. Besonders bevorzugt ist es, wenn das organische leitfähige oder halbleitende Material, insbesondere für die mindestens eine Sensorelektrode, jedoch auch möglicherweise für andere Elemente des elektrischen Sensors, mindestens ein Material aufweist, ausgewählt aus einem konjugierten Polymer, insbesondere einem konjugierten Polymer aus der Gruppe bestehen aus einem Polythiophen, einem Polyphenylenvinylen, einem Polyanilin, einer Polysulfonsäure, einem Fluoren, einem Polyacetylen, einem Polyparaphenylen, einem Polyazulen, einem Polyparaphenylensulfid, einem Polypyrrol, einem Polycarbazol, einem Polydiaminophtalen und/oder Derivaten derselben, also beispielsweise Derivaten, bei welchen Substitutionen vorgenommen wurden, und einem organischen Monomer oder Oligomer mit einem konjugierten Doppelbindungssystem, insbesondere einem Monomer ausgewählt aus der Gruppe bestehend aus Anthracen, Pentacen und einem Triphenylamin und Derivaten derselben.

Wie oben beschrieben, kann die Sensorelektrode insbesondere der Detektorkammer zuweisen und/oder zugeordnet sein und/oder in der Detektorkammer angeordnet sein und/oder auch ganz oder teilweise bauteilidentisch mit der Detektorkammer sein, sodass diese Sensorelektrode von der Detektorkammer aus zugänglich ist und/oder durch die Detektorkammer vorzugsweise elektrisch beeinflussbar ist. Alternativ oder zusätzlich kann die mindestens eine Sensorelektrode jedoch auch außerhalb der Detektorkammer angeordnet sein, sodass beispielsweise zwischen der Detektorkammer und der Sensorelektrode mindestens ein Abstandhalter vorgesehen sein kann. Insbesondere kann der mindestens eine Abstandhalter mindestens eine Abstandhalterschicht umfassen. Beispielsweise kann mindestens eine elektrisch isolierende Abstandhalterschicht vorgesehen sein, vorzugsweise mindestens eine Schicht eines organischen Isolators.

Wie oben beschrieben, ist es besonders bevorzugt, wenn die Sensorvorrichtung vollständig oder teilweise in ein Körpergewebe eines Benutzers implantierbar ist. Besonders bevorzugt ist es, wenn der elektrische Sensor zumindest teilweise als langgestreckter Sensor ausgestaltet ist, wobei der langgestreckte Sensor zumindest die Detektorkammer oder einen Teil desselben und vorzugsweise die Sensorelektrode umfasst. Der langgestreckte Sensor kann insbesondere eingerichtet sein, um zumindest teilweise in ein Körpergewebe eines Benutzers insertiert zu werden. Unter einem langgestreckten Sensor ist dabei ein Sensor zu verstehen, welcher eine Längserstreckung aufweist, die seine laterale Ausdehnung um mindestens einen Faktor 2, vorzugsweise um mindestens einen Faktor 5 und besonders bevorzugt um mindestens einen Faktor 10 übersteigt. Beispielsweise kann der langgestreckte Sensor eine maximale laterale Ausdehnung, senkrecht oder quer zur Längserstreckung, aufweisen, welche 7 mm nicht übersteigt, vorzugsweise 5 mm nicht übersteigt und besonders bevorzugt 3 mm nicht übersteigt oder sogar 2 mm nicht übersteigt. Der Vorteil insbesondere organischer Schichtaufbauten ist darin zu sehen, dass diese äußerst dünn ausgestaltet werden können, beispielsweise in einem Bereich von kleiner als 1 $\mu$m, insbesondere in einem Bereich von kleiner als 500 nm und beispielsweise sogar im Ångströmbereich.

Besonders bevorzugt ist es, wenn der langgestreckte Sensor, insbesondere die Sensorvorrichtung oder mindestens ein Teil der Sensorvorrichtung, zumindest teilweise flexibel und/oder biegsam ausgestaltet ist, sodass dieser beispielsweise um eine Achse quer zur Längserstreckung, beispielsweise eine Achse parallel zu einem Schichtaufbau des langgestreckten Sensors, biegbar ist, beispielsweise unter üblichen, in einem insertierten Zustand im Körpergewebe auftretenden Kräften, beispielsweise Kräften von 5-10 N, sodass beispielsweise eine Auslenkung von Enden des langgestreckten Sensors gegeneinander um mindestens 100 Mikrometer, insbesondere um mindestens 500 Mikrometer oder sogar um mindestens 1 mm bei diesen Kräften möglich ist. Derartige Flexibilität, welche Elastizitätseigenschaften und/oder plastische Eigenschaften beinhalten kann, kann ebenfalls durch organische Schichtaufbauten leicht gewährleistet werden, da diese ohne weiteres flexibel ausgestaltet werden können, beispielsweise durch Wahl entsprechender Substrate. So kann beispielsweise der oben beschriebene bevorzugte Kondensator und/oder der oben beschriebene bevorzugte Feldeffekttransistor, beide Bauelemente insbesondere als organische Bauelemente, mindestens ein Substratmaterial umfassen, beispielsweise ein Glasmaterial und/oder ein Kunststoffmaterial, auf welches eine oder mehrere Schichten, beispielsweise eine oder mehrere organische Schichten, aufgebracht sind. Dieses Substratmaterial kann äußerst dünn ausgestaltet werden, beispielsweise mit einer Dicke von weniger als 500 $\mu$m, insbesondere von weniger als 100 $\mu$m, und kann dementsprechend flexible Eigenschaften aufweisen.

Der langgestreckte Sensor kann insbesondere eine Gestalt aufweisen, ausgewählt aus der Gruppe bestehend aus einer Streifenform und einer Stabform, insbesondere einer Stabform mit einem runden Querschnitt. Unter einer Stabform ist dabei eine zylindrische Form zu verstehen. Wie oben beschrieben, kann der langgestreckte Sensor im Falle einer zylindrischen Form insbesondere einen konzentrischen Aufbau und/oder einen Aufbau mit Elektroden auf Mantelflächen aufweisen.

Wie oben beschrieben, kann die Sensorvorrichtung insbesondere über mindestens eine Schnittstelle verfügen.

Die Sensorvorrichtung kann eingerichtet sein, um über die mindestens eine Schnittstelle, insbesondere über mindestens eine drahtlose Schnittstelle, beispielsweise eine Funkschnittstelle und/oder eine kapazitive Schnittstelle, insbesondere über mindestens einen Transponder, mit einem Lesegerät zu kommunizieren. Auch diese Ausgestaltung ist besonders bevorzugt im Falle eines implantierbaren Sensors.

Die Sensorvorrichtung kann insbesondere eingerichtet sein, um einer Umgebung, insbesondere einem Körpergewebe, Energie zu entnehmen, insbesondere mittels eines sogenannten Energy Harvestings. Auch diese Ausgestaltung ist besonders bevorzugt im Falle eines zumindest teilweise implantierbaren elektrischen Sensors, beispielsweise des oben beschriebenen langgestreckten Sensors. Unter einem Energy Harvesting ist dabei ein Vorgang zu verstehen, bei welchem grundsätzlich beliebige Energieformen einer Umgebung zur Energiegewinnung genutzt werden, beispielsweise ohne dass gezielt ein Energiespeicher und/oder eine Energieversorgung bereitgestellt werden müssen, welche gleichwohl innerhalb des Sensorsystems, insbesondere innerhalb des elektrischen Sensors und/oder innerhalb des langgestreckten Sensors, vorgesehen sein können. So kann beispielsweise aus Temperaturdifferenzen zur Umgebung Energie gewonnen werden. Alternativ oder zusätzlich können beispielsweise Vibrationseffekte und/oder Beschleunigungskräfte genutzt werden, beispielsweise mittels entsprechender piezoelektrischer Vorrichtungen. Wiederum alternativ oder zusätzlich können beispielsweise elektrochemische Prozesse eingesetzt werden, um Energie aus der Umgebung zu gewinnen. Derartige Vorgänge des Energy Harvestings sind dem Fachmann grundsätzlich bekannt. Alternativ oder zusätzlich zu einer Einrichtung zum Energy Harvesting kann die Sensorvorrichtung jedoch, wie oben beschrieben, mindestens eine interne oder externe Energieversorgung aufweisen. Beispielsweise kann mindestens ein elektrischer Energiespeicher innerhalb der Sensorvorrichtung, insbesondere innerhalb des elektrischen Sensors und/oder einer Ansteuerung, vorgesehen sein, beispielsweise mindestens ein Kondensator, insbesondere einen Supercap, mindestens eine Batterie und/oder mindestens ein Akkumulator. Wiederum alternativ oder zusätzlich kann eine Energie auch von außen, beispielsweise zwecks Speicherung und/oder direkter Verwendung, eingebracht werden, beispielsweise indem diese Energie von außen eingestrahlt wird. Verschiedene Möglichkeiten der Energieversorgung sind gegeben.

In einem weiteren Aspekt der vorliegenden Erfindung wird ein Sensorsystem vorgeschlagen. Dieses Sensorsystem umfasst mindestens eine Sensorvorrichtung gemäß einer oder mehrerer der oben beschriebenen Ausgestaltungen. Weiterhin umfasst das Sensorsystem mindestens ein Lesegerät. Die Sensorvorrichtung und das Lesegerät sollen eingerichtet sein, um miteinander zu kommunizieren, insbesondere drahtlos, beispielsweise über eine kapazitive Schnittstelle und/oder eine induktive Schnittstelle und/oder eine Schnittstelle zum Austausch elektromagnetischer Signale, beispielsweise eine Funkschnittstelle. Der Austausch der Signale kann unidirektional in einer der beiden Richtungen oder auch bidirektional erfolgen. Unter einem Lesegerät ist allgemein ein Gerät zu verstehen, welches mit der Sensorvorrichtung kommunizieren kann. Darüber hinaus kann das Lesegerät weitere Funktionen beinhalten. Beispielsweise kann das Lesegerät mindestens eine Ansteuer- und/oder Auswertevorrichtung umfassen, um die Sensorvorrichtung zu mindestens einer Messung einer Analytkonzentration anzusteuern und/oder um eine oder mehrere Signale der Sensorvorrichtung zu empfangen und vollständig oder teilweise auszuwerten. Beispielsweise kann das Lesegerät zu diesem Zweck mindestens einen Mikrocontroller umfassen und/oder eine andere Datenverarbeitungsvorrichtung und/oder mindestens einen Datenspeicher. Das Lesegerät kann weiterhin mindestens eine Benutzerschnittstelle umfassen, um beispielsweise eine Eingabe von Steuerbefehlen durch einen Benutzer zu ermöglichen und/oder um Informationen an einen Benutzer und/oder ein anderes Gerät ausgeben zu können, beispielsweise über mindestens ein Display, mindestens ein Bedienelement wie beispielsweise eine oder mehrere Tasten und/oder andere dem Fachmann grundsätzlich bekannte Benutzerschnittstellen.

In einem weiteren Aspekt der vorliegenden Erfindung wird ein Verfahren zum Nachweis mindestens eines Analyten in einem Fluid vorgeschlagen. Dieses Verfahren kann insbesondere unter Verwendung einer Sensorvorrichtung gemäß einer oder mehrerer der oben beschriebenen Ausgestaltungen durchgeführt werden, sodass bezüglich möglicher Ausgestaltungen des Verfahrens auf die obige Beschreibung der Sensorvorrichtung verwiesen werden kann. Das Verfahren kann zur in vitro-Diagnostik mindestens eines Analyten in einer Körperflüssigkeit eingesetzt werden. Alternativ oder zusätzlich kann das Verfahren jedoch auch zur in vivo-Diagnostik eingesetzt werden, beispielsweise in einem Körpergewebe.

Bei dem Verfahren werden mindestens eine geschlossene Detektorkammer und mindestens ein elektrischer Sensor mit mindestens einer Sensorelektrode verwendet. Der elektrische Sensor ist vorzugsweise elektrisch durch die Detektorkammer beeinflussbar. Die Detektorkammer wird bei dem Verfahren derart mit dem Fluid in Verbindung gebracht, dass der Analyt in die Detektorkammer eindringt. Die Detektorkammer umfasst mindestens eine Detektorsubstanz, welche mindestens eine elektrische Eigenschaft des elektrischen Sensors in Abhängigkeit von einer Konzentration des Analyten in der Detektorkammer beeinflusst.

Die oben beschriebene Sensorvorrichtung, das oben beschriebene Sensorsystem und das oben beschriebene Verfahren weisen gegenüber bekannten Vorrichtungen, Systemen und Verfahren zahlreiche Vorteile auf.

Insbesondere die optionale Verwendung organischer leitender und/oder halbleitender Materialien führt zu Vorteilen bei der Herstellung und/oder Verwendung. Organische leitende und/oder halbleitende Materialien können insbesondere mit nasschemischen Verfahren aufgebracht werden, beispielsweise Druckprozessen wie zum Beispiel Inkjet-Drucken, Sieb-Drucken, Flexo-Drucken, Tampon-Drucken oder anderen Drucktechniken. Alternativ oder zusätzlich können Rakeltechniken, Spin Coating, Roller Coating oder andere nasschemische Techniken eingesetzt werden. Wiederum alternativ oder zusätzlich kann eine Abscheidung aus der Gasphase verwendet werden, beispielsweise physikalische Abscheideverfahren wie Aufdampfen und/oder Sputtern, und/oder chemische Abscheideverfahren aus der Gasphase. Das Abscheiden aus der flüssigen Phase kann aus Lösungen, Emulsionen oder Dispersionen erfolgen. Insbesondere unter Verwendung organischer leitender und/oder halbleitender Materialien können gezielt strukturelle Nachweisprozesse mit elektrischen Messverfahren kombiniert werden. Hierdurch lässt sich die serielle Abarbeitung diverser chemischer Reaktionsabläufe vorteilhafterweise vermeiden, und es lässt sich insbesondere direkt in einer der ersten Prozessstufen ein elektrisch verwertbares Signal generieren. Durch Abkürzung von Signalverarbeitungsschritten werden gegebenenfalls Fehlerquellen und/oder Störquellen, wie beispielsweise Rauscheinflüsse, reduziert.

Insbesondere kann im Rahmen der vorliegenden Erfindung ein biogener Analyt, beispielsweise eine in einer Messflüssigkeit vorliegende Glucose, direkt auf elektrisch detektierbare Größen einwirken und unter Zuhilfenahme beispielsweise affiner Wechselwirkungen, mittels elektronischer Methoden, dessen Konzentration ermittelt werden.

Die Sensorvorrichtung kann mehrteilig oder auch einteilig ausgestaltet sein, wobei insbesondere sogar monolytische Aufbauten möglich sind. So lässt sich beispielsweise durch die Kombination spezifischer modifizierter Polymermaterialien mindestens ein Polymermonolit herstellen, welcher beispielsweise mehrere oder alle unterschiedliche Funktionsstufen umfasst. Diese Funktionsstufen können bezüglich ihrer Materialien und/oder ihrer Herstellungsprozesse aufeinander abgestimmt sein. Insbesondere können Teile der Sensorvorrichtung und/oder die ganze Sensorvorrichtung in einem sogenannten Reel-to-Reel-Prozess preisgünstig gefertigt werden.

Der Analytnachweis im Rahmen der vorliegenden Erfindung kann insbesondere, wie oben beschrieben, ohne chemische Umsetzungen ausgestaltet werden. Damit verbunden ist der Vorzug, dass vorzugsweise ein Verbrauch der Detektorsubstanz und/oder Teilen derselben und/oder ein Verbrauch des Analyten selbst nicht auftritt. Beispielsweise kann auf diese Weise ein Verbrauch von Glucose und deren Reaktanden vermieden werden. Weiterhin kann vermieden werden, dass Reaktionsprodukte auftreten, beispielsweise Gluconolacton, Wasserstoffperoxid und/oder Wasserstoff, welche gegebenenfalls ansonsten aus der Reaktionszone, beispielsweise der Detektorkammer, abgeführt werden müssten, um beispielsweise ein Bersten der Detektorkammer und/oder eine negative Beeinflussung der Detektorkammer durch gasförmige Produkte zu vermeiden. Der Nachweis des Analyten läuft vorzugsweise über die Einstellung eines nur von der Analytkonzentration abhängigen Verteilungsgleichgewichts. Dieses und dessen konzentrationsabhängige Dynamik können registriert werden, über die oben beschriebene mindestens eine elektrische Eigenschaft des elektrischen Sensors.

Beispielsweise kann ein nachzuweisender Analyt statistisch und vorzugsweise reversibel, insbesondere zeitlich limitiert, Analyt-spezifische Bindungsstellen eines Rezeptors besetzen und diese gegebenenfalls wieder freigeben. Der Belegungsgrad und/oder Besetzungsgrad ist bezüglich der zeitlichen Dynamik in der Regel dann nur abhängig von der diffusionsbedingten Analytkonzentration und den Affinitäten. Die Sensorvorrichtung und insbesondere die Detektorkammer und/oder die Detektorsubstanz können daher insbesondere derart ausgelegt werden, dass der diagnostisch relevante Bereich, insbesondere die Detektorkammer, durch das Verhältnis der Bindungsstellen zur maximalen Analytkonzentration abgedeckt ist.

Aufgrund der bevorzugten Ausgestaltung, bei welcher kein Verbrauch von Detektorsubstanz und/oder Analyt stattfindet, kann der Nachweisprozess in vitro theoretisch unbegrenzt lange ablaufen. In vivo können zeitliche Grenzen nicht durch Verbrauch und Reaktionsprodukte, sondern allein durch Reparaturmaßnahmen des Gewebes vorgegeben sein, beispielsweise durch eine Einkapselung, Heilungsprozesse oder dergleichen, in der Regel jedoch nicht durch Sensor-seitige Vorgänge limitiert sein.

Da vorteilhafterweise eine Gleichgewichtseinstellung, insbesondere innerhalb der Detektorkammer, bei Abwesenheit von Analyt umkehrbar ist, können nach Durchführung eines definierten Abreicherungs-Prozesses, gefolgt von einer definierten Konzentrations-Vorlage, Relativkalibrationen der vorgeschlagenen Sensorvorrichtung durchgeführt werden, insbesondere seriell. So können beispielsweise, insbesondere in vitro gemessene, Messwerte einer definierten Referenzlösung zu einem in vivo-Messwert ins Verhältnis gesetzt werden, ohne dass das Sensorelement durch diesen Vorgang unbrauchbar wird, im Gegensatz beispielsweise zu herkömmlichen Teststreifen.

Da die beteiligten chemischen Substanzen, insbesondere der mindestens einen Detektorsubstanz, vorzugsweise elektrochemisch passiv ausgestaltet sein können, können diese in der Regel in quasi-wässriger Lösung oder in wässriger Lösung arbeiten, beispielsweise in interstitieller Flüssigkeit. So kann beispielsweise die Detektorkammer mit einem fluiden Medium gefüllt werden, welches unmittelbar von seiner Grundzusammensetzung her dem Fluid, in welchem der Analyt nachzuweisen ist, entspricht oder welches beispielsweise chemisch ähnlich zu diesem aufgebaut sein kann, beispielsweise indem isotonische Lösungen verwendet werden. Prinzipiell sind die vorgeschlagene Sensorvorrichtung und das vorgeschlagene Verfahren jedoch auch für den Einsatz in nicht-flüssigen Fluiden geeignet, beispielsweise in Gasen.

Insbesondere für den Einsatz im Humanbereich ist es besonders bevorzugt, wenn die Sensorvorrichtung vollständig oder teilweise biokompatibel ausgestaltet wird, insbesondere ein in ein Körpergewebe insertierbarer Teil der Sensorvorrichtung, beispielsweise der oben beschriebene langgestreckte Sensor. Dies kann beispielsweise dadurch gewährleistet werden, dass biokompatible Werkstoffe eingesetzt werden, beispielsweise biokompatible organische Materialien. Alternativ oder zusätzlich kann die Sensorvorrichtung jedoch auch vollständig oder teilweise mit einer Kapselung versehen werden, beispielsweise einer Schutzhülle, welche biokompatibel ist und welche beispielsweise eingerichtet ist, um ein Austreten von Substanzen der Sensorvorrichtung in das umgebende Körpergewebe und/oder Reaktionen des Körpergewebes mit der Sensorvorrichtung oder Teilen derselben zu verhindern. Die optionale Verkapselung kann getrennt von dem optionalen Gehäuse der Detektorkammer ausgestaltet sein, kann jedoch auch ganz oder teilweise in dieses integriert werden oder gemeinsam mit diesem ausgebildet werden. Verkapselungen sind aus dem Bereich implantierbarer Sensorelemente dem Fachmann grundsätzlich bekannt. Insbesondere können auf diese Weise allergische Reaktionen vermieden werden.

Weiterhin können im Rahmen der vorliegenden Erfindung auch die Erfordernisse einer Sterilisation beachtet werden. Im Gegensatz zu elektrochemischen Sensoren können die erfindungsgemäßen Sensorvorrichtungen in der Regel nicht nur strahlensterilisiert werden, sondern gegebenenfalls auch thermisch und/oder mit chemischen Methoden sterilisiert werden.

Die Erfindung ist beschrieben in den unabhängigen Ansprüchen. Bevorzugte Ausführungsbeispiele sind beschrieben in den abhängigen Ansprüchen.

Im Einzelnen zeigen:

| | |
|---|---|
| Figur 1A | eine Prinzipskizze eines Beispiels einer Sensorvorrichtung mit einem Operationsverstärker; |
| Figur 1B | ein weiteres Beispiel einer Sensorvorrichtung mit einem Operationsverstärker; |
| Figuren 2A und 2B | ein Ausführungsbeispiel einer Sensorvorrichtung der Erfindung mit einem Feldeffekttransistor mit Analyt (Figur 2A) und ohne Analyt (Figur 2B); |
| Figuren 3A und 3B | ein Ausführungsbeispiel einer Sensorvorrichtung der Erfindung mit einem Kondensator, mit Analyt (Figur 3A) und ohne Analyt (Figur 3B); |
| Figur 4 | ein Ausführungsbeispiel einer Sensorvorrichtung mit einem zylindrischen Kondensator; |
| Figur 5 | ein Blockschaltbild einer möglichen Ausgestaltung einer Sensorvorrichtung; |
| Figur 6 | ein Blockschaltbild eines Ausführungsbeispiels eines Sensorsystems; und |
| Figur 7 | ein Ausführungsbeispiel einer Sensorvorrichtung innerhalb einer Messbrücke. |

Ausführungsbeispiele

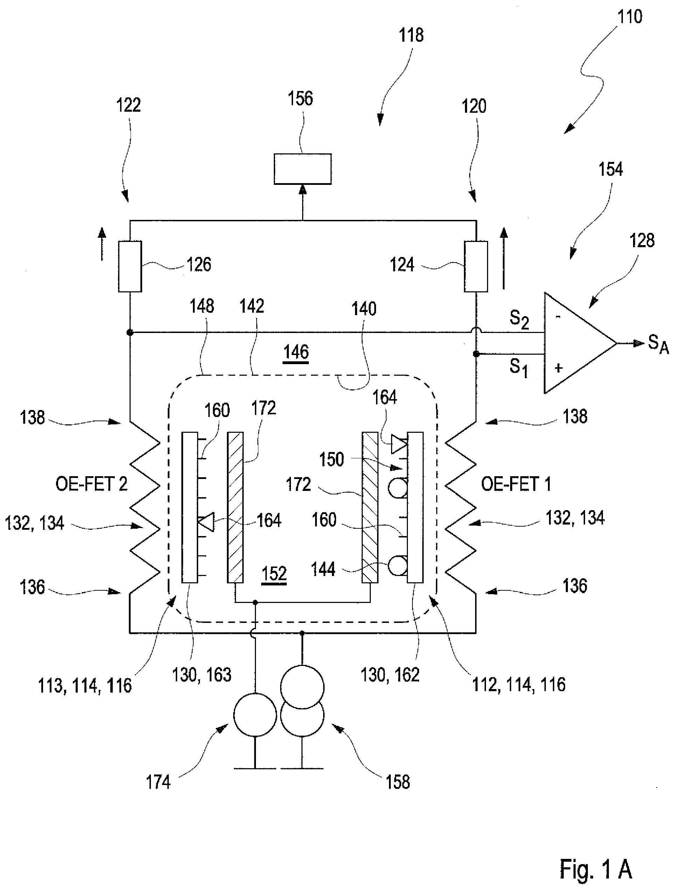

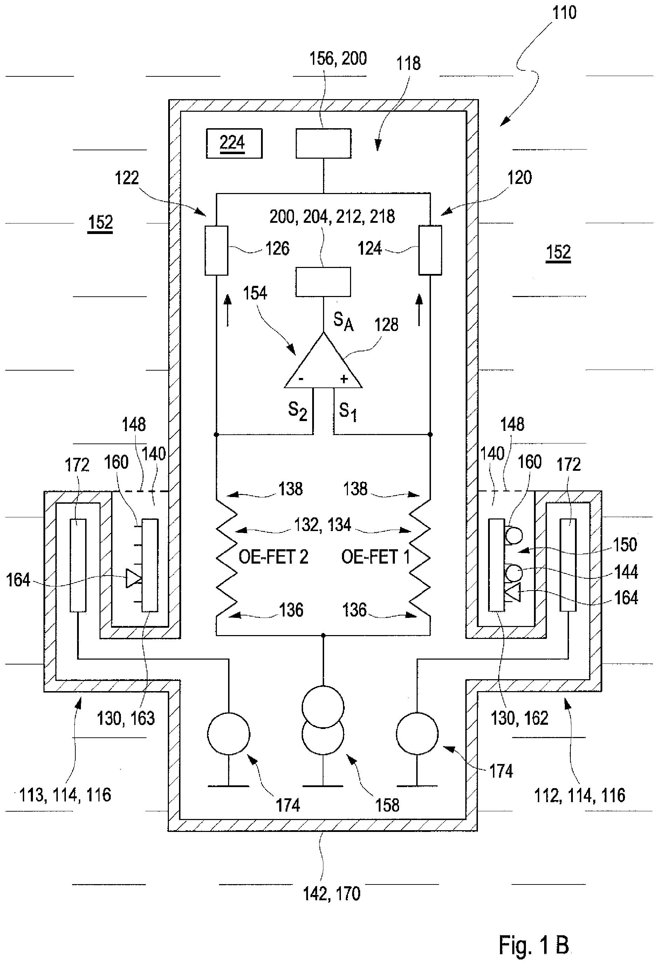

In den Figuren 1A und 1B sind in stark schematisierter Darstellung Ausführungsbeispiele einer Sensorvorrichtung 110 dargestellt, die auf der Verwendung eines oder mehrerer elektrischer Sensoren 112 basieren, die in diesen Ausführungsbeispielen als Feldeffekttransistoren 114 ausgestaltet sind, vorzugsweise als organische Feldeffekttransistoren 116, in den Figuren 1A und 1B auch mit OE-FET 1 und OE-FET 2 bezeichnet. In den Ausführungsbeispielen gemäß den Figuren 1A und 1B oder auch in anderen Ausgestaltungen können die Sensorvorrichtungen 110 jeweils mindestens einen elektrischen Sensor 112 mit Analyt-spezifischen Eigenschaften aufweisen sowie optional mindestens einen Kompensationssensor 113, welcher zumindest im Wesentlichen unbeeinflusst durch eine Analyt-Konzentration ist. In den Figuren 1A und 1B stellt jeweils der OE-FET 1 den elektrischen Sensor 112 dar, und der OE-FET 2 stellt den Kompensationssensor 113 dar.

Mit halbleitenden oder leitenden Polymeren lassen sich bereits heute Feldeffekttransistoren 114, 116 mit guten digitalen Schalteigenschaften herstellen. Prinzipiell stellt ein Feldeffekttransistor 114, 116 einen spannungsgesteuerten Widerstand dar und ist ein Element, mit welchem analoge Signale verarbeitet werden können. In der heutigen Elektronik stellen in der Regel Operationsverstärker 118 auf Siliciumbasis zentrale Elemente zur Signalverstärkung dar. Operationsverstärker 118 arbeiten in der Regel bildlich gesprochen nach dem Prinzip einer Balkenwaage, wobei in der Regel ein konstanter Strom zwischen zwei steuerbaren Widerstandszweigen 120, 122 aufgeteilt wird. In den Figuren 1A und 1B umfasst exemplarisch die Sensorvorrichtung 110 einen derartigen Operationsverstärker 118 mit den beiden Widerstandszweigen 120, 122, in welche die Feldeffekttransistoren 114 OE-FET 1 (elektrischer Sensor 112) und OE-FET 2 (Kompensationssensor 113) eingebunden sind. Diese Widerstandszweige 120, 122 setzen sich beispielsweise aus den steuerbaren Feldeffekttransistoren 114 und Festwiderständen 124, 126 zusammen. Durch Anlegen von Steuersignalen an diese Feldeffekttransistoren 114 kann die im Ruhezustand ausbalancierte "Stromwaage" aus dem Gleichgewicht gebracht werden. Dadurch ändert sich der Spannungsabfall über den Festwiderständen 124, 126, beispielsweise in gleichem Maße. Die beiden Spannungsabfälle können in einer nachfolgenden Stufe (Subtrahierstufe 128) subtrahiert und das Ergebnis gegebenenfalls weiterverstärkt werden. Bei herkömmlichen Operationsverstärkern 118 bilden die beiden steuerbaren Eingänge die differentiellen Eingänge des Operationsverstärkers 118. Liegen an den differentiellen Eingängen identische Signale an, wird das Ausgangssignal, welches in den Figuren 1A und 1B mit "$S_A$" bezeichnet ist, abgesehen von möglichen Offsets, zu Null. Weichen die Eingangssignale hingegen voneinander ab, so wird die Differenz verstärkt.

Gesteuert werden die steuerbaren Feldeffekttransistoren 114, indem ein Elektrodenpotenzial, eine Ladung oder eine Spannung auf sogenannte Gate-Elektroden 130 aufgebracht wird. Diese wirken, in der Regel isoliert, auf einen Bahnwiderstand eines Halbleiters 132, in den Figuren 1A und 1B insbesondere organischen Leiters oder Halbleiters 134 der Feldeffekttransistoren 114 ein, welcher eine Source-Elektrode 136 mit einer Drain-Elektrode 138 der Feldeffekttransistoren 114 verbindet, die in den Figuren 1A und 1B, wie auch der organische Halbleiter 134, lediglich angedeutet sind. Ist beispielsweise die Gate-Elektrode 130 mit einer negativen Ladung beaufschlagt, so kann die Widerstandsstrecke, also der Bahnwiderstand, des organischen Halbleiters 134 verarmen, und es ändert sich, je nach Dotierung des organischen Halbleiters 134 und/oder dessen Eigenschaften (beispielsweise Verarmungstyp oder Anreicherungstyp) dessen elektrischer Widerstand.

Im Rahmen der vorliegenden Erfindung und der vorliegenden Sensorvorrichtung 110 wird diese Ladungssteuerung und/oder Spannungssteuerung an der Gate-Elektrode 130 nicht durch Anlegen einer externen Spannungsquelle erreicht, sondern indem Analyt-abhängig elektrische Ladungen auf die Gate-Elektrode 130 gebracht und/oder von dieser entfernt werden und/oder indem allgemein eine Ladungsverteilung innerhalb einer Detektorkammer 140 verändert wird. Bei organischen Halbleitern 134 kann eine Einflussnahme beispielsweise mit intrinsischer oder auch mittels äußerer Ladungsträger erfolgen, insbesondere indem $\pi$-Elektronen der organischen Halbleiter 134, beispielsweise eines Polymers, beeinflusst werden.

Die Sensorvorrichtungen 110 gemäß den Figuren 1A und 1B weisen dementsprechend in diesen Ausführungsbeispielen den elektrischen Sensor 112 und den Kompensationssensor 113, jeweils in Form eines oder mehrerer Feldeffekttransistoren 114, insbesondere organischer Feldeffekttransistoren 116, auf, deren Gate-Elektroden 130 durch Vorgänge innerhalb der mindestens einen Detektorkammer 140 beeinflussbar sind. Der elektrische Sensor 112 und der Kompensationssensor 113 unterscheiden sich dabei, wie unten noch näher ausgeführt wird, darin, ob diese Beeinflussung von der Konzentration des Analyten in dem fluiden Medium 152 abhängig ist oder nicht. Während die Beeinflussung der Gate-Elektrode 130 des Kompensationssensors 113, welche als Kompensations-Sensorelektrode 163 wirkt, zumindest weitgehend unabhängig von der Konzentration des Analyten ist, erfolgt bei dem elektrischen Sensor 112 und dessen als Sensorelektrode 162 fungierender Gate-Elektrode 130 eine von der Konzentration des Analyten abhängige Beeinflussung der elektrischen Eigenschaften.

In beiden Ausführungsbeispielen sind die als Sensorelektrode 162 bzw. als Kompensations-Sensorelektrode 163 wirkenden Gate-Elektroden 130 des elektrischen Sensors 112 und des Kompensationssensors 113 exemplarisch in mindestens einer Detektorkammer 140 angeordnet und/oder mit mindestens einer Detektorkammer 140 verbunden. Dabei können die Sensorelektrode 162 und die Kompensations-Sensorelektrode 163 in und/oder an derselben Detektorkammer 140 angeordnet sein oder auch in unterschiedlichen Detektorkammern 140. In der mindestens einen Detektorkammer 140 ist mindestens ein fluides Medium 152 aufgenommen, welches vorzugsweise in reversiblem Analytaustausch mit einem Fluid 146 außerhalb der Detektorkammer 140 steht, beispielsweise über mindestens eine Membran 148.

Die Ausführungsbeispiele in den Figuren 1A und 1B unterscheiden sich unter anderem in der Ausgestaltung der Detektorkammern 140. Während das Ausführungsbeispiel in Figur 1A die Sensorvorrichtung 110 und die Anordnung der Detektorkammer 140 sowie des Fluids 146 lediglich schematisch zeigt, ist in Figur 1B ein Ausführungsbeispiel gezeigt, bei welcher eine Ansteuerung 154 der Sensorvorrichtung 110 gekapselt ausgestaltet ist. Allgemein können empfindliche elektronische Bauelemente der Sensorvorrichtung 110, insbesondere einer Ansteuerung 154 der Sensorvorrichtung 110, gegenüber dem Fluid 146 und/oder dem fluiden Medium 152 einzelnen, gruppenweise oder insgesamt gekapselt ausgestaltet werden. So ist in Figur 1A das Fluid 146 lediglich schematisch außerhalb der Detektorkammer 140 dargestellt, so dass beispielsweise ein Analytaustausch mit dem fluiden Medium 152 innerhalb der Detektorkammer 140 erfolgen kann.

Vorzugsweise kommt das Fluid 146 dabei jedoch nicht unmittelbar mit elektronischen Bauelementen außerhalb der Detektorkammer 140 in Kontakt, beispielsweise durch eine entsprechende Kapselung dieser Bauelemente. In dem Ausführungsbeispiel gemäß Figur 1B hingegen ist ein Gehäuse 142 vorgesehen, welches, in Zusammenwirkung mit der Membran 148, einen Innenraum des Gehäuses 142, in welchem die Ansteuerung 154 aufgenommen ist, die Detektorkammer 140 mit dem fluiden Medium 152 und einen Außenraum mit dem Fluid 146 voneinander trennt. Diese Trennung bewirkt, dass weder das Fluid 146 noch das fluide Medium 152 in den Innenraum zu der Ansteuerung 154 eindringen können. Weiterhin bewirkt die Trennung, dass ein Analytaustausch zwischen dem Fluid 146 und dem fluiden Medium 152 möglich ist.

Die Ausgestaltung der Detektorkammer 140 in der Darstellung gemäß Figur 1B ist lediglich exemplarisch zu verstehen, wobei auch zahlreiche andere Ausgestaltungen möglich sind. Beispielsweise kann die Detektorkammer 140, wie in Figur 1B dargestellt, in Form einer oder mehrerer Vertiefungen, Ringspalte, Taschen oder anderer Hohlräume ausgestaltet sein, welche durch das Gehäuse 142 gebildet werden und welche gegenüber dem Au-ßenraum durch die Membran 148 getrennt sind. Auch andere Ausgestaltungen sind jedoch grundsätzlich möglich.

Weiterhin sind in den Ausgestaltungen gemäß den Figuren 1A und 1B jeweils die Gate-Elektroden 130 exemplarisch innerhalb der Detektorkammer 140 angeordnet, was ebenfalls nicht notwendigerweise der Fall sein muss, da diese Gate-Elektroden 130 lediglich von der Detektorkammer 140 aus elektrisch beeinflussbar sein sollten.

Zusätzlich zu der mindestens einen Sensorelektrode 162 kann der elektrische Sensor 112 in diesem oder auch in anderen Ausführungsbeispielen mindestens eine Gegenelektrode 172 aufweisen, welche elektrisch kontaktiert oder auch nichtkontaktiert sein kann. Die Gegenelektrode 172 kann durch die Analytkonzentration unbeeinflusst sein. Ebenso kann auch optional der mindestens eine Kompensationssensor 113 mindestens eine Gegenelektrode 172 aufweisen, so dass beispielsweise wiederum, abgesehen von der Sensitivität gegenüber dem Analyten, der elektrische Sensor 112 und der Kompensationssensor 113 zumindest im Wesentlichen baugleich ausgestaltet sein können. Die Ausführungsbeispiele in den Figuren 1A und 1B zeigen optional derartige Ausgestaltungen mit Gegenelektroden 172. Die Gegenelektroden 172 können beispielsweise ganz oder teilweise innerhalb oder ganz oder teilweise außerhalb der Detektorkammer 140 angeordnet sein. So ist in Figur 1A exemplarisch eine Ausgestaltung gezeigt, bei welcher die Gegenelektroden 172 innerhalb der Detektorkammer 140 angeordnet sind, während in Figur 1B eine Ausgestaltung gezeigt ist, bei welcher die Gegenelektroden 172 außerhalb der Detektorkammer 140 angeordnet sind, beispielsweise im Innenraum des Gehäuses 142. Auch eine andere Ausgestaltungen ist jeweils möglich. Insbesondere kann die Detektorkammer 140 ganz oder teilweise zwischen der Sensorelektrode 162 bzw. der Kompensations-Sensorelektrode 163 und der jeweiligen Gegenelektrode 172 angeordnet sein. Auch andere Ausgestaltungen sind jedoch denkbar.

Wie oben ausgeführt, kann mindestens eine Ansteuerung 154 vorgesehen sein, welche ganz oder teilweise gegenüber dem Fluid 146 und/oder dem fluiden Medium 152 gekapselt sein kann. Die Detektorkammer 140 ist beispielsweise als Flüssigkeits-Kompartement ausgestaltet. Die Detektorkammer 140 kann beispielsweise ein Gehäuse 142 aufweisen oder das Gehäuse 142 kann beispielsweise wie in Figur 1B dargestellt mindestens einen Teil, insbesondere mindestens einen Teil einer Wand, der Detektorkammer 140 bilden. Das Gehäuse 142 kann beispielsweise vollständig oder teilweise durchlässig für einen nachzuweisenden Analyten 144 in einem die Detektorkammer 140 vollständig oder teilweise umgebenden Fluid 146 sein, beispielsweise indem dieses eine oder mehrere Membranen 148 aufweist. In dem in Figur 1B dargestellten Ausführungsbeispiel ist das Gehäuse 142 vorzugsweise hermetisch dicht, insbesondere vollständig undurchlässig für den nachzuweisenden Analyten 144 und/oder das Fluid 146 und/oder das fluide Medium 152, beispielsweise um einen Kurzschluss, insbesondere zwischen elektronischen Komponenten, beispielsweise ein Überbrücken, zu verhindern. Unter einem Kurzschluss kann hierbei insbesondere ein elektrischer Widerstand von kleiner als $10^9$ Ohm verstanden werden. Diese Undurchlässigkeit kann beispielsweise lediglich durch die Membran 148 unterbrochen sein, welche, wie oben ausgeführt, einen Analytaustausch, beispielsweise einen reversiblen Analytaustausch, zwischen dem Fluid 146 und dem fluiden Medium 152 gewährleisten kann. Das Gehäuse 142 kann wie in Figur 1B dargestellt beispielsweise ganz oder teilweise die Ansteuerung 154 und/oder die Drain-Elektrode 138 und/oder die Source-Elektrode 136 und/oder den Halbleiter 132 und/oder die Gegenelektrode 172 und/oder einen Kompensationssensor 113 und/oder den elektrischen Sensor 112 umgeben. Beispielsweise können das Gehäuse 142 und/oder die Detektorkammer 140 und/oder ein Teil derselben mindestens eine Membran 148 aufweisen, welche durchlässig für den Analyten 144 ist, welche jedoch eingerichtet ist, um Bestandteile einer in Figur 1B lediglich angedeuteten Detektorsubstanz 150 in der Detektorkammer 140 zurückzuhalten. Die Detektorkammer 140 kann beispielsweise vollständig oder teilweise mit einem fluiden Medium 152 gefüllt sein, beispielsweise einem fluiden Medium 152, welches identisch mit dem Fluid 146 sein kann und/oder welches chemisch ähnlich zu dem Fluid 146 ausgestaltet werden kann. Die Gate-Elektrode 130 sollte vorzugsweise isoliert, insbesondere hochohmig getrennt, gegenüber der Source-Elektrode 136 und/oder der Gate-Elektrode 130 sein und/oder vorzugsweise auf definiertem Potenzial vorgespannt sein.

Weiterhin umfassen die Sensorvorrichtungen 110 in den in den Figuren 1A und 1B dargestellten Ausführungsbeispielen, wie oben ausgeführt, optional jeweils die mindestens eine Ansteuerung 154, welche die Widerstandszweige 120, 122, die Subtrahierstufe 128 sowie optional eine Versorgungsspannung 156 und/oder eine Stromquelle 158 umfassen kann, die in den Figuren 1A und 1B lediglich angedeutet sind. Die Versorgungsspannung 156 und/oder die Stromquelle 158 können mit den parallel geschalteten Widerstandszweigen 120, 122 verbunden sein. Die Subtrahierstufe 128 kann beispielsweise jeweils Signale S1 und S2 zwischen den Feldeffekttransistoren 114 und den zugehörigen Festwiderständen 124 bzw. 126 in den jeweiligen Widerstandszweigen 120, 122 abgreifen.

Die Detektorkammer 140, wie beispielsweise in Figur 1B dargestellt, kann auf verschiedene Weise ausgestaltet werden. Alternativ oder zusätzlich zu der Ausgestaltung mit mindestens einem Gehäuse 142 kann die Detektorkammer 140 beispielsweise auch ganz oder teilweise mit einem flüssigkeitsdurchlässigen Feststoffmaterial einer flüssigkeitsdurchlässigen Feststoffstruktur ausgefüllt und/oder umschlossen sein, beispielsweise einem porösen Material, beispielsweise einem porösen, flüssigkeitsdurchlässigen Schaumstoff. Als Beispiele wären Polyurethane oder andere poröse Schaumstoffe zu nennen. Verschiedene Ausgestaltungen sind möglich, wobei vorzugsweise der Feldeffekttransistor 114 und/oder elektrische Kontakte nicht von dem Fluid 146 und/oder dem fluiden Medium 152 kurzgeschlossen, insbesondere elektrisch kurzgeschlossen, werden sollten.

Weitere mögliche Ausgestaltungen betreffen die Detektorsubstanz 150, welche ebenfalls auf verschiedene Weisen ausgestaltet sein kann. Beispielsweise kann in der Detektorkammer 140, beispielsweise als Bestandteil der Detektorsubstanz 150, mindestens ein Rezeptor 160 aufgenommen sein, welcher eingerichtet ist, um den Analyten 144, beispielsweise Glucose, zu binden, insbesondere reversibel. In den in den Figuren 1A und 1B dargestellten Ausführungsbeispielen kann dieser Rezeptor 160 beispielsweise an den Gate-Elektroden 130 und/oder an einer der Gate-Elektroden 130 angeordnet sein. Beispielsweise kann diese Gate-Elektrode 130 durch einen oder mehrere derartiger Rezeptoren 160 besetzt sein.

Besonders bevorzugt ist es, wenn die Gate-Elektroden 130 unterschiedlich ausgestaltet sind, sodass eine dieser Gate-Elektroden 130, in den Figuren 1A und 1B die rechte der Gate-Elektroden 130, als Sensorelektrode 162 des elektrischen Sensors 112 wirkt. Beispielsweise kann ausschließlich die Sensorelektrode 162 mit Rezeptoren 160 besetzt sein, oder die Rezeptoren 160 der Gate-Elektroden 130 können unterschiedlich ausgestaltet sein, sodass beispielsweise die nicht als Sensorelektrode 162 ausgestaltete Gate-Elektrode 130, in den Figuren 1A und 1B die linke der Gate-Elektroden 130, keine Moleküle binden kann oder lediglich unspezifische Moleküle 164 bindet, unabhängig davon, ob es sich um Glucose handelt oder nicht. Die linke der Gate-Elektroden 130 kann insbesondere als Kompensations-Sensorelektrode 163 betrieben werden, wobei die Kompensations-Sensorelektrode 163 insbesondere von dem Kompensationssensor 113 umfasst werden kann. Der Kompensationssensor 113 und/oder die Kompensations-Sensorelektrode 163 können insbesondere mindestens eine Referenz und/oder mindestens einen Referenzwert liefern, beispielsweise um in einem Verfahren zur Kalibrierung und/oder zur Eichung und/oder zur Diagnose von mindestens einer Fehlfunktion, insbesondere im Rahmen des Nachweises des Analyten 144, verwendet zu werden. Die Kompensations-Sensorelektrode 113 kann bevorzugt mindestens eine Fehlergröße, beispielsweise mindestens einen Offset und/oder einen Gleichlauf, kompensieren.

Der Rezeptor 160 kann beispielsweise derart ausgestaltet sein, dass dieser mit dem Analyten 144, beispielsweise Glucose, in Verbindung steht, beispielsweise wie ein Antigen zum Antikörper. Weiterhin wurden beispielsweise von M. McAlpine in "Nanotechnology-Enabled Flexible Sensors for Medical Diagnostics", Vortrag auf "Organic Electronic Congress", Berlin, 27.-29.10.2008 insbesondere Hybrid Nanowire Sensoren vorgestellt sowie Möglichkeiten einer Kopplung von Peptiden als Rezeptor 160 an Nanaowires diskutiert, insbesondere zur Entwicklung elektronischer Nasen. Auch dies ist im Rahmen der vorliegenden Erfindung realisierbar.

Die Bindungen zwischen Rezeptor 160 und Analyt 144, welche auf verschiedene Weise ausgestaltet sein können, können insbesondere reversibel sein und können dann vorzugsweise nur auf einer Affinität des zu detektierenden Analyten 144, beispielsweise eines zu detektierenden Moleküls, zur Gate-Oberfläche und/oder zum Rezeptor 160 beruhen. Vorzugsweise erfolgt keine chemische Umsetzung, und es wird kein Reagens der Detektorsubstanz 150 verbraucht. Im statistischen Mittel kann sich so insbesondere ein mittleres Ladungsträger-Mobilitätsprofil herausbilden, das, integriert über die Zeit, allein zur Analytkonzentration proportional ist. Um dieses Integral zu bilden, kann der Operationsverstärker 118 beispielsweise, was in den Figuren 1A und 1B nicht dargestellt ist, mit einem Kondensator gekoppelt oder rückgekoppelt werden, womit eine weitere Grundschaltung der Signalverarbeitung, ein analoger Integrator, entstehen kann. Die Sensorvorrichtung 110 in diesen Ausführungsbeispielen oder auch allgemein im Rahmen der Erfindung kann also auch als Integrator ausgestaltet sein oder einen derartigen Integrator umfassen.

Die mittlere Bindungsstellenbelegung wird in beiden Widerstandszweigen 120, also in beiden Kanälen des Operationsverstärkers 118, in der Regel durch unspezifische Prozesse, beispielsweise über die unspezifischen Moleküle 164, einen relativ hohen unspezifischen Signaloffset aufweisen. Durch die Differenzverstärkung des Operationsverstärkers 118 wird dieser Offset jedoch in der Vorrichtung gemäß den Figuren 1A und 1B in der Regel zumindest weitgehend kompensiert, da beide Feldeffekttransistoren 114, 116 vorzugsweise räumlich nah beieinander sitzen. Vorzugsweise weist die Sensorvorrichtung 110 in diesen oder auch in anderen Ausführungsbeispielen also, wie oben ausgeführt, mindestens zwei Feldeffekttransistoren 114, vorzugsweise mindestens zwei organische Feldeffekttransistoren 116 auf, insbesondere beispielsweise mindestens ein Feldeffekttransistor 116 umfasst von mindestens einem elektrischen Sensor 112 und mindestens einem Feldeffekttransistor 116 umfasst von mindestens einem Kompensationssensor 113. Die Gate-Elektroden 130 der Feldeffekttransistoren 116 können beispielsweise in oder an mindestens einer gemeinsamen Detektorkammer 140 oder auch in oder an getrennten Detektorkammern 140 angeordnet sein und/oder können vorzugsweise dicht beieinander liegen, beispielsweise in einem Abstand von nicht mehr als 5 mm, insbesondere von nicht mehr als 2 mm, besonders bevorzugt von nicht mehr als 1 mm oder sogar nicht mehr als 500 $\mu$m. Die Feldeffekttransistoren 114, 116 können dabei vorzugsweise in ihrer Grundstruktur identisch sein, wobei jedoch vorzugsweise die Feldeffekttransistoren 114, 116 unterschiedliche spezifische Eigenschaften hinsichtlich des Analyten 144 aufweisen. Insbesondere kann einer der Feldeffekttransistoren 114, 116, insbesondere der Feldeffekttransistor 114, welcher von dem elektrischen Sensor 112 umfasst wird, einen oder mehrere Analyt-spezifische Rezeptoren 116 aufweisen. Beide Feldeffekttransistoren 114, 116 können darüber hinaus auf das umgebende Medium, beispielsweise ein Interstitium, in gleicher Weise reagieren. Ist der Analyt 144 vorhanden, so wird der Analyt-spezifische Feldeffekttransistor 114, 116 mit seiner Sensorelektrode 162 reagieren, insbesondere im Gegensatz zu dem anderen Feldeffekttransistor 114, welcher beispielsweise von dem Kompensationssensor 113 umfasst wird und beispielsweise eine Kompensationssensor-Elektrode 163 aufweisen kann, sodass das Gleichgewicht im Operationsverstärker 118 gestört wird, was sich wiederum im Ausgangssignal bemerkbar macht.

Damit die elektrische Anordnung nicht durch das fluide Medium 152 der Detektorkammer 140, beispielsweise das Interstitium, kurzgeschlossen wird, können sich die Bahnwiderstände zwischen den Source-Elektroden 136 und den Drain-Elektroden 138, insbesondere die Halbleiter 132 bzw. organischen Halbleiter 134, außerhalb der Detektorkammer 140 befinden, beispielsweise isoliert außerhalb des fluiden Mediums 152, insbesondere des Interstitiums. Die Bahnwiderstände sollten vorzugsweise gegenüber den Gate-Elektroden 130 isoliert sein, wobei ein Abstand zwischen den Bahnwiderständen und den Gate-Elektroden 130 vorzugsweise möglichst klein gewählt werden sollte, beispielsweise kleiner als 1 $\mu$m. Lediglich die Gate-Elektroden 130 können sich beispielsweise innerhalb der Detektorkammer 140 befinden und/oder dem fluiden Medium 152 innerhalb desselben ausgesetzt sein. Die Gate-Elektroden 130 können dann beispielsweise auf die Bahnwiderstände der Halbleiter 132 bzw. organischen Halbleiter 134 durch ihre elektrischen Feldkräfte einwirken. Ein Beispiel einer kurzschlussfreien Aufbringung von Gate-Potenzialen ist in den Figuren 2A und 2B dargestellt und wird unten noch näher erläutert.

Sind die Feldeffekttransistoren 114, 116 in den Figuren 1A und 1B im Wesentlichen gleichartig ausgestaltet, so ergibt sich das Ausgangssignal an der Subtrahierstufe 128 zumindest näherungsweise, abgesehen von einem möglichen Verstärkungsfaktor, wie folgt:

$$S_A \quad = \quad S_1 - S_2$$
$$= \quad S_{1Analyt} + S_{1offset} - S_{2offset}.$$

Mit $S_{1offset} \approx S_{2offset}$ folgt daraus:

$$S_A \approx S_{1Analyt}.$$

Dabei sind die Offsets $S_{1offset}$ und $S_{2offset}$ in der Regel ortsabhängig, temperaturabhängig, oder feuchtigkeitsabhängig oder abhängig von anderen Parametern. Beschaltungen zu einer Kompensation, insbesondere der genannten Abhängigkeiten, sollten im Allgemeinen dem Fachmann bekannt sein.

Die Ladungsträgermobilität und/oder Leitfähigkeit von organischen Halbleitermaterialien, beispielsweise leitfähigen Polymeren, basiert in der Regel auf dem Umstand, dass geeignete Polymere, beispielsweise Polythiophen, in langen Molekülketten mit ausgedehnten $\pi$-Elektronen-Systemen vorliegen. Über die beweglichen $\pi$-Elektronen-Systeme können sich die $\pi$-Elektronen mit relativ geringem Energieaufwand entlang der Molekülketten bewegen und somit einen Stromfluss ermöglichen.

In den Figuren 2A und 2B ist ein Ausführungsbeispiel einer Sensorvorrichtung 110 gezeigt, welches grundsätzlich auch mit der Operationsverstärkerschaltung 118 gemäß den Figuren 1A und 1B und/oder einer Integratorschaltung kombiniert werden kann, welches jedoch grundsätzlich auch in Alleinstellung verwendet werden kann. Beispielsweise könnte der rechte der beiden Feldeffekttransistoren 114, 116 in den Figuren 1A und 1B, also der Feldeffekttransistor 114 mit der Sensorelektrode 162, gemäß den Ausführungsbeispielen in den Figuren 2A und 2B ausgestaltet sein. Auch eine andere Ausgestaltung ist jedoch grundsätzlich möglich. So könnten die in den Figuren 2A und 2B dargestellten Schaltungen auch in anderer Weise als mit dem Operationsverstärker 118 gemäß den Figuren 1A und 1B realisiert werden. Verschiedene Kombinationsmöglichkeiten sind denkbar.

Die Figuren 2A und 2B zeigen das Ausführungsbeispiel der Sensorvorrichtung 110 der Erfindung in verschiedenen Betriebszuständen. Wiederum umfasst die Sensorvorrichtung 110 einen elektrischen Sensor 112, welcher in diesem Fall wiederum einen Feldeffekttransistor 114, insbesondere einen organischen Feldeffekttransistor 116, umfasst. Der Feldeffekttransistor 114 umfasst wiederum einen Halbleiter 132, insbesondere in Form eines organischen Halbleiters 134 oder organischen Leiters, beispielsweise eines leitfähigen Polymers, welches eine Source-Elektrode 136 und eine Drain-Elektrode 138 miteinander verbindet. Diese Elektroden 136, 138 sind beispielsweise mit einer Spannungsquelle 166 und/oder Stromquelle 158 verbunden, und es fließt ein Messstrom $I_M$ durch diese Elektroden 136, 138. Dieser Messstrom $I_M$ kann beispielsweise, insbesondere über einen Messwiderstand 180, erfasst werden, beispielsweise wiederum mit einer Verstärkerschaltung und/oder Operationsverstärkerschaltung 118, beispielsweise analog zu den Figuren 1A und 1B.

Die Sensorvorrichtung 110 gemäß den Figuren 2A und 2B weist wiederum mindestens eine Detektorkammer 140 auf, beispielsweise wiederum mit einem Gehäuse 142 und/oder einem Medium, welches eingerichtet ist, um ein fluides Medium 152 aufzunehmen, beispielsweise ein poröses Material, insbesondere ein poröses Kunststoffmaterial, welches vorzugsweise durch das Gehäuse 142 umschlossen sein kann, beispielsweise ein ganz oder teilweise elektrisch isolierendes Gehäuse 142. Die Detektorkammer 140 kann beispielsweise wiederum, wie in den Figuren 2A und 2B exemplarisch gezeigt, als Hohlraum und/oder Vertiefung in dem Gehäuse 142 ausgebildet sein. Wie Detektorkammer 140 kann beispielsweise erforderlich sein, um beispielsweise das poröse Material elektrisch, insbesondere gegenüber elektronischen Komponenten, zu isolieren. In den Figuren 2A und 2B ist exemplarisch wiederum eine optionale Membran 148 dargestellt, beispielsweise eine Glucose-spezifische, semipermeable Membran 148, welche einen Durchtritt von Glucose ermöglicht, welche jedoch ein Austreten von Materialien einer Detektorsubstanz 150 aus der Detektorkammer 140 verhindert. Über die Glucose-spezifische Membran 148 kann sich beispielsweise ein Gleichgewicht 188 an Analyt 144 zwischen dem umgebenden Fluid 146 und einem Inneren der Detektorkammer 140 ausbilden. Die Membran 148 kann beispielsweise Bestandteil des Gehäuses 142 sein und kann die in dem Gehäuse 142 ausgebildete Detektorkammer 140 gegenüber einem das Gehäuse 142 ganz oder teilweise umgebenden Außenraum, in welchem das Fluid 146 aufgenommen ist, trennen, so dass ein Analytaustausch durch die Membran 148 hindurch zwischen dem Fluid 146 und dem fluiden Medium 152 möglich ist.

Der Feldeffekttransistor 114 weist wiederum eine Sensorelektrode 162 in Form einer der Detektorkammer 140 zuweisenden Gate-Elektrode 130 auf. Beispielsweise kann es sich hierbei einfach um die Oberfläche des Halbleiters 132 bzw. organischen Halbleiters 134 handeln, welcher seinerseits auf einem Substrat 168 aufgebracht sein kann. Alternativ oder zusätzlich kann auf dieser Oberfläche auch mindestens eine zusätzliche Schicht aufgebracht sein, welche dann als Gate-Elektrode 130 wirken kann. Zwischen der Gate-Elektrode 130 und der Detektorkammer 140 kann weiterhin, wie in den Figuren 2A und 2B angedeutet, mindestens ein Abstandhalter 170 vorgesehen sein, beispielsweise mindestens eine isolierende Abstandhalterschicht 170.

Der elektrische Sensor 112 und/oder der Kompensationssensor 113 umfassen exemplarisch in den in den Figuren 1A, 1B, 2A und 2B gezeigten optionalen Aufbauten optional jeweils mindestens eine Gegenelektrode 172. Diese Gegenelektrode 172 ist vorzugsweise, jedoch nicht notwendigerweise, auf einer der Gate-Elektrode 130 gegenüberliegenden Seite der Detektorkammer 140 angeordnet und/oder auf andere Weise derart, dass zwischen der Gegenelektrode 172 und der Gate-Elektrode 130 zumindest ein Teil der Detektorkammer 140 angeordnet ist. Die Gegenelektrode 172 kann physikalisch auch als Einheit mit der Gate-Elektrode 130 oder als Bestandteil derselben betrachtet werden, da diese, gemeinsam mit der Gegenelektrode 172, und gegebenenfalls gemeinsam mit den Vorgängen innerhalb der Detektorkammer 140, das Potenzial der Gate-Elektrode 130 und damit das elektrische Feld innerhalb des Halbleiters 132 beeinflusst.

Zwischen der Gegenelektrode 172 und der Gate-Elektrode 130 ist in dem dargestellten Ausführungsbeispiel exemplarisch eine weitere Spannungsquelle 174 angeordnet, welche, wie auch die Spannungsquelle 166, zu einer Ansteuerung 154 der Sensorvorrichtung 110 zuzurechnen ist und welche beispielsweise die Gegenelektrode 172 mit einem negativen Potenzial (-) beaufschlagt und beispielsweise die Drain-Elektrode 138 und, über diese und den Halbleiter 132, 134 bzw. das leitfähige Polymer, die Gate-Elektrode 130 mit einem positiven Elektrodenpotenzial. Die Spannungsquelle 174 kann beispielsweise mit einem Umschalter 175 ausgestaltet sein, welcher die Spannungsquelle 174 abschaltet oder auch umpolen kann. Auch zwischen der Gegenelektrode 172 und dem fluiden Medium 152 und/oder der Detektorkammer 140 kann optional mindestens ein Abstandhalter 170, beispielsweise mindestens eine Isolatorschicht, vorgesehen sein.

Der Halbleiter 132, insbesondere der organische Halbleiter 134, kann beispielsweise derart ausgestaltet werden, dass dieser auf dem Substrat 168 aufgebracht ist. Dieses Substrat 168 kann als passives Substrat 168 ausgestaltet sein, kann jedoch in diesem oder in anderen Ausführungsbeispielen grundsätzlich auch eine oder mehrere elektronische Bauelemente und/oder Leiterbahnen und/oder andere elektrische oder elektronische Komponenten umfassen. Insbesondere kann es sich hierbei um ein organisches elektronisches Substrat 168 handeln. Dieses organische elektronische Substrat 168 kann auch beispielsweise die Anschlüsse für die Elektroden 136, 138 umfassen.

Die Detektorkammer 140 ist vorzugsweise elektrisch isoliert gegenüber dem Halbleiter 132 und/oder organischen Halbleiter 134, insbesondere gegenüber dem leitfähigen Polymer, abgegrenzt. An der Gate-Elektrode 130, welche wiederum als Sensorelektrode 162 fungieren kann, können, beispielsweise als Bestandteil der Detektorsubstanz 150, elektrisch geladene Teilchen 176 vorgesehen sein. Unter elektrisch geladenen Teilchen 176 sind dabei Teilchen zu verstehen, welche zumindest unter den in der Detektorkammer 140, beispielsweise in dem fluiden Medium 152, herrschenden Umgebungsbedingungen stets eine Netto-Ladung aufweisen. Beispielsweise kann es sich hierbei um geladene Moleküle handeln. Diese geladenen Moleküle sind permanente Ladungsträger und werden im Folgenden auch als GM bezeichnet. Beispielsweise können diese stets eine negative Ladung tragen, wie in den Figuren 2A und 2B angedeutet.

Die elektrisch geladenen Teilchen 176 können insbesondere innerhalb der Detektorkammer 140 in ihrer Beweglichkeit eingeschränkt sein. Beispielsweise kann zu diesem Zweck, insbesondere wiederum als Bestandteil der Detektorsubstanz 150, in der Detektorkammer 140 mindestens ein Spacer 178 vorgesehen sein, welcher in den Figuren 2A und 2B ebenfalls angedeutet ist. Dieser 178 kann insbesondere als beweglicher Abstandshalter ausgestaltet sein. Alternativ oder zusätzlich zur Verwendung eines oder mehrerer Spacer 178 können geeignete andere Maßnahmen vorgesehen sein, mittels derer die elektrisch geladenen Teilchen 176 beweglich innerhalb der Detektorkammer 140 aufgenommen sind. Beispielsweise sind in den Figuren 2A und 2B zur Verdeutlichung einer optional erforderlichen Lokalisierung der elektrisch geladenen Teilchen 176 Spacer 178 vorgesehen, über welche die elektrisch geladenen Teilchen 176 im Bereich ihrer Bindungsstellen beweglich sind, in der gesamten Detektorkammer 140 jedoch immobilisiert sind. Auf diese Weise ist in einem gewissen Rahmen eine Umverteilung der elektrisch geladenen Teilchen 176 und damit eine Umverteilung der räumlichen Ladung innerhalb der Detektorkammer 140 möglich.

Die Gegenelektrode 172, welche, wie oben beschrieben, elektrisch oder physikalisch auch als Bestandteil der Gate-Elektrode 130 angesehen werden kann, ist, wie oben ausgeführt, ebenfalls vorzugsweise gegenüber der Detektorkammer 140 isoliert ausgebildet. In dem Ausführungsbeispiel gemäß Figur 1B ist alternativ die Gegenelektrode 172 elektrisch oder physikalisch getrennt von der Gate-Elektrode 130 angeordnet. Bevorzugt kann sich jedoch ein elektrisches Feld von der Gegenelektrode 172 zu der Gate-Elektrode 130 und/oder der Detektorkammer 140 ausbreiten, insbesondere um beispielsweise auf die elektrisch geladenen Teilchen 176 zu wirken. Die Gegenelektrode 172 kann insbesondere eingerichtet sein, um derart auf die elektrisch geladenen Teilchen 176 einzuwirken, dass diese beispielsweise einem mehrmals hintereinander durchgeführten Nachweis des Analyten 144 dienen können, beispielsweise indem die elektrisch geladenen Teilchen 176 nach einem durchgeführten Nachweis wieder in eine Ausgangsposition, gebracht werden können. An diese Gegenelektrode 172 kann, wie in den Figuren 2A und 2B dargestellt, die umschaltbare Spannungsquelle 174 mit ihrem Umschalter 175 geschaltet sein. Diese Spannungsquelle 174 kann beispielsweise zwischen die Gegenelektrode 172 und die Gate-Elektrode 130 geschaltet sein und/oder zwischen die Gegenelektrode 172 und eine der beiden Elektroden 136, 138. Auch andere Ausgestaltungen sind jedoch grundsätzlich möglich.

Wie oben bereits ausgeführt, kann die Gegenelektrode 172 insbesondere elektrisch kontaktiert sein und kann vorzugsweise mit einem definierten Potenzial beaufschlagt werden. Bei dem Ausführungsbeispiel in den Figuren 2A und 2B ist beispielsweise die Spannungsquelle 174 zwischen die Gegenelektrode 172 und die Drain-Elektrode 138 geschaltet, und in den Figuren 1A und 1B ist die Spannungsquelle 174 exemplarisch zwischen die Gegenelektrode 172 und die Masse geschaltet. Die Spannungsquelle 174 kann beispielsweise die Gegenelektrode 172 mit einer Gleichspannung oder einer Wechselspannung oder einer Spannungssequenz, beispielsweise mit einer pulsförmigen Spannungssequenz, beaufschlagen. Liegt beispielsweise der Minuspol der Spannungsquelle 174 an der Gegenelektrode 172 an, so wirkt auf die exemplarisch negativ geladenen GM 176 ein elektrisches Feld E, welches diese in Richtung auf die Gate-Elektrode 130 treibt. In dem zu Figur 1A gehörenden Ausführungsbeispiel ist beispielsweise für beide Gegenelektroden 172 genau eine Spannungsquelle 174 vorgesehen. Dadurch können insbesondere beide Gegenelektroden 172 mit der gleichen oder zumindest einer zueinander proportionalen Spannungssequenz beaufschlagt werden, wohingegen das zu Figur 1B gehörende Ausführungsbeispiel insbesondere zwei separate Spannungsquellen 174 für die Gegenelektroden 172 aufweist, wodurch die Gegenelektrode 172 des elektrischen Sensors 112 und des Kompensationssensors 113 mit unterschiedlichen Spannungssequenzen beaufschlagt werden können.

Weiterhin sind in der Detektorkammer 140, beispielsweise der Detektorsubstanz 150, wiederum ein oder mehrere Rezeptoren 160 vorgesehen, welche beispielsweise wiederum mit den anhand der Figuren 1A und 1B beschriebenen Eigenschaften ausgestaltet sein können und an welche der Analyt 144, beispielsweise Glucose, binden kann. Die Rezeptoren 160 sind beispielsweise derart ausgestaltet, dass diese zwischen den elektrisch geladenen Teilchen 176 in ihrer am weitesten von der Gate-Elektrode 130 entfernten Position und der Gate-Elektrode 130 angeordnet sind. Beispielsweise kann wiederum eine affine Bindung mit dem Analyten 144 erfolgen. Halten sich beispielsweise an den affinen Bindungsstellen der Rezeptoren 160 viele Analytmoleküle, beispielsweise Glucosemoleküle auf, da die Analytkonzentration, beispielsweise die Glucosekonzentration, hoch ist, so können die elektrisch geladenen Teilchen 176 nicht derart nahe an die Gate-Elektrode 130 herantreten, also beispielsweise an den Halbleiter 132 und/oder den organischen Halbleiter 134, insbesondere das leitfähige Polymer. Somit ist in diesem Fall, welcher in Figur 2A dargestellt ist, das auf die Gate-Elektrode 130 und somit auf den Halbleiter 132 bzw. den organischen Halbleiter 134 wirkende elektrische Feld geringer. Damit ist beispielsweise eine höhere Leitfähigkeit der $\pi$-Elektronen in dem leitfähigen Polymer gegeben. Sind hingegen, wie in Figur 2B dargestellt, wenige Analyte 144, beispielsweise wenige Glucosemoleküle, vorhanden, so sind viele der Bindungsstellen der Rezeptoren 160 unbesetzt und die elektrisch geladenen Teilchen 176 können im Mittel näher zur Gate-Elektrode 130 gelangen. Dementsprechend wirkt ein höheres negatives elektrisches Feld auf die Ladungsträger im Halbleiter 132 bzw. organischen Halbleiter 134, insbesondere auf das leitfähige Polymer. Beispielsweise kann hierdurch, je nach Halbleitertyp, der Stromfluss durch den Halbleiter 132 bzw. organischen Halbleiter 134 abnehmen. Prinzipiell kann bei Feldeffekttransistoren 114 alternativ oder zusätzlich auch ein Anreicherungsprinzip angewandt werden.

Wie in Figur 2B weiterhin angedeutet, können die Rezeptoren 160 sich neutral gegenüber den elektrisch geladenen Teilchen 176 verhalten, also nicht mit diesen binden. Alternativ oder zusätzlich kann der Rezeptor 160 jedoch auch an die elektrisch geladenen Teilchen 176 binden, beispielsweise reversibel, sodass wiederum eine Affinität gegeben sein kann.

Damit sich allgemein das Analyt-affine System besser auf eine gegebenenfalls zwischenzeitlich geänderte Analytkonzentration anpassen kann und/oder damit Veränderungen messtechnisch rascher und präziser festgestellt werden können, können die Positionen der elektrisch geladenen Teilchen 176 insbesondere durch ein alternierendes Anlegen des Potenzials im System, beispielsweise an der Gate-Elektrode 130 und/oder der Gegenelektrode 172, moduliert werden, beispielsweise durch ein wiederholtes Umschalten, beispielsweise periodisches Umschalten, der Spannungsquelle 174 mittels des Umschalters 175.

Das wirksame elektrische Feld E ist in der Regel eine vektorielle Größe und abhängig vom Abstand s zwischen dem resultierenden Potenzial und der Ebene der Gate-Elektrode 130 und/oder des Halbleiters 132 bzw. organischen Halbleiters 134, beispielsweise des leitfähigen Polymers:

$$E = \frac{U_1}{s_1} + \frac{U_{Spacer}}{s_{Spacer}}.$$

Dabei bedeuten:

E = elektrisch wirksames Gesamtfeld
$s_1$ = Abstand zwischen Gate-Elektrode 130 und Gegenelektrode 172 und/oder zwischen Gegenelektrode 172 und Halbleiter 132 bzw. organischem Halbleiter 134,
$U_1$ = Spannung zwischen Gate-Elektrode 130 und Gegenelektrode 172 und/oder zwischen Elektrode 136, 138 und Gegenelektrode 172,
$U_{Spacer}$ = gesamte Spannung aller elektrisch geladenen Teilchen 176,
$s_{Spacer}$ = Abstand der elektrisch geladenen Teilchen 176 von der Gate-Elektrode 130 und/oder dem Halbleiter 132 bzw. organischen Halbleiter 134, und

$$U_{Spacer} = \frac{n \cdot Q \cdot s_{Spacer}}{\varepsilon \cdot A},$$

mit:

n = Anzahl der elektrisch geladenen Teilchen 176,
Q = Elementarladung $\approx 1{,}6 \cdot 10^{-19}$ As,
$\varepsilon$ = die Elektrizitätskonstante des Vakuums $\approx 8{,}854 \cdot 10^{-12}$ As/Vm,
A = wirksame Fläche der elektrisch geladenen Teilchen 176,

$$E = \frac{U_1}{s_1} + \frac{n \cdot Q}{\varepsilon \cdot A},$$

$$n = F \cdot \int c_A \cdot dt_{verweil},$$

mit

F = Proportionalitätsfaktor,
$c_A$ = Analytkonzentration,
$t_{verweil}$ = mittlere Zeit, die ein Teilchen des Analyten 144, beispielsweise ein Analytmolekül, in dem System, beispielsweise der Detektorkammer 140 und/oder zwischen den Elektroden 130, 172, verweilt.

Die Verweildauer ist in der Regel insbesondere abhängig vom Grad der Affinität der Analytmoleküle zu den verwendeten Rezeptoren 160.

Die resultierende Feldstärke ist damit insgesamt:

$$E = \frac{U_1}{s_1} + \frac{F \cdot Q \cdot \int c_A \cdot dt_{verweil}}{\varepsilon \cdot A}.$$

Die Ladungsträgerbeweglichkeit in dem Halbleiter 132, insbesondere dem organischen Halbleiter 134, ist in vielen Fällen zumindest näherungsweise abhängig von der Feldstärke:

$$L = k \cdot E,$$

wobei L die Dimension eines Leitwerts aufweist und wobei k ein spezifischer Faktor des Halbleiters 132, insbesondere des organischen Halbleiters 134 und besonders bevorzugt des leitfähigen Polymers ist. Damit ergibt sich für den Messstrom $I_M$:

$$I_M = L \cdot U_2.$$

Dieser Strom $I_M$ wirkt beispielsweise über den Messwiderstand 180 in den Figuren 2A und/oder 2B und/oder über einen oder beide der Festwiderstände 124, 126 in den Figuren 1A und 1B, insbesondere über den Festwiderstand 124. Auf diese Weise werden die Messsignale $S_1$ und $S_2$ erzeugt. Da es sich grundsätzlich bei den behandelten Größen teilweise um vektorielle Größen handelt, können insbesondere deren Richtungen, beispielsweise Feldrichtungen, das heißt deren Vorzeichen, prinzipiell auch ausgetauscht werden, das heißt die in den Figuren 2A und 2B erläuterte Anordnung, welche auch auf die Figuren 1A und Figur 1B übertragbar ist, ist grundsätzlich auch für elektrisch geladene Teilchen 176 in Form positiver Ladungen wirksam.

Wie oben beschrieben, kann die in den Figuren 2A und 2B dargestellte Struktur insbesondere gemäß der Figuren 1A und 1B angeordnet werden, also in einen Operationsverstärker 118 integriert werden. Geht man davon aus, dass die Feldeffekttransistoren 114, insbesondere die organischen Feldeffekttransistoren 116, und/oder die geometrischen Strukturen identisch sind, so subtrahieren sich die additiven Terme, sodass beispielsweise für das Analytsignal $S_A$ gilt:

$$S_A = \frac{U_2 \cdot k \cdot F \cdot Q \cdot t_{verweil}}{\varepsilon \cdot A} \cdot (1 - c_A),$$

wobei $c_A$ die im Zeitintervall mittlere Konzentration des Analyten 144 ist.

Da vorzugsweise, jedoch nicht notwendigerweise, in der Detektorsubstanz 150 kein Reagens vorgesehen ist, welches bei dem Nachweis des Analyten 144 verbraucht wird, kann im Prinzip das System der Sensorvorrichtung 110 seriell in einer in vitro-Referenz abgeglichen werden, beispielsweise um ein Signal $S_{Ref}$ zu einer Analytkonzentration $c_{ref}$ zu erzeugen, bevor die Sensorvorrichtung 110 vollständig oder teilweise beispielsweise in ein Körpergewebe implantiert wird. Ein Abgleichfaktor A kann sich dann beispielsweise folgendermaßen ergeben:

$$A = \frac{S_A}{S_{Ref}} = \frac{1 - c_A}{1 - c_{ref}}$$

Der Faktor A kann der Sensorvorrichtung 110, beispielsweise der Ansteuerung 154, übergeben werden. Beispielsweise kann der Faktor A in der Sensorvorrichtung 110 und/oder in einer davon räumlich getrennten Datenverarbeitungsvorrichtung gespeichert und/oder verarbeitet werden und/oder einem Steuerprogramm und/oder einem Auswertungsprogramm übergeben werden. In den Figuren 3A und 3B ist ein weiteres Ausführungsbeispiel einer erfindungsgemäßen Sensorvorrichtung 110 dargestellt, welches beispielsweise nach einem Wechselfeldprinzip eingesetzt werden kann. In diesem Ausführungsbeispiel umfasst die Sensorvorrichtung 110 beispielsweise einen elektrischen Sensor 112 in Form eines oder mehrerer Kapazitäten, das heißt mindestens eines Kondensators 182. Dieser kann grundsätzlich ähnlich aufgebaut sein zu der Zelle gemäß den Figuren 2A und 2B und kann eine erste Elektrode 184 umfassend, welche beispielsweise als Sensorelektrode 162 ausgebildet sein kann und eine zweite Elektrode 186, welche beispielsweise als Gegenelektrode 172 wirken kann. Zwischen diese Elektroden 184, 186 ist wiederum eine Detektorkammer 140 und/oder ein Teil derselben eingebracht, bezüglich deren Ausgestaltung wiederum exemplarisch auf die obige Beschreibung der Figuren 2A und 2B verwiesen werden kann. Auch andere Ausgestaltungen sind jedoch grundsätzlich möglich. Die Elektroden 184, 186 können beispielsweise wiederum direkt an der Detektorkammer 140 und/oder dem Medium in der Detektorkammer 140 ausgestaltet sein, können jedoch auch vorzugsweise wiederum durch einen oder mehrere Abstandhalter 170, beispielsweise Isolatorschichten, gegenüber dieser und gegeneinander isoliert ausgestaltet sein.

Innerhalb der Detektorkammer 140 können beispielsweise wiederum eine oder mehrere Detektorsubstanzen 150 aufgenommen sein. Wiederum kann die Detektorsubstanz 150 insbesondere derart ausgestaltet sein, dass eine Ladungsverteilung innerhalb der Detektorkammer 140, insbesondere zwischen den Elektroden 184, 186, durch die Anwesenheit eines oder mehrerer Analyte 144, insbesondere spezifisch, beeinflussbar ist. Der mindestens eine Analyt 144, beispielsweise Glucose, kann wiederum beispielsweise in einem Diffusionsgleichgewicht mit einer äußeren Umgebung der Sensorvorrichtung 110, insbesondere des elektrischen Sensors 112, stehen, beispielsweise einem umgebenden Körpergewebe, beispielsweise mit einem Fluid 146, insbesondere interstitiellem Fluid 146. Dieses Gleichgewicht ist in Figur 3A, wie auch in Figur 2A, durch die Bezugsziffer 188 angedeutet. Auf diese Weise kann sich beispielsweise, insbesondere durch die Membran 148, innerhalb der Detektorkammer 140 ein Gleichgewicht 188 der Analytkonzentration zu einem Raum außerhalb des elektrischen Sensors 112, insbesondere in einem umgebenden Körpergewebe, einstellen.

Insbesondere kann die mindestens eine Sensorelektrode 162, wobei auch beide Elektroden 184, 186 grundsätzlich als Sensorelektroden 162 ausgestaltet sein können, derart ausgestaltet sein, dass diese den Analyten 144 bindet. Zu diesem Zweck können beispielsweise wiederum auf der mindestens einen Sensorelektrode 162 ein oder mehrere Rezeptoren 160 vorgesehen sein, welche in Figur 3A nicht dargestellt sind, welche jedoch optional ebenfalls vorhanden sein können. Die elektrisch geladenen Teilchen 176 können beispielsweise wiederum durch Spacer 178 an die Sensorelektrode 162 und/oder in anderer Weise an die Detektorkammer 140 gebunden sein oder können, alternativ oder zusätzlich, auch vollständig oder teilweise frei beweglich sein, analog zur Ausgestaltung gemäß den Figuren 2A und 2B. Wiederum kann auf diese oder auch auf andere Weise eine Ladungsverteilung innerhalb der Detektorkammer 140 durch Anwesenheit oder Abwesenheit des Analyten 144 beeinflusst werden, was wiederum über den elektrischen Sensor 112 erfassbar ist, beispielsweise durch mindestens eine elektrische Eigenschaft des Kondensators 182.

Die Figuren 3A und 3B zeigen somit ein Ausführungsbeispiel, nach welchem beispielsweise chemische und/oder physikalische Prozesse direkt in elektrisch auswertbare Signale verwandelt werden. Dabei wird der Analyt-spezifische elektrische Sensor 112 exemplarisch direkt als Kondensator 182 ausgebildet. Dieser kann beispielsweise mit der Ansteuerung 154 versehen werden, um die mindestens eine elektrische Eigenschaft, welche direkt oder indirekt durch die Anwesenheit des Analyten 144 beeinflusst wird, zu messen. Beispielsweise kann der Kondensator 182 eingesetzt werden, um einen Frequenzgenerator zu verstimmen. Alternativ oder zusätzlich kann die Ansteuerung 154 jedoch auch mindestens einen Wechselspannungsgenerator 190 und/oder mindestens einen Wechselstromgenerator umfassen, welcher mit den Elektroden 184, 186 verbunden ist. Beispielsweise kann zur Erfassung der elektrischen Beeinflussung eine Arbeit gemessen werden, die in einem derartigen Wechselspannungsgenerator 190 und/oder Frequenzgenerator aufgewandt werden muss, wenn dieser an den Kondensator 182 angeschlossen ist. Die Arbeit kann insbesondere für die Änderung der Ladungsverteilung, insbesondere für eine Ladungsbewegung, beispielsweise verbunden mit einer Bewegung mindestens einer der Spacer 178, in dem elektrischen Feld aufgewendet werden. Um diese Arbeit aufwenden zu können kann dem System bevorzugt von außen, insbesondere von außerhalb des elektrischen Sensors 112, beispielsweise über die Spannung $U_1$, Energie zugeführt werden.

Beispielsweise können in der Anordnung gemäß den Figuren 3A und 3B wiederum als elektrisch geladene Teilchen 176 große Moleküle gleichmäßig und/oder ortsgebunden und/oder auch frei in der Detektorkammer 140 eingebracht sein. In die Detektorkammer 140 kann wiederum Fluid 146 und/oder Analyt 144 eintreten. Die beiden Elektroden 184, 186 können an der Detektorkammer 140 angebracht sein. Die Elektroden 184, 186 können mit dem Wechselspannungsgenerator 190 verbunden sein. Gelangt Analyt 144, ähnlich wie in den Abbildungen 2A und 2B, in die Detektorkammer 140, so können die elektrisch geladenen Teilchen 176 in ihrer Beweglichkeit je nach Konzentration des Analyten 144 behindert werden und die dielektrischen Kräfte können zunehmen, umfassend beispielsweise insbesondere eine Erhöhung des Dielektrizitätsfaktors mindestens eines Anteils der Detektorkammer 140. Die Vorgänge in der Detektorkammer 140 sind damit vergleichbar zur obigen Beschreibung in den Figuren 2A und 2B. Dies bedeutet wiederum, dass die Arbeit, die beispielsweise von dem Wechselspannungsgenerator 190 und/oder in anderen Komponenten des Systems aufgebracht werden muss, um beispielsweise eine Frequenz zu halten, ebenfalls zunimmt. Ohne Analyt 144 ist die Beweglichkeit beispielsweise höher, und die dielektrischen Kräfte nehmen ab, umfassend beispielsweise insbesondere eine Abnahme des Dielektrizitätsfaktors mindestens eines Anteils der Detektorkammer 140. Es muss dann in der Regel weniger Arbeit in dem System aufgebracht werden. Die beispielsweise von der Ansteuerung 154 aufgebrachte Arbeit ist somit ein Maß für die Konzentration des Analyten 144 in der Detektorkammer 140, beispielsweise indem diese proportional ist zur Konzentration des Analyten 144. Weiterhin spielt wiederum die mittlere Verweildauer eine Rolle. Somit kann die von der Ansteuerung 154 aufgewandte Arbeit somit abhängig sein von der Analytkonzentration in der Umgebung des elektrischen Sensors 112, beispielsweise in dem Körpergewebe, insbesondere einer Glucosekonzentration im Interstitium. In Figur 3A ist wiederum, analog zu Figur 2A, ein Fall dargestellt, in welchem eine hohe Konzentration an Analyt 144 vorliegt, sodass eine hohe Arbeit aufgewandt werden muss, wohingegen in Figur 3B wiederum, analog zu Figur 2B, eine niedrige Analytkonzentration vorliegt, sodass eine vergleichsweise geringe dielektrische Arbeit aufgewandt werden muss.

Die Dielektrizitätskonstante des Kondensators 182 ist in der Regel abhängig von der Beweglichkeit der elektrisch geladenen Teilchen 176, welche als Teilchen mit einer Nettoladung oder auch als elektrische Dipole oder Multipole ausgestaltet sein können. Beispielsweise kann eine Dielektrizitätskonstante abhängig sein von einer Beweglichkeit elektrischer Dipole. Sind somit die elektrisch geladenen Teilchen 176, gegebenenfalls einschließlich der Spacer 178, weniger beweglich, beispielsweise in Folge der hohen Konzentration der Analytmoleküle des Analyten 144, so steigt die Dielektrizitätskonstante. In der einfachen, in den Figuren 3A und 3B exemplarisch dargestellten Parallelplattenkonstruktion des Kondensators 182 kann beispielsweise die in dem Kondensator 182 gespeicherte Energie W ausgedrückt werden durch:

$$W = \frac{\varepsilon \cdot E^2 \cdot A \cdot s}{2},$$

wobei W die Arbeit oder gespeicherte Energie ist, wobei E die wirksame Feldstärke ist, also der Effektivwert der Wechselfeldstärke, wobei ε ein Dielektrizitätsfaktor ist, wobei A die Fläche der Anordnung ist und wobei S der Abstand der Elektroden 184, 186 des Kondensators 182 ist. Die elektrische Energie W kann gemessen werden, indem die Generatorwechselspannung des Wechselspannungsgenerators 190 erfasst wird. Beispielsweise muss in diese bei höherer Konzentration erhöht werden, um die Frequenz konstant zu halten. Umgekehrt kann sich aber, bei konstanter Generatorspannung, die Frequenz der Oszillation der elektrisch geladenen Teilchen 176 in Abhängigkeit von der Konzentration des Analyten 144 verändern. Damit kann die Konzentration des Analyten 144 direkt in ein digital auswertbares Signal, beispielsweise in Schwingungen pro Zeitintervall, umgewandelt werden, und diese können beispielsweise gezählt werden. Dabei ergibt sich:

$$\varepsilon = k \cdot c_A,$$

mit:

$$C = \frac{\varepsilon \cdot A \cdot}{s} = \frac{k \cdot c_A \cdot A}{s},$$

C = Kapazität des Kondensators 182 in Farad, und
k = ein vom Gesamtsystem abhängiger Faktor.

Ein direkter Zusammenhang zwischen der Analytkonzentration und der elektrischen Messgröße kann beispielsweise dann erreicht werden, wenn der elektrische Sensor 112 und/oder der Kondensator 182 als frequenzbeeinflussende Kapazität in einen Oszillator geschaltet wird, beispielsweise in einen elektrischen Schwingkreis. Die Schwingkreisfrequenz f kann sich dann beispielsweise ergeben zu:

$$f = \frac{1}{2\pi\sqrt{LC}} = \frac{1}{2\pi\sqrt{\frac{kc_A A}{s}}},$$

wobei L eine Induktivität des Schwingkreises ist und $c_A$ wiederum die Analytkonzentration.

Alternativ oder zusätzlich zu einem Schwingkreis können jedoch auch andere Arten von Schwingungsschaltungen eingesetzt werden, beispielsweise Schwingungsschaltungen unter Einsatz von Feldeffekttransistoren 114, wie beispielsweise Kollpitz-Oszillatoren oder ähnliche Oszillatoren.

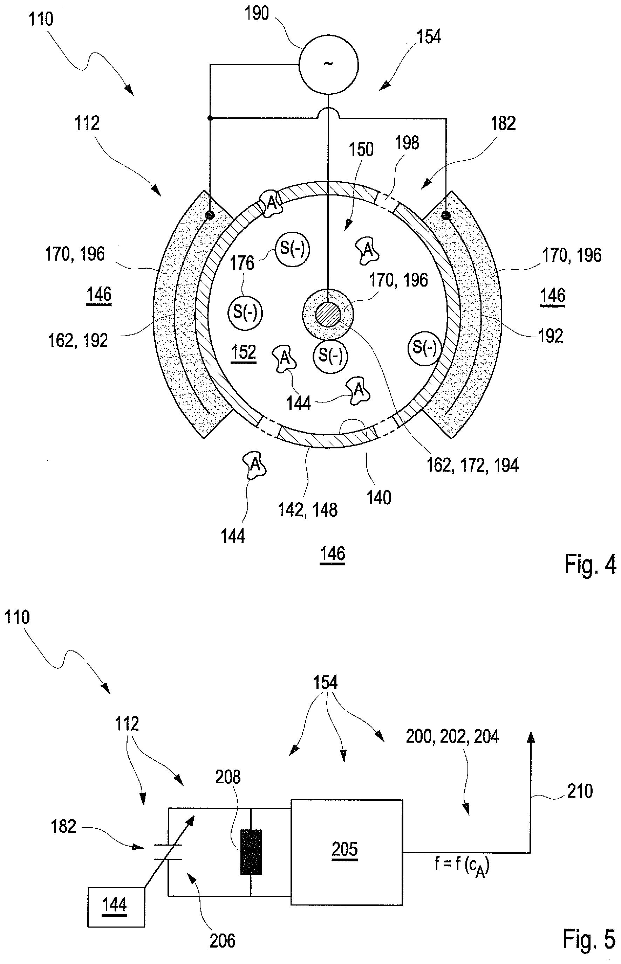

In Figur 4 ist ein weiteres Ausführungsbeispiel einer Sensorvorrichtung 110 mit einem elektrischen Sensor 112 dargestellt, welcher wiederum einen oder mehrere Kondensatoren 182 umfasst. Dargestellt ist, im Gegensatz zu den Figuren 2A-3B, welche Längsschnitte parallel zu einer Insertionsrichtung des elektrischen Sensors 112 zeigen, ein Querschnitt durch den elektrischen Sensor 112. In dieser Ausgestaltung umfasst der Kondensator 182 des elektrischen Sensors 112 wiederum Elektroden 192, 194, in einem konzentrischen, beispielsweise koaxialen, Aufbau. Eine dieser Elektroden 192, welche in diesem Ausführungsbeispiel exemplarisch zweiteilig ausgestaltet ist, welche jedoch auch grundsätzlich einteilig oder mit mehr als zwei Teilen ausgestaltet werden kann, ist dabei auf einer Manteloberfläche eines zylinderförmigen Gehäuses 142 einer Detektorkammer 140 angeordnet. Beispielsweise kann diese zweiteilige Elektrode 192, wiederum durch einen oder mehrere Abstandshalter 170 von der Detektorkammer 140 getrennt sein, beispielsweise durch einen oder mehrere Isolatoren 196, in welche diese Elektrode 192 eingebettet sein kann. Beispielsweise kann es sich hierbei um organische und/oder anorganische Isolatoren handeln.

Die zweite Elektrode 194 kann wiederum als Gegenelektrode 172 ausgebildet sein und ist in diesem Ausführungsbeispiel exemplarisch als zentrale Elektrode, beispielsweile als zentraler Draht und/oder zentraler Stift, ausgestaltet. Auch diese zentrale Elektrode 194 kann beispielsweise wiederum durch einen oder mehrere Abstandhalter 170, beispielsweise wiederum durch einen oder mehrere Isolatoren 196, gegenüber einem in der Detektorkammer 140 aufgenommenen fluiden Medium 152 isoliert sein.

Eine oder beide der Elektroden 192, 194 können in diesem Ausführungsbeispiel als Sensorelektrode 162 dienen, über welche die mindestens eine elektrische Eigenschaft nachgewiesen werden kann, welche durch Anwesenheit des mindestens einen Analyten 144 beeinflussbar ist.

Das Gehäuse 142 ist in dem dargestellten Ausführungsbeispiel semipermeabel für den Analyten 144 ausgestaltet. Dies bedeutet, dass der Analyt 144 beispielsweise wiederum ein Gleichgewicht 188 zwischen dem umgebenden Fluid 146 und dem fluiden Medium 152 innerhalb der Detektorkammer 140 bilden kann. Beispielsweise kann zu diesem Zweck das Gehäuse 142 vollständig oder teilweise als semipermeable Membran 148 ausgestaltet sein, welche durchlässig für den Analyten 144 ist, welche jedoch beispielsweise eine Detektorsubstanz 150 und/oder Bestandteile desselben im Inneren der Detektorkammer 140 zurückhalten kann. Diese mindestens eine Detektorsubstanz 150 kann beispielsweise wiederum, analog zum Beispiel gemäß den Figuren 3A und 3B, mindestens eine Sorte elektrisch geladener Teilchen 176 umfassen, beispielsweise wiederum wie in Figur 4 dargestellt, negativ geladene Teilchen, beispielsweise negativ geladene Moleküle.

Die Sensorvorrichtung 110 gemäß Figur 4 kann wiederum eine Ansteuerung 154 enthalten, beispielsweise eine Ansteuerung 154 analog zu dem Ausführungsbeispiel in den Figuren 3A und 3B. Wiederum kann diese Ansteuerung 154 beispielsweise mindestens einen Wechselspannungsgenerator 190 umfassen, welcher mit einem Anschluss mit der zweiteiligen ersten Elektrode 192 und mit einem anderen Anschluss mit der Gegenelektrode 172 verbunden sein kann. Beispielsweise kann die Sensorvorrichtung 110 gemäß Figur 4 auch Bestandteil mindestens eines Phase-locked loop (PLL) sein. Auch andere Beschaltungen sind jedoch grundsätzlich möglich.

Die Sensorchemie im Inneren der Detektorkammer 140 kann wiederum auf verschiedene Weisen ausgestaltet sein. Beispielsweise können die elektrisch geladenen Teilchen 176 wiederum, abhängig von einer Konzentration des Analyten 144, eine Ladungsverteilung im Inneren der Detektorkammer 140 bewirken. Die elektrisch geladenen Teilchen 176 können beispielsweise wiederum über Spacer 178 an eine oder mehrere Innenoberflächen der Detektorkammer 140 gebunden sein. Alternativ oder zusätzlich können die elektrisch geladenen Teilchen 176 jedoch auch, wie in Figur 4 dargestellt, frei innerhalb der Detektorkammer 140 beweglich sein. Wiederum alternativ oder zusätzlich kann die mindestens eine Detektorsubstanz 150 auch, analog zu den Figuren 3A und 3B und den Figuren 2A und 2B, wiederum optional mindestens einen Rezeptor 160 umfassen, beispielsweise an einer oder beiden Elektroden 192, 194, beispielsweise um, bei Anwesenheit von Analyt 144, den Bewegungsspielraum der geladenen Teilchen 176 einzuschränken. Alternativ oder zusätzlich kann jedoch auch alleine der Verdrängungseffekt durch den Analyten 144 ausreichen, um eine eingeschränkte Beweglichkeit der elektrisch geladenen Teilchen 176 zu bewirken und damit ein verändertes dielektrisches Verhalten in einem Wechselfeld des Kondensators 182.

In vivo-Sensoren, als welche der elektrische Sensor 112 (alternativ oder zusätzlich zu einem Einsatz als in-vitro-Sensor) eingesetzt werden kann, bei denen vorzugsweise keine chemische Umsetzung des Analyten 144 erfolgt, könnten theoretisch sehr lange arbeiten, ohne ausgetauscht zu werden. Physiologische Eigenschaften im Körper bewirken jedoch, dass sich in der Regel eine Sensoroberfläche nach einer gewissen Zeit zusetzt, beispielsweise durch Proteinanlagerungen, Gerinnungsprodukte oder ähnliche Effekte. Dennoch ergeben sich höhere Langzeitstabilitäten im Vergleich zu herkömmlichen subkutanen Sensorsystemen. Somit ist eine Möglichkeit zur subkutanen Tragweite des elektrischen Sensors 112 oder Teilen desselben durch minimalinvasive Eingriffe ermöglicht, und die vorgeschlagenen elektrischen Sensoren 112 gemäß ein oder mehreren der beschriebenen Ausführungsbeispiele ist vorteilhaft gegenüber herkömmlichen Sensorelementen mit Stoffumsatz. Ein derartiges Sensorelement könnte praktisch beispielsweise als katheterförmiges, Membranumschlossenes, vorzugsweise flexibles, Gebilde in ein subkutanes Gewebe eingebracht werden, beispielsweise mit der in Figur 4 beschriebenen Ausgestaltung. Beispielsweise kann die Detektorkammer 140 einen Durchmesser von beispielsweise ungefähr 1 mm, insbesondere von 50 μm bis 2 mm aufweisen. Die Detektorkammer 140 kann beispielsweise als Kapillare ausgestaltet sein, mit einer oder mehreren Öffnungen 198, welche optional in Figur 4 angedeutet sind und welche beispielsweise derart kleindimensioniert werden können, dass diese lediglich von dem Analyten 144, nicht jedoch von der Detektorsubstanz 150, beispielsweise den elektrisch geladenen Teilchen 176, durchdrungen werden können. Beispielsweise können, alternativ oder zusätzlich zu Öffnungen 198, poröse Wandmaterialien für das Gehäuse 142 eingesetzt werden. Wiederum alternativ oder zusätzlich können die Öffnungen 198 auch vollständig oder teilweise von einer Membran 148, beispielsweise gemäß den oben beschriebenen Eigenschaften, umschlossen und/oder ausgefüllt sein, um die beschriebenen semipermeablen Eigenschaften zu gewährleisten. Beispielsweise kann das derart Membran-umschlossene Gebilde in ein subkutanes Gewebe eingebracht werden, derart, dass die erwähnten elektrisch geladenen Teilchen 176 in der Detektorkammer 140 eingeschlossen sind und, gegebenenfalls außer dem Fluid 146, beispielsweise interstitieller Flüssigkeit, möglichst nur der Analyt 144, beispielsweise Glucose, durch die Membranoberfläche und/oder die Öffnungen 198 ausgetauscht werden kann. Die Detektorsubstanz 150, insbesondere die elektrisch geladenen Teilchen 176, können nun in der Detektorkammer 140 eingeschlossen sein und müssen nicht mehr notwendigerweise an eine Oberfläche über Spacer 178 fixiert sein, sondern können sich vorzugsweise in der Detektorkammer 140 frei bewegen. Dies reduziert die Anforderungen an die Struktur und Applikation der elektrisch geladenen Teilchen 176 beträchtlich. Die räumliche Struktur des Kondensators 182 kann beispielsweise durch eine rotationssymmetrische Anordnung gemäß Figur 4 realisiert werden. Auch andere geometrische Anordnungen sind jedoch grundsätzlich möglich. Exemplarisch ist in Figur 4 ein konzentrischer Aufbau mit der rotationssymmetrischen zentralen Elektrode 194 dargestellt, welcher beispielsweise in Form eines Hohlkatheters realisierbar ist. An den Außenwandungen liegen die in den Isolatoren 196 isolierten Teile der ersten Elektrode 192 auf, welche als einfache Elektroden oder, analog zum Aufbau gemäß den Figuren 3A und 3B, oder als Bestandteile eines oder mehrerer Feldeffekttransistoren 114, analog zum Aufbau gemäß den Figuren 2A und 2B, ausgestaltet sein können. Auch andere geometrische Aufbauten sind jedoch möglich, beispielsweise mit mehreren Gegenelektroden 172, mit anders gestalteten äußeren Elektroden 192 oder mit nicht-rotationssymmetrischen Aufbauten. Die äußeren Elektroden 192 sind vorzugsweise derart ausgestaltet, dass sich zwischen diesen freie Flächen des Gehäuses 142 befinden, durch welcher der Analyt 144 aus und in die Detektorkammer 140 gelangen kann, beispielsweise durch eine Permeation durch eine Membran 148.

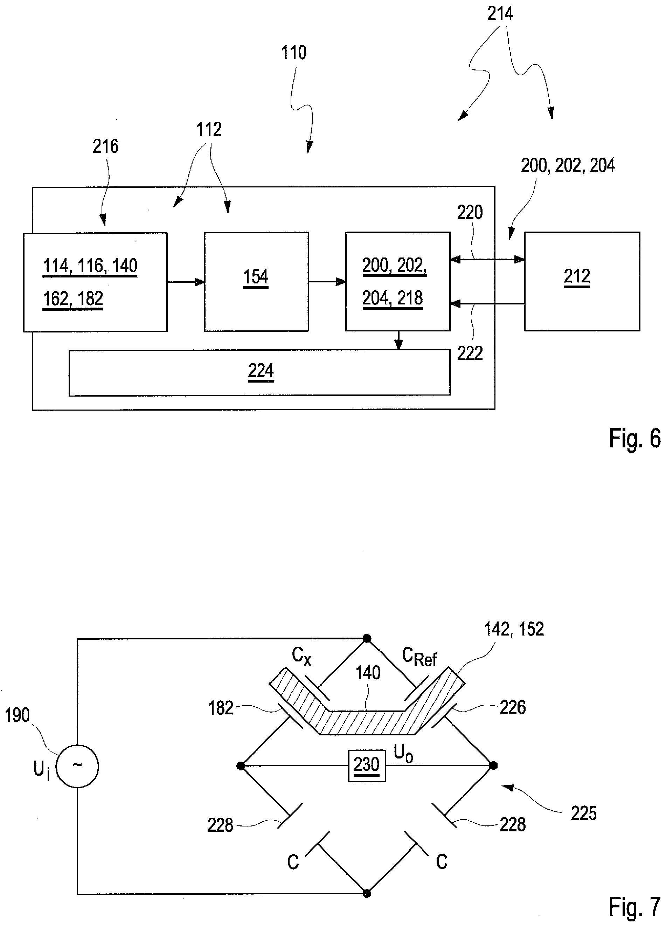

Wie oben beschrieben, eignen sich kapazitive Systeme, mit mindestens einem Kondensator 182, besonders gut für einen Nachweis mindestens einer durch den Analyten 144 beeinflussten elektrischen Eigenschaft. Insbesondere kann, wie oben ausgeführt, mindestens eine Frequenz mindestens eines Schwingers durch eine Kapazität und/oder durch die elektrischen Eigenschaften des Kondensators 182 beeinflusst werden. Diese beeinflusste Frequenz und/oder ein daraus abgeleitetes Signal kann beispielsweise über mindestens eine Schnittstelle, welche in einem Ausführungsbeispiel gemäß Figur 5 mit der Bezugsziffer 200 bezeichnet ist, von der Sensorvorrichtung 110 abgegeben werden, beispielsweise über mindestens eine drahtlose Schnittstelle 202, insbesondere eine Funkschnittstelle 204. In Figur 5 ist ein schematischer Aufbau einer Sensorvorrichtung 110 gezeigt, mittels derer dieses Prinzip realisierbar ist. Hierbei kann beispielsweise eine Ansteuerung 154 der Sensorvorrichtung 110, deren elektrischer Sensor 112 beispielsweise gemäß den Ausführungsbeispielen in den Figuren 3A, 3B oder 4 ausgestaltet sein kann, mindestens einen Oszillator 205 enthalten, der einen Schwingkreis 206 anregt. Dieser Schwingkreis 206 kann neben dem mindestens einen Kondensator 182 beispielsweise weiterhin mindestens eine Spule 208 und/oder eine andere Art von induktivem Element umfassen. Durch den Schwingkreis 206 stellt sich der Oszillator 205 auf die Resonanzfrequenz des Schwingkreises 206 abhängig von dem Analyten 144 ein, was in Figur 5 durch eine Beeinflussung der Kapazität des Kondensators 182 durch den Analyten 144 angedeutet ist. Diese Frequenz f ist somit eine Funktion der Analytkonzentration $c_A$. Die Frequenz kann direkt oder indirekt, beispielsweise nach Umwandlung durch Frequenzmodulation und/oder Amplitudenmodulation und/oder Phasenmodulation in ein weiteres Signal über die Schnittstelle 200 weitergeleitet werden, beispielsweise über mindestens eine Antenne 210. Beispielsweise kann dieses Signal von einem Lesegerät, welches in einem exemplarischen Blockschaltbild gemäß Figur 6 mit der Bezugsziffer 212 bezeichnet ist, empfangen werden. Hierbei kann es sich beispielsweise um ein Lesegerät 212 mit einem Funkempfänger und/oder um ein RFID-Lesegerät handeln. Auch andere Ausgestaltungen sind möglich. Beispielsweise kann in der Ausgestaltung gemäß Figur 5 in dem Oszillator 205 der Sensorvorrichtung 110 eine Funk-Träger-Frequenz direkt durch die Eigenschaften des Kondensators 182 moduliert werden.

In Figur 6 ist ein Ausführungsbeispiel eines Sensorsystems 214 beschrieben, welches neben mindestens einer Sensorvorrichtung 110 mit mindestens einem elektrischen Sensor 112, beispielsweise gemäß einem oder mehreren der oben beschriebenen Ausgestaltungen, mindestens ein Lesegerät 212 umfassen kann. Der elektrische Sensor 112 kann beispielsweise in beliebiger Weise ausgestaltet sein und kann beispielsweise wiederum einen oder mehrere Feldeffekttransistoren 114, insbesondere organische Feldeffekttransistoren 116, und/oder einen oder mehrere Kondensatoren 182 und/oder eine oder mehrere Sensorelektroden 162 umfassen, wie in Figur 6 angedeutet. Der elektrische Sensor 112 kann insbesondere ein Implantat 216 und/oder einen in ein Körpergewebe insertierbaren Teil umfassen. Weiterhin kann der elektrische Sensor 112 und/oder die Sensorvorrichtung 110 wiederum mindestens eine Ansteuerung 154 umfassen, beispielsweise eine Ansteuerung 154 mit einer Signalverarbeitung, und/oder eine Ansteuerung 154 gemäß einer oder mehreren der oben beschrieben Ausgestaltungen. Weiterhin kann diese Ansteuerung 154 insbesondere wiederum mit einer oder mehreren Schnittstellen 200 verbunden sein oder teilidentisch mit mindestens einer derartigen Schnittstelle 200 sein. Im Rahmen der in Figur 6 gezeigten Ausgestaltung ist es besonders bevorzugt, wenn diese mindestens eine Schnittstelle 200 mindestens einen Transponder 218 umfasst. Auch andere Ausgestaltungen sind jedoch grundsätzlich möglich. Über diese mindestens eine Schnittstelle 200 ist ein Datenaustausch 220 mit dem Lesegerät 212 in einer unidirektionalen und auch bidirektionalen Weise möglich, beispielsweise ein kapazitiver und/oder induktiver und/oder elektromagnetischer Datenaustausch 220. Weiterhin kann jedoch von dem Lesegerät 212 auch optional Energie in die Sensorvorrichtung 110 eingespeist werden, was in Figur 6 mit der Bezugsziffer 222 bezeichnet ist. Diese Energieeinspeisung 222 kann beispielsweise analog zu herkömmlichen Transpondern 218 erfolgen, beispielsweise RFID-Transpondern, bei welchen, beispielsweise kapazitiv und/oder induktiv und/oder über mindestens einen Schwingkreis 206, elektromagnetische Energie von dem Lesegerät 212 eingespeist wird. Alternativ oder zusätzlich kann die Sensorvorrichtung 110 auch eine oder mehrere Energieversorgungen 224 umfassen, beispielsweise eine Energieversorgung 224 über eine Schnittstelle 200 und/oder eine Energieversorgung 224 über mindestens einen internen Energiespeicher, beispielsweise mindestens eine Batterie und/oder mindestens einen Akkumulator, und/oder mindestens eine Energieversorgung 224 über eine Energieentnahme aus einer Umgebung, beispielsweise ein sogenanntes Energy Harvesting. Wird, wie in Figur 6 als Option beschrieben, Energie drahtlos von außen aus dem Lesegerät 212 eingespeist, zum Beispiel nach einem Transponderprinzip, so kann damit zusätzlich eine Langzeitinsertion oder Langzeitimplantation begünstigt werden, da beispielsweise biologische Abstoßungseffekte von Zuleitungskabeln vermieden werden können und/oder da die gesamte Sensorvorrichtung 110 und/oder Teile derselben vergleichsweise kleinvolumig ausgestaltet werden können. Die Energiebereitstellung kann, wie oben beschrieben, beispielsweise über Induktion von außen nach dem RFID-Prinzip erfolgen.

In den Ausführungsbeispielen gemäß den Figuren 3A, 3B und 4 oder auch in anderen Ausführungsbeispielen, in denen die Sensorvorrichtung 110 mindestens einen elektrischen Sensor 112 in Form mindestens eines Kondensators 182 aufweist, können ebenfalls wiederum optional einen oder mehrere Kompensationssensoren 113 vorgesehen sein, deren elektrische Eigenschaften nicht oder nur unwesentlich durch die Analytkonzentration beeinflusst werden. Beispielsweise können jeweils ein oder mehrere Kompensationskondensatoren vorgesehen sein, was in den Figuren nicht dargestellt ist. Diese Kompensationskondensatoren können beispielsweise wiederum zwei oder mehr Elektroden aufweisen, beispielsweise in einer zum eigentlichen elektrischen Sensor 112 analogen Ausgestaltung, jedoch unspezifisch hinsichtlich der Analytkonzentration. Beispielsweise kann der mindestens eine optionale Kompensationskondensator mindestens eine Kompensations-Kondensatorelektrode und mindestens eine Gegenelektrode aufweisen.

Weiterhin können allgemein der elektrische Sensor 112 und/oder der Kompensationssensor 113 in eine elektrische Brückenschaltung 225 und/oder eine Kompensationsschaltung eingebunden sein. Dies kann sowohl bei der oben beschriebenen Ausgestaltungen unter Verwendung eines oder mehrerer Feldeffekttransistoren 114 oder auch bevorzugt bei den Ausgestaltungen mit einem oder mehreren Kondensatoren 182 erfolgen. Dies ist exemplarisch in Figur 7 anhand eines Ausführungsbeispiels mit Kondensatoren 182 gezeigt. Insbesondere kann der mindestens eine Kondensator 182 Bestandteil der Brückenschaltung 225, insbesondere einer Brücke, sein, wie beispielsweise in dem Ausführungsbeispiel gemäß Figur 7. Die Brückenschaltung 225 kann insbesondere mindestens einen Kondensator 182, beispielsweise als elektrischer Sensor 112 ausgestaltet, und/oder mindestens einen Kompensationskondensator 226, beispielsweise als Kompensationssensor 113 ausgestaltet, und/oder optional mindestens einen, vorzugsweise zwei, Brückenkondensatoren 228 und/oder mindestens eine Messvorrichtung 230 und/oder mindestens eine Ansteuerung 154 und/oder mindestens eine Detektorkammer 140 umfassen. Prinzipiell kann dieses Ausführungsbeispiel alternativ oder zusätzlich auch andere Komponenten, beispielsweise wie bei den anderen Ausführungsbeispielen beschrieben, umfassen, beispielsweise mindestens einen Feldeffekttransistor 114.

Der elektrische Sensor 112 und der Kompensationssensor 113 können beispielsweise ausgestaltet sein wie in den Ausführungsbeispielen gemäß den Figuren 3A und 3B oder 4 beschrieben. Die Brückenkondensatoren 228 können bevorzugt wie Kondensatoren 182 ausgestaltet sein. Prinzipiell kann die Brückenschaltung 225 alternativ oder zusätzlich auch ohmsche Widerstände und/oder induktive Widerstände, beispielsweise mindestens eine Spule 208, umfassen.

Der elektrische Sensor 112, insbesondere der Kondensator 182, und/oder der Kompensationssensor 113, insbesondere der Kompensationskondensator 226, können zumindest teilweise von dem fluiden Medium 152 umspült sein. Die Detektorkammer 140 kann ebenfalls ähnlich den vorhergehenden Ausführungsbeispielen ausgestaltet sein, insbesondere kann die Detektorkammer 140 zumindest teilweise innerhalb des elektrischen Sensors 112, insbesondere des Kondensators 182, und/oder des Kompensationssensors 113, insbesondere des Kompensationskondensators 226, angeordnet sein, insbesondere zumindest teilweise jeweils zwischen der Sensorelektrode 162, beispielsweise als Gate-Elektrode 130, vorzugsweise als Kondensatorelektrode, ausgestaltet, und der Gegenelektrode 172, beispielsweise ebenfalls als Kondensatorelektrode ausgestaltet. Der prinzipielle Aufbau der Brückenschaltung 225 kann insbesondere dem Fachmann bekannt sein. In dem Ausführungsbeispiel gemäß Figur 7 ist beispielsweise eine Reihenschaltung aus dem elektrischen Sensor 112 und einem der beiden Brückenkondensatoren 228 parallel zu einer Reihenschaltung aus dem Kompensationssensor 113 und dem anderen der beiden Brückenkondensatoren 228 geschaltet. Beide Reihenschaltungen können insbesondere mit einem Wechselspannungsgenerator 190 mit einer Wechselspannung, insbesondere einer Eingangsspannung $U_i$, beaufschlagt werden. Eine Brückendiagonale, insbesondere die beiden Reihenschaltungen mittig elektrisch verbindend, kann insbesondere eine Messvorrichtung 230 umfassen, bevorzugt um eine elektrische Spannung, bevorzugt eine elektrische Wechselspannung, insbesondere eine Ausgangsspannung $U_0$, zu erfassen. Die Messvorrichtung 230 kann insbesondere mindestens eine Spannungsmessvorrichtung, bevorzugt mindestens eine Wechselspannungsvorrichtung, und/oder mindestens eine Strommessvorrichtung, bevorzugt mindestens eine Wechselstrommessvorrichtung, umfassen. Ein Verhältnis aus Ausgangsspannung $U_0$ und Eingangsspannung $U_i$ kann beispielsweise ausgedrückt werden durch:

$$\frac{U_0}{U_i} = \frac{C_x - C_{Ref}}{C},$$

wobei $C_x$ eine Kapazität des elektrischen Sensors 112, insbesondere des Kondensators 182, darstellen kann, $C_{Ref}$ eine Kapazität des Kompensationskondensators 226 und/oder des Kompensationssensors 113 und C eine Kapazität mindestens eines, vorzugsweise zweier, Brückenkondensatoren 228. Die Brückenschaltung 225 kann somit insbesondere verwendet werden, um die Kapazität $C_x$ des elektrischen Sensors 112, welche insbesondere beispielsweise von einer Dielektrizitätskonstante bestimmt sein kann, zu bestimmen, bevorzugt kann hierbei der mindestens eine Analyt 144 nachgewiesen werden.

Ausführungsbeispiele zur Ausgestaltung der Detektorsubstanz 150:

Hinsichtlich der Ausgestaltung der Detektorsubstanz 150, insbesondere hinsichtlich der elektrisch geladenen Teilchen 176 und/oder der Spacer 178 und/oder der Rezeptoren 160, bestehen verschiedene Möglichkeiten, die grundsätzlich auf alle Ausführungsbeispiele anwendbar sind. Hierbei ist grundsätzlich zu unterscheiden zwischen zwei Prinzipien, welche auch miteinander kombinierbar sind, nämlich dem Prinzip der Generierung von Affinität ohne eine Analyt-Umsetzung und dem Prinzip einer Generierung von Affinität mit einer Analyt-Umsetzung.

1. Ausführungsbeispiele elektrisch geladener Teilchen 176:

Als elektrisch geladene Teilchen 176 können in den verschiedenen Ausführungsbeispielen sowohl freie als auch optional über mindestens einen Spacer 178 in ihrer Mobilität eingeschränkte elektrische geladene Teilchen 176 eingesetzt werden.

Beispielsweise lassen sich ein oder mehrere Arten von Molekülen mit geladenen ionischen Gruppen einsetzen, welche optional Spacer-gebunden sein können oder welche, alternativ oder zusätzlich, durch eine geeignete Kompartimentierung in der Detektorkammer 140 räumlich in ihrem Funktionsbereich und/oder Bewegungsbereich fixiert sein können.

Die elektrisch geladenen Teilchen 176 sollten in der Regel folgende Eigenschaften aufweisen:

a) Eine Molekülgröße deutlich voluminöser als die Spacer 178, in der Regel in der Größenordnung des Analyten 144, jedoch vorzugsweise keine Polymere.

b) Eine definierte, einheitliche Struktur, idealerweise kugelförmig (globulär, jedoch vorzugsweise ohne chemische oder sterische Affinität zur Oberfläche).

c) Puffereigenschaften, die dazu führen, dass im Bereich der herrschenden Umgebungsbedingungen eine konstante Ladung und eine eindeutige einheitliche Ladung, beispielsweise stets negativ, gegeben sind. Kritischer wären elektrisch geladene Teilchen 176, die aufgrund einer physiologischen pH-Änderung eine variierende Ladung aufweisen würden und daher mangels einer nur Analyt-abhängigen Gleichgewichtseinstellung zwischen dem Medium und der Oberfläche in der Regel ausscheiden.

d) Zur Steigerung der Feldstärken eine vorzugsweise relativ hohe Ladung, beispielsweise durch mehr als eine, vorzugsweise durch mehr als zwei anionische Gruppen.

e) Eine leichte synthetische Zugänglichkeit, insbesondere der bevorzugten Möglichkeit der Anbindung über mindestens einen Spacer 178.

Ausführungsformen elektrisch geladener Teilchen 176 sind beispielsweise Polycarbonsäuren des Benzols, also Benzolcarbonsäuren. Diese sind in der Regel auch sehr gute Puffer, da diese durch Interaktion über hydroxylische H-Brücken zwischen benachbarten dissoziierten und undissoziierten Carboxylgruppen eine Glättung der pKs-Werte der einzelnen Säuregruppen bewirken. Dies bedeutet, dass die pKs-Werte der einzelnen Stufen in der Regel bei gezielter Positionierung der Carboxylgruppen nahe beisammen liegen, sodass pH-Änderungen hervorragend abgepuffert werden, ohne dass sich prinzipiell eine grundsätzliche Ladungsumkehr, also von negativ nach neutral oder positiv, ergibt. Alternativ oder zusätzlich sind aufgrund der Positionierung pKs-Sprünge vorhanden. Es gibt also in dieser Stoffklasse eine variable Vielfalt.

Als Beispiele, welche auch beliebig paarweise oder in Gruppen untereinander oder mit anderen Beispielen kombinierbar sind, bietet sich für die elektrisch geladenen Teilchen 176 insbesondere der Prototyp der mehrwertigen Benzolcarbonsäuren an und zwar ausgewählt aus der Gruppe bestehend aus Benzoldicarbonsäure (H2), beispielsweise Phthalsäure, Benzoltricarbonsäure (H3) unterschiedlicher Art, beispielsweise Trimesinsäure oder Trimellitsäure, Benzoltetracarbonsäure (H4), beispielsweise Pyromellitsäure, Benzolpentacarbonsäure (H5) und Benzolhexacarbonsäure (H6), (Mellitsäure); wobei in dieser eine Anbindung an den Spacer 178 nur über eine der sechs Carboxylgruppen möglich wäre, und beispielsweise von den genannten Substanzen abgeleitete Moleküle, beispielsweise Dimere und/oder Trimere und/oder Oligomere und/oder aber auch deren Komplexe mit Metallkationen und/oder mit protonierten organischen Basen, beispielsweise Alkylamine und/oder Heterocyclen mit jeweils mehreren basischen Aminogruppen und/oder Stickstoffatomen. Die Mellitsäure bietet sich wie erwähnt dann an, wenn ein an deren Carboxylgruppe gebundener Spacer 178 bevorzugt ist, insbesondere deshalb, da die Mellitsäure auf Grund der Anordnung der Carboxylgruppen der Mellitsäure satt auf der Oberfläche fixiert wären und somit beispielsweise kaum oder keine Bewegungsfreiheit aufweisen würde.

2. Ausführungsbeispiele für Spacer 178:

In dem Fall, in welchem die elektrisch geladenen Teilchen 176 oder Teile derselben an mindestens eine Oberfläche über mindestens einen Spacer 178 angebunden werden sollen, können beispielsweise ein oder mehrere der oben beschriebenen Arten ionisch vorliegender Moleküle und/oder andere Arten elektrisch geladener Teilchen 176 an bewegliche Spacer 178 gebunden werden, und diese Spacer 178 dann wiederum durch Pfropfung beispielsweise auf einer polymeren Oberfläche und/oder einer anderen Art einer organischen oder anorganischen Oberfläche verankert werden.

Beispiele beweglicher Spacer 178, welche auch kombinierbar sind, sind insbesondere aliphatische Ketten (Alkylketten), Polyether-Ketten, Polyimino-Ketten oder Kombinationen der genannten und/oder anderer Arten von Spacer 178. Eine Ladungsneutralität der Spacer 178 sollte dabei gewährleistet sein. Eine Besetzungsdichte und/oder eine Hydrophilie der Spacer-Ketten können einen Einfluss auf die Bindung des Analyten 144, beispielsweise Glucose, im Pfropfbereich haben. In bestimmten Anwendungen können auch unpolare Spacer-Ketten vorteilhaft sein, beispielsweise zur Kanalisierung einer Analyt-Annäherung, insbesondere einer Glucose-Annäherung an einen oder mehrere Rezeptoren 160. Andererseits können in bestimmten Fällen polare Anteile im Spacer 178 den Analyten 144, beispielsweise die Glucose, leichter an den mindestens einen Rezeptor 160 heranführen.

3. Ausführungsbeispiele für Rezeptoren 160:

Um den mindestens einen optionalen Rezeptor 160 in der Detektorsubstanz 150 bereitzustellen, bestehen mehrere Möglichkeiten. Insbesondere kann eine Analyt-spezifische Affinität an mindestens eine Oberfläche innerhalb der Detektorkammer 140, beispielsweise eine Oberfläche der mindestens einen Sensorelektrode 162, generiert werden, sodass bezüglich der Rezeptoren 160 ohne Beschränkung weiterer möglicher Ausgestaltungen, im Folgenden allgemein von einer Affinität gesprochen wird. Mittels dieser Rezeptoren 160 kann beispielsweise die Veränderung der Ladungsverteilung und/oder die Veränderung anderer elektrischer Eigenschaften gewährleistet werden, beispielsweise die Veränderung dielektrischer Eigenschaften innerhalb der Detektorkammer 140 und/oder die Veränderung elektrischer Felder. Ein Nachweis kann beispielsweise über polare Gruppen und Dielektrizitätsänderungen erfolgen.

Wie oben beschrieben, kann die mindestens eine Sensorelektrode 162 mindestens eine leitfähige Schicht umfassen, kann jedoch auch, alternativ oder zusätzlich, einfach eine Oberfläche eines Bauelements sein oder umfassen, insbesondere eine Oberfläche eines Feldeffekttransistors 114, vorzugsweise eines organischen Feldeffekttransistors 116, beispielsweise eine Oberfläche eines Halbleiters 132, insbesondere eines organischen Halbleiters 134. Beispielsweise kann es sich hierbei um eine Polymer-Oberfläche handeln. Alternativ oder zusätzlich kann zwischen dieser mindestens einen Oberfläche und der Detektorkammer 140, insbesondere das Medium innerhalb der Detektorkammer 140, mindestens ein Abstandhalter 170 eingebracht sein, wie oben beschrieben, sodass auch eine Oberfläche beispielsweise des Abstandhalters 170 und/oder eine Innenoberfläche eines Gehäuses 142 der Detektorkammer 140 im Bereich der Gate-Elektrode 130 bzw. Sensor-Elektrode 162 als relevante Oberfläche angesehen werden kann.

Beispielsweise kann eine anfängliche Polymer-Oberfläche des elektrischen Sensors 112 (POS) vorgesehen sein. Beispielsweise kann ein elektrisch leitendes Polymer mit einer chemisch modifizierten Sensor-Oberfläche vorgesehen sein, an welcher der Analyt 144 über eine affine, reversible Bindung andocken kann. Diese Analyt-erkennende Oberfläche der Sensorelektrode 162 und/oder allgemein eine Oberfläche, welche den mindestens einen Rezeptor 160 aufweist, und/oder der Rezeptor 160 selbst, werden im Folgenden als Sensor-Oberflächen-Chemie (SOC) bezeichnet. Die SOC beinhaltet somit die Detektorsubstanz 150 oder einen Teil derselben, insbesondere den optionalen Rezeptor 160 oder Teile desselben, insbesondere die Analyt-spezifischen Nachweisstrukturen und/oder Teile derselben. Die im Folgenden aufgeführten Analytnachweise beruhen in der Regel auf der variablen und zeitlich aufgelösten Annäherung von geladenen chemischen Gruppen der SOC an die Polymer-Oberfläche des Sensors (POS) und/oder an eine andere Oberfläche, wodurch die elektrischen oder dielektrischen Eigenschaften des Mediums in der Detektorkammer 140 beeinflusst werden können.

Da es zu den geladenen und gegebenenfalls an ein organisches Material, insbesondere ein Polymer, gebundenen Gruppen, insbesondere die elektrisch geladenen Teilchen 176, auch in der Regel Gegenionen gibt, beispielsweise leichter bewegliche Gegenionen, besteht eine Voraussetzung in der Regel darin, dass bei Ladungsbewegungen zwischen diesen beiden Ionensorten relativ zu dem elektrischen Sensor 112, beispielsweise über das Anlegen eines Wechselfeldes, ein Potenzialgefälle besteht, sich beispielsweise Dielektritzitätsschwankungen ergeben und diese registriert werden können.

A: Generierung von Affinität ohne Analyt-Umsetzung

Um den Analyten 144 spezifisch und reversibel zu binden, beispielsweise mittels geeigneter Rezeptoren 160, bedarf es in der Regel affiner Bindungsstellen, zum Beispiel der Bindungsstellen eines Analyt-spezifischen Enzyms. Vorzugsweise findet keinerlei Umsetzung des Analyten 144 trotz einer Detektion statt. Der mindestens eine Rezeptor 160 kann also beispielsweise mindestens ein Enzym enthalten.

Ohne eine Analyt-Umsetzung wird die herkömmliche Nachweiskette, welche üblicherweise mit einer chemischen Umsetzung beginnt, maximal möglich verkürzt. Der bevorzugte Ausschluss einer enzymatischen Umsetzung als Option im Rahmen der vorliegenden Erfindung gestattet es im Prinzip, nur den Bereich der affinen Bindungsstellen eines Enzyms als Bindungspartner einzusetzen und die volle Funktionalität des Enzyms unberücksichtigt zu lassen. Dies kann die Nachweisfähigkeit des affinen Systems, beispielsweise des Rezeptors 160, gegenüber einer Inaktivierung, beispielsweise gegenüber einer Denaturierung, robuster gestalten und eine pH-Abhängigkeit, welche klassischerweise die Umsatzrate mitbestimmt, herabsetzen oder in einem hinreichend stabilen Umfeld sogar unbedeutend machen, was gegebenenfalls auch die Anwesenheit geladener Spacer-Gruppen erleichtert.

Die ursprüngliche Eigenschaft eines Enzyms bei klassischen Sensoren wird dabei vorzugsweise reduziert auf eine affine substanzspezifische Bindung ohne chemische und/oder enzymatische Umsetzung. Für den Fall des Nachweises von Glucose als Beispiel eines Analyten 144 ist ein konsequenter Schritt in diese Richtung die Einführung von Lectinen als Rezeptor 160, welche per Definitionen spezifisch Zucker binden. Besonders bevorzugt ist es also, wenn der mindestens eine Rezeptor 160 mindestens ein Lectin umfasst, beispielsweise ausgewählt aus der Gruppe Glucosebindender Lectine, insbesondere solcher Lectine, die für einen Glucose-Nachweis eingesetzt werden können. Ein bekanntes Beispiel ist Concanavalin A, auch Con A, welches beispielsweise aus der Schwertbohne oder der Jackbohne gewonnen wird, und/oder lens culinaris-Agglutinin, welches beispielsweise aus der Linse gewonnen wird, und/oder Pealectin-I (PSA), welches beispielsweise aus der Erbse gewonnen wird (pisum sativum). Die Verwendung von Con A als Glucose-Rezeptor ist grundsätzlich aus der Literatur bekannt, beispielsweise aus U. Beyer et al.: Recording of subcutaneous glucose dynamics by a viscometric affinity sensor, Diabetologia (2001) 44:416-423. In diesem oder auch in anderen Ausführungsbeispielen kann die Detektorsubstanz 150 beispielsweise auch mindestens einen Konkurrenten umfassen, beispielsweise einen Konkurrenten, welcher mit dem nachzuweisenden Analyten 144 um die Bindung an den Rezeptor 160 konkurriert. Für den Fall des Con A als Rezeptor 160 und beispielsweise Glucose als Analyt 144 ist beispielsweise die Verwendung von Glucose-markiertem makromolekularen Dextran (Dex-Gluc4) bekannt, welches mit freier Glucose um die Bindung zu Con A konkurriert. Da das Dex-Gluc4 und Con A poylvalent binden können, entsteht in der Regel im wässrigen bzw. physiologischen Medium rasch ein polymeres Adukt hoher Viskosität. In dem Maße wie Glucose hinzutritt werden die (in der Regel je vier) polyvalenten Verknüpfungen von Dextran-Gluc und Con A aufgebrochen und durch monomere Con A-(4)-Glucose-Einheiten ersetzt, womit die Viskosität abnimmt. Dieser Mechanismus kann in verschiedener Ausgestaltung im erfindungsgemäßen Sensor eingesetzt werden, beispielsweise zur Veränderung einer Mobilität und/oder zur Beeinflussung einer Ladungsverteilung und/oder zur Beeinflussung dielektrischer Eigenschaften des Mediums innerhalb der Detektorkammer 140, beispielsweise durch Belegung einer oder mehrerer der Elektroden, insbesondere der Sensorelektrode 162, beispielsweise mit dem Ziel, dass geladene Kopfgruppen der beschrieben Spacer 178 sich mehr oder weniger gut an eine Oberfläche annähern können. Die Möglichkeit der Annäherung kann beispielsweise reziprok eine Funktion der Anteile an Dextran (Dex-Gluc4)-Con A und damit direkt eine Funktion der Konzentration freier Glucose sein, beispielsweise wenn die Beweglichkeit entsprechender Gegenionen unterschiedlich ausgestaltet ist.

Eine in der Regel riskante, kontinuierliche Versorgung des elektrischen Sensors 112, insbesondere der Detektorkammer 140, mit Lectin (beispielsweise Con A) ist hierfür in der Regel nicht erforderlich. Stattdessen reicht in der Regel eine einmalige, geringe Belegung, beispielsweise der Sensorelektrode 162 und/oder einer anderen Innenoberfläche der Detektorkammer 140. Hierdurch kann insbesondere das Problem einer mengenabhängigen Toxizität ausgeschlossen werden. Idealerweise kann der Rezeptor 160 in der Detektorkammer 140 immobilisiert sein, beispielsweise an der Sensorelektrode und/oder einer anderen Elektrode und/oder an einer anderen Innenoberfläche der Detektorkammer 140, beispielsweise einem Inneren des Gehäuses 142 und/oder auf andere Weise innerhalb der Detektorkammer 140, beispielsweise in einem oder mehreren zusätzlich in der Detektorkammer 140 aufgenommenen Elemente.

Dextran als weiteres Agens, insbesondere als Konkurrent, ist in der Regel untoxisch, beispielsweise als geladenes Reportermolekül, welches optional auch beispielsweise, jedoch nicht zwingenderweise, an einen Spacer 178 gekoppelt sein kann. Vorzugsweise sind hier ebenfalls untoxische Substanzen zu wählen, die als erforderliche Randbedingungen in der Regel eine konstante Ladung aufweisen sollten und nicht membrangängig sein sollten.

B: Generierung von Affinität mit Analyt-Umsetzung

Um den Analyten 144 über dessen Umsetzung nachzuweisen, was optional zu einem Umsetzungs-freien Nachweis möglich ist, bedarf es in der Regel eines Analyt-spezifischen Reagens, beispielsweise eines Analyt-spezifischen Enzyms. In der Regel entstehen zunächst als Hauptprodukt der umgesetzte Analyt 144 und ein oder mehrere Nebenprodukte. Bei den klassischen Nachweisen eines enzymatisch umgesetzten Analyten 144 werden dann häufig die Nebenprodukte über eine spezielle Testchemie weiter umgesetzt und diese dann erst nachgewiesen. Beispielsweise wird bei der enzymatischen Oxidation von Glucose nicht das oxidierte Gluconolacton, sondern das Beiprodukt $H_2O_2$, insbesondere der darin befindliche reduzierte Sauerstoff, über eine oder mehrere weitere Folgereaktionen, beispielsweise über eine Farbstoffbildung und/oder elektrochemisch, detektiert. Bei einem Messprinzip unter Umsetzung des Analyten 144 ist es nun prinzipiell möglich, diesen optional allein über die Anwesenheit und Zersetzung des Nebenprodukts, vorzugsweise nicht jedoch über dessen weitere Umsetzung, zu detektieren. Auch in diesem Fall wird somit die klassische Nachweiskette bevorzugt abgekürzt. Für eine solche abgekürzte Detektion des Analyten 144 können folgende Möglichkeiten genannt werden:

B1: Detektion über eine pH-abhängige Volumen- und Ladungsänderung am Beispiel von Glucose:

Beispielsweise zerfällt das primäre Umsetzungsprodukt $H_2O_2$ zu $H_2O$ und $H^+$ und führt so zu einer Erniedrigung des pH-Werts innerhalb der Detektorkammer 140, was wiederum über den folgenden Mechanismus detektiert werden kann: als Sensor-Oberflächen-Chemie-SOC kann ein polyanionisches Polymer verwendet werden, beispielsweise ein Polyacrylsäurederivat, wobei im gequollenen Zustand schwach saure Gruppen anionisch vorliegen können. Das Polymer kann gequollen ausgestaltet sein, da dieses hydrophil sein kann und da sich die geladenen Gruppen gegenseitig abstoßen können. Setzt nun eine Analyt-spezifische Reaktion ein, bei welcher Protonen entstehen, so werden die anionischen Gruppen durch Protonierung ladungsneutral. Die ladungsbedingte Quellung des Polymers geht somit zurück, was sich wiederum auf die elektrischen Eigenschaften des elektrischen Sensors 112 auswirkt, beispielsweise die dielektrischen Eigenschaften des Kondensators 182. Alternativ kann beispielsweise eine Längsstreckung eines Polyanions, insbesondere auf Grund einer Abstoßung von negativen Ladungen im Polyanion, beispielsweise eine Strukturänderung in Form einer Verknäuelung erfahren, insbesondere, wenn beispielsweise der pH-Wert abnimmt und/oder das gestreckte Polyanion durch Protonierung entladen wird.

B2: Definition über die Ladung ionischer Gruppen und Dipolmomente:

Findet eine Quellung der Detektorsubstanz 150 und/oder von Teilen derselben bei Anwesenheit und/oder Abwesenheit des Analyten 144 statt, sodass sich ein Quellungszustand der Detektorsubstanz 150 und/oder von Teilen derselben ändert, so kann diese Quellung von dem elektrischen Sensor 112 beispielsweise über zusätzliche Ladungen an der Sensorelektrode 162 und/oder deren Abstände von der Sensorelektrode 162, beispielsweise über zusätzliche Ladungen des Polymers und/oder deren Abstände von der Polymer-Oberfläche des Sensors (POS), registriert werden. So können beispielsweise zusätzliche Ladungen von stabilen Anionen höherer Säurestärke, beispielsweise Sulfonaten und/oder Heparinähnlicher Polymere, als Funktion ihres Abstands von der POS in der Detektorkammer 140 registriert werden. Dies hieße, dass in einem quellfähigen, beispielsweise hydrophilen Polymer funktionelle Gruppen vorhanden sein könnten, die bei Protonierung ladungs-neutral werden und die Quellung reduzieren. Ferner würde dies bedeuten, dass einige andere Gruppen weiterhin geladen bleiben könnten, wenn ein Enzym durch Umsetzung des Analyten 144 unter anderem beispielsweise Protonen liefert. Weiterhin könnte dies bedeuten, dass die Beweglichkeit von geladenen Gruppen vom elektrischen Sensor 112 verfolgt werden kann, aufgrund der Änderung elektrischer Eigenschaften des elektrischen Sensors 112. Wegen des unterschiedlichen Wassergehalts in Folge der Quellung könnte dann auch oder bevorzugt das Dipolmoment der quellfähigen Detektorsubstanz 150, beispielsweise der quellfähigen Polymerschicht, an der Sensorelektrode 162 ein Maß für die Glucosekonzentration oder allgemein die Analytkonzentration darstellen.

B3: Abgleich:

Da beispielsweise in dem elektrischen Sensor 112 zwei Bereiche vorhanden sein können, die sich beispielsweise nur durch die Aussage Analyt-Bindung: ja/nein bzw. Enzymaktivität: vorhanden/nicht vorhanden unterscheiden können, wären Analyt-unspezifische Veränderungen, wie beispielsweise eine pH-Wert-Erhöhung in der Detektorkammer 140 beispielsweise nach kurzer Zeit erkennbar, d.h. die Tatsache einer Analyt-bedingten pH-Erhöhung wäre in der Regel gegenüber einer unspezifischen Änderung detektierbar.

B4: Regenerationsphasen:

Eine Analyt-bedingte pH-Erniedrigung würde beispielsweise durch Diffusion zu einem pH-Ausgleich

a) zwischen verschiedenen Bereichen der Detektorkammer 140, beispielsweise zwischen zwei Kompartimenten der Detektorkammer 140, führen und hierdurch das Differenzsignal nivellieren, und/oder

b) zwischen einem Inneren der Detektorkammer 140 und einer Membran-getrennter Körperflüssigkeit führen.

Da die Diffusion sowohl von Analyt 144, insbesondere Glucose, als auch ein pH-Ausgleich in der Regel kontinuierlich ablaufen, sollte durch das Design der Sensorvorrichtung 110, insbesondere der Detektorkammer 140, gewährleistet sein, dass einer bestimmten Analyt-Konzentration, beispielsweise einem bestimmten Glucosegehalt, ein stabiles eindeutiges mit der Analytkonzentration korrelierendes Sensorsignal gegenübersteht. Beispielsweise könnte ein pH-Ausgleich in eine Peripherie rascher verlaufen als zwischen verschiedenen Sensorkomponenten der Sensorvorrichtung 110.

B5: Detektion über den Nachweis eines Quellungsdrucks:

Eine weitere Möglichkeit der Ausgestaltung der Detektorsubstanz 150 besteht wiederum darin, eine Analyt-bedingte Quellung mindestens einer Komponente der Detektorsubstanz 150 zu gewährleisten. Beispielsweise kann wiederum eine pH-bedingte Quellung direkt oder indirekt nachgewiesen werden. Beispielsweise kann eine Detektorkammer 140 mit zwei oder mehr Kompartimenten verwendet werden. Beispielsweise wäre dann eine unspezifische Quellung, da diese von einer externen Seite her erfolgt, gleich groß. Dem würde sich die in beispielsweise einem Kompartiment ablaufende Enzym-bedingte Quellung überlagern. Diese Quellung selbst könnte beispielsweise über eine Drucksensorik registriert werden, sofern eine geeignete Membran 148 einen Druckausgleich nach außen verhindern könnte. Ein Druck könnte sich beispielsweise in Form einer Abstandsänderung und damit einer Kapazitätsänderung auf den Kondesator 182 auswirken, beispielsweise über ein Elastomer. Abstandsänderungen und damit Kapazitätsänderungen könnten auf diese Weise erzielt werden und wiederum in einem oder mehreren der oben beschriebenen Verfahren und Sensorvorrichtungen 110 einsetzbar sein.

<div align="center">Bezugszeichenliste</div>

| | |
|---|---|
| 110 Sensorvorrichtung | 174 Spannungsquelle |
| 112 elektrischer Sensor | 175 Umschalter |
| 113 Kompensationssensor | 176 elektrisch geladene Teilchen |
| 114 Feldeffekttransistor | 178 Spacer |
| 116 organischer Feldeffekttransistor | 180 Messwiderstand |
| 118 Operationsverstärker | 182 Kondensator |
| 120 Widerstandszweig | 184 erste Elektrode |
| 122 Widerstandszweig | 186 zweite Elektrode |
| 124 Festwiderstand | 188 Gleichgewicht |
| 126 Festwiderstand | 190 Wechselspannungsgenerator |
| 128 Subtrahierstufe | 192 Elektrode |
| 130 Gate-Elektrode | 194 Elektrode |
| 132 Halbleiter | 196 Isolator |
| 134 organischer Halbleiter | 198 Öffnung |
| 136 Source-Elektrode | 200 Schnittstelle |
| 138 Drain-Elektrode | 202 drahtlose Schnittstelle |
| 140 Detektorkammer | 204 Funkschnittstelle |
| 142 Gehäuse | 205 Oszillator |
| 144 Analyt | 206 Schwingkreis |
| 146 Fluid | 208 Spule |
| 148 Membran | 210 Antenne |
| 150 Detektorsubstanz | 212 Lesegerät |
| 152 fluides Medium | 214 Sensorsystem |
| 154 Ansteuerung | 216 Implantat |
| 156 Versorgungsspannung | 218 Transponder |
| 158 Stromquelle | 220 Datenaustausch |
| 160 Rezeptor | 222 Energieeinspeisung |
| 162 Sensorelektrode | 224 Energieversorgung |
| 163 Kompensations-Sensorelektrode | 225 Brückenschaltung |
| 164 unspezifische Moleküle | 226 Kompensationskondensator |
| 166 Spannungsquelle | 228 Brückenkondensator |
| 168 Substrat | 230 Messvorrichtung |
| 170 Abstandhalter | |
| 172 Gegenelektrode | |

**Patentansprüche**

1. Sensorvorrichtung (110) zum Nachweis mindestens eines Analyten (144) in einem Fluid (146), insbesondere in einer Körperflüssigkeit, umfassend mindestens eine geschlossene Detektorkammer (140) und mindestens einen elektrischen Sensor (112) mit mindestens einer Sensorelektrode (162), wobei die Detektorkammer (140) derart mit dem Fluid (146) in Verbindung bringbar ist, dass der Analyt (144) in die Detektorkammer (140) eindringen kann, wobei die Detektorkammer (140) mindestens eine Detektorsubstanz (150) umfasst, wobei die Detektorsubstanz (150) eingerichtet ist, um mindestens eine elektrische Eigenschaft des elektrischen Sensors (112), insbesondere mindestens eine elektrische Eigenschaft der Sensorelektrode (162), in Abhängigkeit von einer Konzentration des Analyten (144) in der Detektorkammer (140) zu beeinflussen, wobei die Detektorsubstanz (150) derart ausgestaltet ist, dass dielektrische Eigenschaften der Detektorsubstanz (150) abhängig sind von einer Konzentration des Analyten (144) in der Detektorkammer (140), wobei die Detektorsubstanz (150) elektrisch geladene Teilchen (176) umfasst, insbesondere elektrisch geladenen Partikel und/oder elektrisch geladene Moleküle, wobei die Detektorkammer (140) und die Detektorsubstanz (150) derart ausgestaltet sind, dass ein mittlerer Abstand der elektrisch geladenen Teilchen (176) von der Sensorelektrode (162) und/oder eine Ladungsverteilung innerhalb der Detektorkammer (140) abhängig sind von einer Konzentration des Analyten (144) in der Detektorkammer (140), wobei in der Detektorkammer (140) mindestens ein Rezeptor (160) aufgenommen ist, wobei der Rezeptor (160) eingerichtet ist, um den Analyten (144) reversibel zu binden.

2. Sensorvorrichtung (110) nach dem vorhergehenden Ansprüche, wobei die Detektorsubstanz (150) weiterhin mindestens einen Spacer (178) umfasst, wobei der Spacer (178) eingerichtet ist, um die elektrisch geladenen Teilchen (176) an die Sensorelektrode (162) und/oder eine Oberfläche der Detektorkammer (140) an der Sensorelektrode (162) zu binden, wobei der Spacer (178) eingerichtet ist, um mindestens zwei Stellungen einzunehmen, in welchen die elektrisch geladenen Teilchen (176) einen unterschiedlichen Abstand zu der Sensorelektrode (162) aufweisen.

3. Sensorvorrichtung (110) nach einem der vorhergehenden Ansprüche, wobei der Rezeptor (160) ein immobilisierter Rezeptor (160) ist, vorzugsweise ein an der Sensorelektrode (162) und/oder einer Oberfläche der Detektorkammer (140) an der Sensorelektrode (162) immobilisierter Rezeptor (160).

4. Sensorvorrichtung (110) nach einem der vorhergehenden Ansprüche, wobei die Detektorsubstanz (150) mindestens ein Nachweisreagens umfasst, wobei das Nachweisreagens eingerichtet ist, um mit dem Analyten (144) zu reagieren, insbesondere mindestens ein Enzym.

5. Sensorvorrichtung (110) nach einem der vorhergehenden Ansprüche, wobei die Detektorkammer (140) mindestens eine Membran (148) aufweist, insbesondere mindestens eine Analyt-spezifische Membran (148), wobei die Membran (148) eingerichtet ist, um das Eindringen des Analyten (144) in die Detektorkammer (140) reversibel zu ermöglichen und vorzugsweise die mindestens eine Detektorsubstanz (150) in der Detektorkammer (140) zurückzuhalten.

6. Sensorvorrichtung (110) nach einem der vorhergehenden Ansprüche, wobei der elektrische Sensor (112) zusätzlich zu der Sensorelektrode (162) weiterhin mindestens eine Gegenelektrode (172) umfasst, wobei die Sensorvorrichtung (110) eingerichtet ist, um die Gegenelektrode (172) mit mindestens einem elektrischen Potenzial zu beaufschlagen.

7. Sensorvorrichtung (110) nach einem der vorhergehenden Ansprüche, wobei die Sensorvorrichtung (110) weiterhin mindestens einen Kompensationssensor (113) umfasst.

8. Sensorvorrichtung (110) nach dem vorhergehenden Anspruch, wobei der Kompensationssensor (113) mindestens eine Kompensations-Sensorelektrode (163) umfasst, wobei die Detektorsubstanz (150) mit der Sensorelektrode (162) verbunden ist, wobei die Kompensations-Sensorelektrode (163) frei von der Detektorsubstanz (150) ist.

9. Sensorvorrichtung (110) nach einem der beiden vorhergehenden Ansprüche, wobei der elektrische Sensor (112) und der Kompensationssensor (113) in eine elektrische Brückenschaltung (225) eingebunden sind.

10. Sensorvorrichtung (110) nach einem der vorhergehenden Ansprüche, wobei der elektrische Sensor (112) mindestens einen Feldeffekttransistor (114) umfasst, insbesondere einen organischen Feldeffekttransistor (116), wobei die Sensorelektrode (162) mindestens eine Gate-Elektrode (130) des Feldeffekttransistors (114) umfasst.

11. Sensorvorrichtung (110) nach einem der vorhergehenden Ansprüche, wobei die Sensorvorrichtung (110) weiterhin mindestens eine Ansteuerung (154) umfasst, wobei die Ansteuerung (154) eingerichtet ist, um die mindestens eine elektrische Eigenschaft des elektrischen Sensors (112) zu erfassen.

**12.** Sensorvorrichtung (110) nach einem der vorhergehenden Ansprüche, wobei der elektrische Sensor (112), insbesondere die Sensorelektrode (162), mindestens ein organisches leitfähiges oder halbleitendes Material (134) aufweist, insbesondere mindestens ein leitfähiges oder halbleitendes Polymer.

**13.** Sensorsystem, umfassend mindestens eine Sensorvorrichtung (110) nach einem der vorhergehenden Ansprüche und mindestens ein Lesegerät (212), wobei die Sensorvorrichtung (110) und das Lesegerät (212) eingerichtet sind, um miteinander zu kommunizieren, insbesondere drahtlos.

**14.** Verfahren zum Nachweis mindestens eines Analyten (144) in einem Fluid (146), insbesondere unter Verwendung einer Sensorvorrichtung (110) nach einem der vorhergehenden, eine Sensorvorrichtung (110) betreffenden, Ansprüche, wobei mindestens eine geschlossene Detektorkammer (140) und mindestens ein elektrischer Sensor (112) mit mindestens einer Sensorelektrode (162) verwendet werden, wobei die Detektorkammer (140) derart mit dem Fluid (146) in Verbindung gebracht wird, dass der Analyt (144) in die Detektorkammer (140) eindringt, wobei die Detektorkammer (140) mindestens eine Detektorsubstanz (150) umfasst, wobei die Detektorsubstanz (150) mindestens eine elektrische Eigenschaft des elektrischen Sensors (112) in Abhängigkeit von einer Konzentration des Analyten (144) in der Detektorkammer (140) beeinflusst, wobei die Detektorsubstanz (150) derart ausgestaltet ist, dass dielektrische Eigenschaften der Detektorsubstanz (150) abhängig sind von einer Konzentration des Analyten (144) in der Detektorkammer (140), wobei die Detektorsubstanz (150) elektrisch geladene Teilchen (176) umfasst, insbesondere elektrisch geladenen Partikel und/oder elektrisch geladene Moleküle, wobei die Detektorkammer (140) und die Detektorsubstanz (150) derart ausgestaltet sind, dass ein mittlerer Abstand der elektrisch geladenen Teilchen (176) von der Sensorelektrode (162) und/oder eine Ladungsverteilung innerhalb der Detektorkammer (140) abhängig sind von einer Konzentration des Analyten (144) in der Detektorkammer (140), wobei in der Detektorkammer (140) mindestens ein Rezeptor (160) aufgenommen ist, wobei der Rezeptor (160) eingerichtet ist, um den Analyten (144) reversibel zu binden.

**Claims**

**1.** Sensor device (110) for detecting at least one analyte (144) in a fluid (146), in particular in a bodily fluid, comprising at least one closed detector chamber (140) and at least one electrical sensor (112) having at least one sensor electrode (162), wherein the detector chamber (140) is able to be connected to the fluid (146) such that the analyte (144) is able to enter into the detector chamber (140), wherein the detector chamber (140) comprises at least one detector substance (150), wherein the detector substance (150) is designed so as to influence at least one electrical property of the electrical sensor (112), in particular at least one electrical property of the sensor electrode (162), depending on a concentration of the analyte (144) in the detector chamber (140), wherein the detector substance (150) is designed such that dielectric properties of the detector substance (150) are dependent on a concentration of the analyte (144) in the detector chamber (140), wherein the detector substance (150) comprises electrically charged particles (176), in particular electrically charged particles and/or electrically charged molecules, wherein the detector chamber (140) and the detector substance (150) are designed such that an average spacing between the electrically charged particles (176) and the sensor electrode (162) and/or a charge distribution within the detector chamber (140) are dependent on a concentration of the analyte (144) in the detector chamber (140), wherein at least one receptor (160) is accommodated in the detector chamber (140), wherein the receptor (160) is designed so as to reversibly bind the analyte (144).

**2.** Sensor device (110) according to the preceding claim, wherein the detector substance (150) furthermore comprises at least one spacer (178), wherein the spacer (178) is designed so as to bind the electrically charged particles (176) to the sensor electrode (162) and/or a surface of the detector chamber (140) on the sensor electrode (162), wherein the spacer (178) is designed so as to adopt at least two positions in which the electrically charged particles (176) have a different spacing from the sensor electrode (162).

**3.** Sensor device (110) according to either of the preceding claims, wherein the receptor (160) is an immobilized receptor (160), preferably a receptor (160) immobilized on the sensor electrode (162) and/or a surface of the detector chamber (140) on the sensor electrode (162).

**4.** Sensor device (110) according to one of the preceding claims, wherein the detector substance (150) comprises at least one detection reagent, wherein the detection reagent is designed so as to react with the analyte (144), in particular at least one enzyme.

5. Sensor device (110) according to one of the preceding claims, wherein the detector chamber (140) has at least one membrane (148), in particular at least one analyte-specific membrane (148), wherein the membrane (148) is designed so as to allow the analyte (144) to reversibly enter into the detector chamber (140) and preferably to keep the at least one detector substance (150) in the detector chamber (140).

6. Sensor device (110) according to one of the preceding claims, wherein the electrical sensor (112), in addition to the sensor electrode (162), furthermore comprises at least one counter-electrode (172), wherein the sensor device (110) is designed so as to apply at least one electrical potential to the counter-electrode (172).

7. Sensor device (110) according to one of the preceding claims, wherein the sensor device (110) furthermore comprises at least one compensation sensor (113).

8. Sensor device (110) according to the preceding claim, wherein the compensation sensor (113) comprises at least one compensation sensor electrode (163), wherein the detector substance (150) is connected to the sensor electrode (162), wherein the compensation sensor electrode (163) is free from the detector substance (150).

9. Sensor device (110) according to one of the two preceding claims, wherein the electrical sensor (112) and the compensation sensor (113) are incorporated into an electrical bridge circuit (225) .

10. Sensor device (110) according to one of the preceding claims, wherein the electrical sensor (112) comprises at least one field-effect transistor (114), in particular an organic field-effect transistor (116), wherein the sensor electrode (162) comprises at least one gate electrode (130) of the field-effect transistor (114) .

11. Sensor device (110) according to one of the preceding claims, wherein the sensor device (110) furthermore comprises at least one controller (154), wherein the controller (154) is designed so as to record the at least one electrical property of the electrical sensor (112).

12. Sensor device (110) according to one of the preceding claims, wherein the electrical sensor (112), in particular the sensor electrode (162), has at least one organic conductive or semiconductor material (134), in particular at least one conductive or semiconductor polymer.

13. Sensor system, comprising at least one sensor device (110) according to one of the preceding claims and at least one reader (212), wherein the sensor device (110) and the reader (212) are designed so as to communicate with one another, in particular wirelessly.

14. Method for detecting at least one analyte (144) in a fluid (146), in particular using a sensor device (110) according to one of the preceding claims relating to a sensor device (110), wherein at least one closed detector chamber (140) and at least one electrical sensor (112) having at least one sensor electrode (162) are used, wherein the detector chamber (140) is connected to the fluid (146) such that the analyte (144) enters into the detector chamber (140), wherein the detector chamber (140) comprises at least one detector substance (150), wherein the detector substance (150) influences at least one electrical property of the electrical sensor (112) depending on a concentration of the analyte (144) in the detector chamber (140), wherein the detector substance (150) is designed such that dielectric properties of the detector substance (150) are dependent on a concentration of the analyte (144) in the detector chamber (140), wherein the detector substance (150) comprises electrically charged particles (176), in particular electrically charged particles and/or electrically charged molecules, wherein the detector chamber (140) and the detector substance (150) are designed such that an average spacing between the electrically charged particles (176) and the sensor electrode (162) and/or a charge distribution within the detector chamber (140) are dependent on a concentration of the analyte (144) in the detector chamber (140), wherein at least one receptor (160) is accommodated in the detector chamber (140), wherein the receptor (160) is designed to reversibly bind the analyte (144).

**Revendications**

1. Dispositif détecteur (110), destiné à déceler au moins un analyte (144) dans un fluide (146), notamment dans un fluide corporel, comprenant au moins une chambre de détection (140) close et au moins un capteur (112) électrique,

pourvu d'au moins une électrode de détection (162), la chambre de détection (140) étant susceptible d'être amenée en liaison avec le fluide (146) de telle sorte que l'analyte (144) puisse pénétrer dans la chambre de détection (140), la chambre de détection (140) comprenant au moins une substance de détection (150), la substance de détection (150) étant élaborée de sorte à influencer au moins une propriété électrique du capteur (112) électrique, notamment au moins une propriété électrique de l'électrode de détection (162) en fonction d'une concentration de l'analyte (144) dans la chambre de détection (140), la substance de détection (150) étant conçue de telle sorte que les propriétés électriques de la substance de détection (150) soient dépendantes d'une concentration de l'analyte (144) dans la chambre de détection (140), la substance de détection (150) comprenant des petits éléments (176) chargés électriquement, notamment des particules chargées électriquement et/ou des molécules chargées électriquement, la chambre de détection (140) et la substance de détection (150) étant conçues de telle sorte qu'une distance moyenne entre les petits éléments (176) chargés électriquement et l'électrode de détection (162) et/ou qu'une distribution des charges à l'intérieur de la chambre de détection (140) soient dépendant(e)s d'une concentration des analytes (144) dans la chambre de détection (140), dans la chambre de détection (140) étant réceptionné au moins un récepteur (160), le récepteur (160) étant élaboré de sorte à lier l'analyte (144) de manière réversible.

2. Dispositif détecteur (110) selon la revendication précédente, **caractérisé en ce que** la substance de détection (150) comprend en outre au moins un espaceur (178), l'espaceur (178) étant élaboré pour lier les petits éléments (176) chargés électriquement sur l'électrode de détection (162) et/ou pour lier une surface de la chambre de détection (140) sur l'électrode de détection (162), l'espaceur (178) étant élaboré pour adopter au moins deux positions dans lesquelles les petits éléments (176) chargés électriquement présentent un écart par rapport à l'électrode de détection (162) .

3. Dispositif détecteur (110) selon l'une quelconque des revendications précédentes, le récepteur (160) étant un récepteur (160) immobilisé, de préférence un récepteur (160) immobilisé sur l'électrode de détection (162) et/ou sur une surface de la chambre de détection (140), sur l'électrode de détection (162).

4. Dispositif détecteur (110) selon l'une quelconque des revendications précédentes, la substance de détection (150) comprenant au moins un réactif révélateur, le réactif révélateur étant élaboré pour réagir avec l'analyte (144), notamment au moins un enzyme.

5. Dispositif détecteur (110) selon l'une quelconque des revendications précédentes, la chambre de détection (140) comportant au moins une membrane (148), notamment au moins une membrane (148) spécifique à l'analyte, la membrane (148) étant élaborée pour permettre de manière réversible la pénétration de l'analyte (144) dans la chambre de détection (140) et de préférence pour retenir l'au moins une substance de détection (150) dans la chambre de détection (140).

6. Dispositif détecteur (110) selon l'une quelconque des revendications précédentes, le capteur (112) électrique comprenant en sus de l'électrode de détection (162), par ailleurs au moins une contre-électrode (172), le dispositif détecteur (110) étant élaboré pour soumettre la contre-électrode (172) à au moins un potentiel électrique.

7. Dispositif détecteur (110) selon l'une quelconque des revendications précédentes, le dispositif détecteur (110) comprenant par ailleurs au moins un capteur compensateur (113).

8. Dispositif détecteur (110) selon la revendication précédente, le capteur compensateur (113) comprenant au moins une électrode compensatrice de capteur (163), la substance de détection (150) étant reliée avec l'électrode de détection (162), l'électrode compensatrice de capteur (163) étant exempte de substance de détection (150).

9. Dispositif détecteur (110) selon l'une quelconque des deux revendications précédentes, le capteur (112) électrique et le capteur compensateur (113) étant intégrés dans un circuit en pont (225).

10. Dispositif détecteur (110) selon l'une quelconque des revendications précédentes, le capteur (112) électrique comprenant au moins un transistor à effet de champ (116), notamment un transistor à effet de champ (114) organique, l'électrode de détection (162) comprenant au moins une électrode de grille (130) du transistor à effet de champ (114) .

11. Dispositif détecteur (110) selon l'une quelconque des revendications précédentes, le dispositif détecteur (110) comprenant par ailleurs au moins un dispositif de commande (154), le dispositif de commande (154) étant élaboré pour détecter l'au moins une propriété électrique du capteur (112) électrique.

**12.** Dispositif détecteur (110) selon l'une quelconque des revendications précédentes, le capteur (112) électrique, notamment l'électrode de détection (162) comportant au moins une matière (134) organique conductrice ou semi-conductrice, notamment au moins un polymère conducteur ou semiconducteur.

**13.** Système détecteur comprenant au moins un dispositif détecteur (110) selon l'une quelconque des revendications précédentes et au moins un lecteur (212), le dispositif détecteur (110) et le lecteur (212) étant élaborés pour communiquer l'un avec l'autre, notamment sans fil.

**14.** Procédé, destiné à déceler au moins un analyte (144) dans un fluide (146), notamment en utilisant un dispositif détecteur (110) selon l'une quelconque des revendications précédentes concernant un dispositif détecteur (110), au moins une chambre de détection (140) close et au moins un capteur (112) électrique pourvu d'au moins une électrode de détection (162) étant utilisés, la chambre de détection (140) étant amenée en liaison avec le fluide (146) de telle sorte que l'analyte (144) pénètre dans la chambre de détection (140), la chambre de détection (140) comprenant au moins une substance de détection (150), la substance de détection (150) influençant au moins une propriété électrique du capteur (112) électrique en fonction d'une concentration de l'analyte (144) dans la chambre de détection (140), la substance de détection (150) étant élaborée de telle sorte que les propriétés électriques de la substance de détection (150) soient dépendantes d'une concentration de l'analyte (144) dans la chambre de détection (140), la substance de détection (150) comprenant des petits éléments (176) chargés électriquement, notamment des particules chargées électriquement et/ou des molécules chargées électriquement, la chambre de détection (140) et la substance de détection (150) étant élaborées de telle sorte qu'une distance moyenne entre les petits éléments (176) chargés électriquement et l'électrode de détection (162) et/ou qu'une distribution de charge à l'intérieur de la chambre de détection (140) soient dépendant(e)s d'une concentration de l'analyte (144) dans la chambre de détection (140), dans la chambre de détection (140) étant réceptionné au moins un récepteur (160), le récepteur (160) étant élaboré pour lier l'analyte (144) de manière réversible.

Fig. 1 A

Fig. 1 B

Fig. 2 A

Fig. 2 B

Fig. 3 A

Fig. 3 B

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6360888 B1
- US 20080242962 A1
- WO 2007130694 A
- US 20050123442 A
- DE 3513168 A1
- DE 3854886 T2
- US 4324256 A
- WO 2006008505 A1
- WO 0004386 A2
- US 4846937 A
- US 20100155239 A1
- EP 2228004 A1
- DE 19591159 A1

**In der Beschreibung aufgeführte Nicht-Patentliteratur**

- **M. A. RAHMAN et al.** Electrochemical Sensors Based on Organic Conjugated Polymers. *Sensors,* 2008, vol. 8, 118-141
- **B. EM: ISFET.** *Theory and Practice, IEEE Sensor Conference Toronto,* Oktober 2003
- **M. MCALPINE.** Nanotechnology-Enabled Flexible Sensors for Medical Diagnostics. *Organic Electronic Congress,* 27. Oktober 2008
- **U. BEYER et al.** Recording of subcutaneous glucose dynamics by a viscometric affinity sensor. *Diabetologia,* 2001, vol. 44, 416-423